# EUROPEAN PATENT APPLICATION

(11) **EP 1 334 711 A1**
(43) Date of publication of application: **13.08.2003**
(21) Application number: 01974898.7
(22) Date of filing: 23.10.2001
(51) Int. Cl.: A61K 6/083, A61K 6/08, C08F 20/26, C08F 20/38

(54) **DENTAL MATERIAL AND COMPOSITION**

(30) Priority: 23.10.2000 JP 2000323144
(71) Applicant: SUN MEDICAL CO., LTD., Shiga-ken 524-0044 (JP); Mitsui Chemicals, Inc., Tokyo 100-6070 (JP)
(72) Inventor: HONDA, Narimichi, SUN MEDICAL CO., LTD., Moriyama-shi, Shiga 524-0044 (JP); KASAGI, Akifumi, SUN MEDICAL CO., LTD., Moriyama-shi, Shiga 524-0044 (JP); TAKAGI, Masatoshi, MITSUI CHEMICALS, INC., Sodegaura-shi, Chiba 299-0265 (JP); OTSUJI, Atsuo, MITSUI CHEMICALS, INC., Sodegaura-shi, Chiba 299-0265 (JP); TAKUMA, Keisuke, MITSUI CHEMICALS, INC., Chiyoda-ku, Tokyo 100-6070 (JP); FUJIYAMA, Takahiro, MITSUI CHEMICALS, INC., Sodegaura-shi, Chiba 299-0265 (JP)
(74) Representative: Ford, Michael Frederick
(86) International application number: JP0109281
(87) International publication number: WO02034207

(57) **Abstract**

Disclosed is a dental material comprising a sulfur containing unsaturated carboxylic acid ester compound having a substituent containing a sulfur atom and at least two (meth)acrylic acid residues through an oxygen atom bonded to a secondary or tertiary carbon atom, specifically, a dental material comprising a polymerizable monomer (A) represented by the following formula (1): 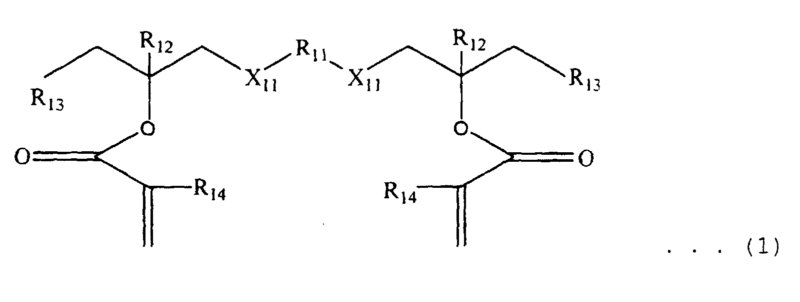 wherein R₁₁ is a divalent organic group, X₁₁ is an oxygen atom, a sulfur atom, -COO- group or -(CH₂)_{q}X₁₂- group (X₁₂ is an oxygen atom or a sulfur atom, and q is an integer of 1 to 3), R₁₂ is a hydrogen atom or an alkyl group, R₁₃ is a substituent containing a sulfur atom, and R₁₄ is a hydrogen atom or a methyl group,
or a dental material comprising a polymerizable monomer (A) which is liquid at ordinary temperature and has a viscosity of 100 to 1,000,000 cps. Also disclosed is a dental composition comprising the polymerizable monomer (A), a polymerization initiator (B) and a filler (C). The dental material and the dental composition are excellent in X-ray opacity, transparency, curability and stain resistance, and they have small polymerization shrinkage.

## Description

### TECHNICAL FIELD

The present invention relates to dental materials and compositions. More particularly, the invention relates to dental materials and dental compositions which are capable of forming cured products having excellent transparency and X-ray opacity, have high curability (particularly photo-curability), have small polymerization shrinkage and are useful for composite resins, hard resins and artificial teeth.

### BACKGROUND ART

As fillers contained in dental compositions for composite resins, hard resins or artificial teeth, powdery inorganic components such as silica and glass are used for the purposes of imparting mechanical strength to the compositions and improving various properties of the resin matrix.

In the dental treatment, the result of the treatment is often confirmed by X-ray photography, so that as a pulverized glass powder contained in the dental composition, a powder obtained by pulverizing a glass containing a heavy metal element having X-ray opacity, such as barium, zirconium or strontium, has been heretofore used so that X-ray photography should be feasible in the dental treatment. The pulverized glass powder is generally prepared by, for example, pulverizing such a glass as mentioned above. In the conventional technique for glass pulverization, however, it is difficult to finely pulverize a glass, and a glass powder having particle diameters of 10 µm to several tens µm has been usually employed. If a dental composition containing a glass powder of such large particle diameters is used, it is very difficult to form a finished and polished surface having a polish clinically similar to that of natural tooth.

In order to solve problems associated with incorporation of such a glass powder, composite resins mainly using a finely pulverized glass powder having an average particle diameter of not more than 2 µm have been developed recently. For example, an invention using a pulverized glass powder having an average particle diameter of 0.1 to 5 µm and/or an inorganic compound having an average particle diameter of 0.01 to 0.04 µm, such as silica fine particles, is disclosed in Japanese Patent Laid-Open Publication No. 194135/1993.

The surface polish that has been a disadvantage with conventional composite resins containing a pulverized glass powder can be improved by using composite resins containing such a finely pulverized glass powder. However, the dental composition containing such a finely pulverized glass powder still has many disadvantages to be improved in the balance of properties, such as transparency, photo-curability, polymerization shrinkage and X-ray opacity.

For example, in case of a dental composition wherein a finely pulverized filler having an average particle diameter of not more than 2 µm is homogeneously contained, the boundary area between the filler and a resin matrix (resin cured product) is markedly increased, and in order to ensure its transparency, refractive indexes of the filler and the resin matrix need to be approximated to each other. However, if the content of the heavy metal element in the filler is increased to ensure high X-ray opacity, the refractive index of the filler becomes high. The refractive index of a resin matrix having been heretofore applied to dental use, such as Bis-GMA, is at most about 1.55, and by approximating the refractive index of the filler to said value, the transparency of the dental composition has been ensured.

As described above, with regard to the transparency of teeth, extremely high transparency in the appearance is often required for the composite resin or the hard resin used for the front tooth cutting edge or the like. In such a part, the light transmittance at the wavelength of 480 nm that is a wavelength of a light from a dental light irradiator needs to be not less than 5 % in many cases. On the other hand, in the dentin portion or the like, the transparency of the dental resin or the hard resin may be relatively low because such a part is free from problems in the appearance, and it is possible to use even a dental resin or a hard resin having a transparency of not more than 1 %. In order to control shade of the teeth, however, a pigment such as titanium oxide or red iron oxide is used, so that even the dentin portion is desired to have high transparency to enhance the degree of freedom of coloring. In case of a dental material of photopolymerization type, there is an advantage that higher transparency brings about larger curing depth and higher degree of polymerization, and as a result, mechanical properties are enhanced.

The light transmittance of a dental composition containing no pigment is preferably not less than 0.05 %, more preferably not less than 1 %, and in the practical use, the light transmittance is particularly preferably not less than 5 %. The X-ray opaque value of such a material commercially available at present is about 200 to 300 % based on aluminum, so that it is feasible to distinguish such a material from the tooth enamel (X-ray opaque value: about 180 % based on aluminum) by X-rays in the usual filling treatment. In case of filling in a small amount or thin filling, however, it becomes difficult to clearly distinguish such a material from the tooth enamel.

As a means to decrease polymerization shrinkage that is a clinically serious problem in the dental material, use of a dental composition containing a composite filler obtained by pulverizing a cured product of a mixture of an inorganic filler and a polymerizable monomer is known. Although the dental composition can be decreased in the polymerization shrinkage, refractive indexes of the inorganic filler, a resin matrix of the composite filler and a resin matrix of the dental composition need to be finely suited to each other in order to ensure the transparency.

The present inventors have earnestly studied to solve the problems in the dental materials and the dental compositions, and as a result, they have found that a cured composition containing, as a main ingredient of a sulfur-containing polymerizable monomer, a sulfur-containing unsaturated carboxylic acid ester compound having a substituent containing a sulfur atom and at least two (meth)acrylic acid residues through an oxygen atom bonded to a secondary or tertiary carbon atom satisfies the requirements for dental materials and dental compositions, has excellent and well-balanced curability, transparency and X-ray opacity though the transparency and the X-ray opacity are hardly compatible with each other, and has small polymerization shrinkage. Based on the finding, the present invention has been accomplished.

### DISCLOSURE OF THE INVENTION

The present invention is intended to solve such problems in the dental compositions as described above.

That is to say, it is an object of the present invention to provide a dental material and a dental composition which have high X-ray opacity and transparency reconciled with each other.

It is another object of the invention to provide a dental composition having excellent curability (particularly photo-curability) and having small polymerization shrinkage.

The dental material of the invention comprises a sulfur-containing unsaturated carboxylic acid ester compound having a substituent containing a sulfur atom and at least two (meth)acrylic acid residues through an oxygen atom bonded to a secondary or tertiary carbon atom.

The dental material of the invention comprises at least one polymerizable monomer represented by the following formula (1): 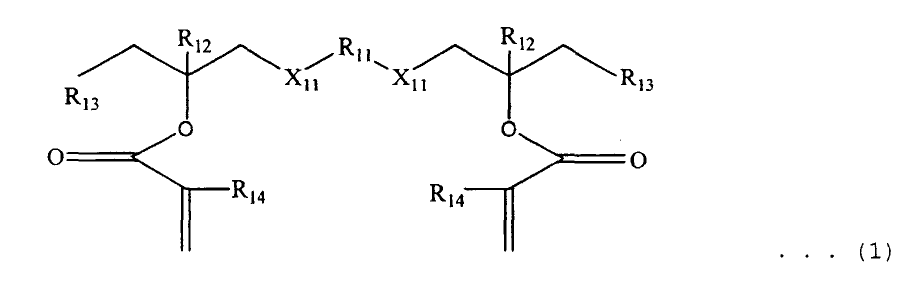 wherein R₁₁ is a divalent organic group, each X₁₁ is independently an oxygen atom, a sulfur atom, -COO- group or -(CH₂)_{q}X₁₂- group (X₁₂ is an oxygen atom or a sulfur atom, and q is an integer of 1 to 3), each R₁₂ is independently a hydrogen atom or an alkyl group, each R₁₃ is independently a substituent containing a sulfur atom, and each R₁₄ is independently a hydrogen atom or a methyl group.

R₁₁ is preferably a divalent organic group represented by any one of the following formulas (2) to (4) : 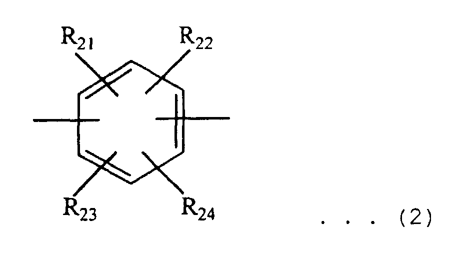 wherein R₂₁, R₂₂, R₂₃ and R₂₄ are each independently a hydrogen atom, an alkyl group, an alkoxyl group, a nitro group or a halogen atom, 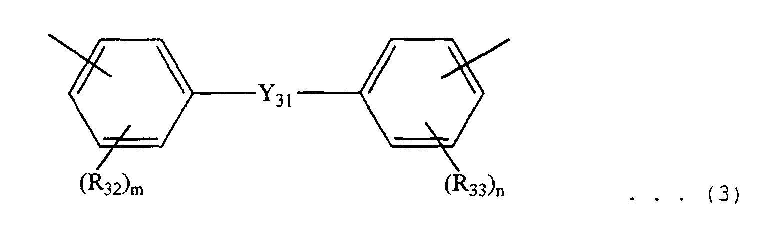 wherein Y₃₁ is a single bond, -C(R₃₁)₂- group (each R₃₁ is independently a hydrogen atom or a methyl group), -O-group, -S- group or -SO₂- group, R₃₂ and R₃₃ are each independently an alkyl group, an alkenyl group, an aralkyl group, an aryl group, an alkoxyl group, an alkylthio group, a nitro group or a halogen atom, and m and n are each independently 0 or an integer of 1 to 4, 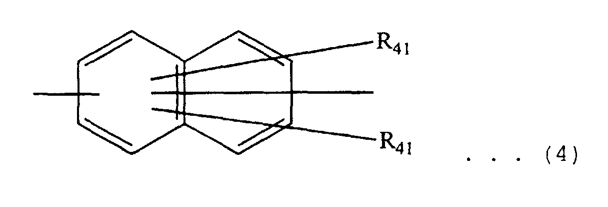 wherein each R₄₁ is independently a hydrogen atom or an alkyl group.

The dental material may comprise the polymerizable monomer which is liquid at ordinary temperature (20°C in the present invention).

The dental composition of the invention comprises:
(A) the polymerizable monomer stated in claim 1 or the polymerizable monomer stated in claim 2 that is represented by the formula (1),
(B) a polymerization initiator, and
(C) a filler.

The dental composition may further comprise (D) a polymerizable monomer other than the polymerizable monomer (A).

In the dental composition, the refractive index of a cured product of only the polymerizable monomer (A) represented by the formula (1) or a cured product of a mixture containing the monomer (A) is preferably not less than 1.55.

The light transmittance of a cured product of the dental composition having a thickness of 1 mm, at a wavelength of 480 nm, is preferably not less than 1 %.

The dental composition usually has X-ray opacity.

The difference between the refractive index of a cured product of only the polymerizable monomer (A) represented by the formula (1) or a cured product of a mixture containing the monomer (A) and the refractive index of the filler (C) is preferably not more than 0.05.

The refractive index of the filler (C) is preferably not less than 1.55.

The average particle diameter of an inorganic compound in the filler (C) is preferably not more than 2 µm.

A part or all of the filler (C) is preferably contained as a composite filler formed from a polymerizable monomer and an inorganic compound.

The dental composition preferably contains:
the filler (C) in an amount of 1 to 2000 parts by weight, and
the radical polymerization initiator (B) in an amount of 0.0001 to 10 parts by weight,
based on 100 parts by weight of the polymerizable monomer in the dental composition.

The filler (C) contained in the dental composition is preferably a composite filler, and this composite filler is desirably contained in an amount of usually 1 to 2000 parts by weight, preferably 5 to 2000 parts by weight, particularly preferably 50 to 700 parts by weight, based on 100 parts by weight of the polymerizable monomer in the dental composition.

The dental composition of the invention is a dental composition containing at least a polymerizable monomer, a filler and a polymerization initiator, and this dental composition contains a sulfur-containing unsaturated carboxylic acid ester compound having a substituent containing a sulfur atom and at least two (meth)acrylic acid residues through an oxygen atom bonded to a secondary or tertiary carbon atom, more specifically, a polymerizable monomer represented by the formula (1).

The dental composition of the invention contains, as the filler, an inorganic compound such as a glass powder. The refractive index of a cured product of only the polymerizable monomer represented by the formula (1) or a cured product of a mixture of the polymerizable monomer and another polymerizable monomer is not less than 1.55, and the difference between said refractive index and a refractive index of the glass powder is preferably not more than 0.05. The glass powder preferably has X-ray opacity.

### BRIEF DESCRIPTION OF THE DRAWING

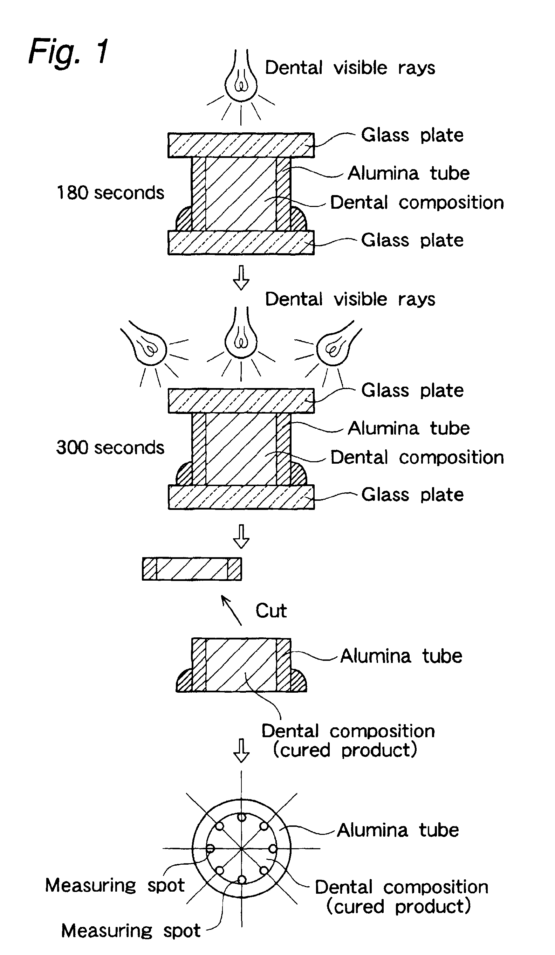

Fig. 1 is a view illustrating a method to measure polymerization shrinkage of a dental composition.

### BEST MODE FOR CARRYING OUT THE INVENTION

The dental material and the dental composition according to the invention are described in detail hereinafter.

The dental material and the dental composition of the invention contain, as a polymerizable monomer, a sulfur-containing unsaturated carboxylic acid ester compound having a substituent containing a sulfur atom and at least two (meth)acrylic acid residues through an oxygen atom bonded to a secondary or tertiary carbon atom, preferably at least one polymerizable monomer (A) represented by the formula (1).

### (A) Polymerizable monomer represented by the formula (1)

First, the polymerizable monomer (A) represented by the following formula (1) is described.

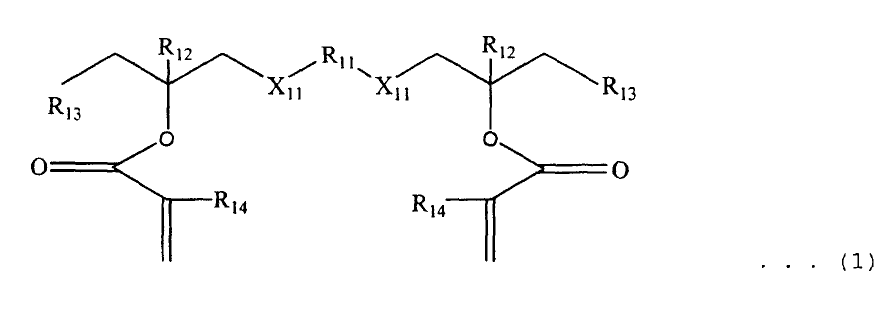

In the formula (1), R₁₁ is a divalent organic group, each X₁₁ is independently an oxygen atom, a sulfur atom, -COO- group or -(CH₂)_{q}X₁₂- group (X₁₂ is an oxygen atom or a sulfur atom, and q is an integer of 1 to 3), each R₁₂ is independently a hydrogen atom or an alkyl group, each R₁₃ is independently a substituent containing a sulfur atom, and each R₁₄ is independently a hydrogen atom or a methyl group.

Details of the polymerizable monomer (A) represented by the formula (1) are clearly explained below.

In the formula (1), R₁₁ is a divalent organic group and is, for example, an alkylene group, an alkenylene group, a cycloalkylene group or an allylene group, which may be unsubstituted or substituted, though it is not limited thereto. Examples of such groups include pentamethylene, hexamethylene, heptamethylene, 1,3-pentylene, 1,4-cyclohexylene, 1,3-phenylene, 1,4-phenylene, 1,4-naphthalene, 2,6-naphthalene, and their substituted groups. R₁₁ is preferably a group represented by any one of the following formulas (2) to (4), particularly preferably a group represented by the formula (2) or (3).

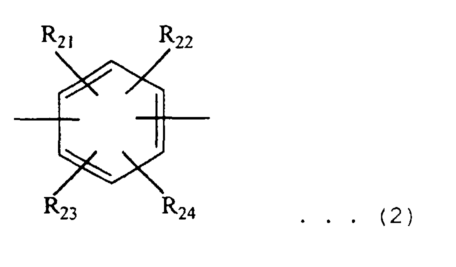

In the formula (2) representing a preferred group of R₁₁, R₂₁, R₂₂, R₂₃ and R₂₄ are each independently a hydrogen atom, an alkyl group, an alkoxyl group, a nitro group or a halogen atom.

The alkyl group is a straight-chain, branched or cyclic alkyl group which may have a substituent, preferably a straight-chain, branched or cyclic alkyl group of 1 to 20 carbon atoms, which may have a substituent.

The alkoxyl group is a straight-chain, branched or cyclic alkoxyl group which may have a substituent, preferably a straight-chain, branched or cyclic alkoxyl group of 1 to 20 carbon atoms, which may have a substituent.

The halogen atom is, for example, a fluorine atom, a chlorine atom, a bromine atom or an iodine atom.

In the formula (2), R₂₁, R₂₂, R₂₃ and R₂₄ are each preferably a hydrogen atom, an unsubstituted straight-chain or branched alkyl group of 1 to 10 carbon atoms, an unsubstituted straight-chain or branched alkoxyl group of 1 to 10 carbon atoms, a nitro group, a chlorine atom or a bromine atom, more preferably a hydrogen atom, a methyl group, an ethyl group, an n-propyl group, an isopropyl group, an n-butyl group, an isobutyl group, a tert-butyl group, a methoxyl group, an ethoxyl group, an n-propoxyl group, an isopropoxyl group, an n-butoxyl group, an isobutoxyl group, a tert-butoxyl group, a nitro group, a chlorine atom or a bromine atom.

In the polymerizable monomer represented by the formula (1), there are three modes as bonding modes between the benzene ring R₁₁ and the two X₁₁ groups bonded to the benzene ring, namely, bonding at the para-positions, the meta-positions and the ortho-positions to R₁₁. Preferable is a compound having a structure wherein the X₁₁ groups are bonded at the para-positions or the meta-positions, preferably the meta-positions.

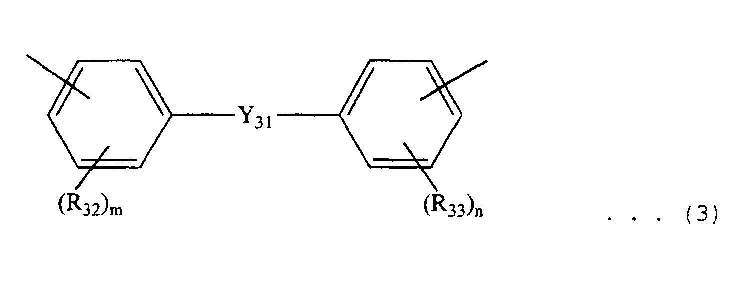

In the formula (3) representing a preferred group of R₁₁, Y₃₁ is a single bond, -C(R₃₁)₂- group (each R₃₁ is independently a hydrogen atom or a methyl group), -O- group, -S- group or -SO₂- group, R₃₂ and R₃₃ are each independently an alkyl group, an alkenyl group, an aralkyl group, an aryl group, an alkoxyl group, an alkylthio group, a nitro group or a halogen atom, and m and n are each independently an integer of 0 to 4.

With regard to the bonding mode between the benzene ring R₁₁ and the two X₁₁ groups bonded to the benzene ring, there is no specific limitation on the positional relation between the two X₁₁ groups.

The alkyl group is a straight-chain, branched or cyclic alkyl group which may have a substituent, preferably a straight-chain, branched or cyclic alkyl group of 1 to 20 carbon atoms, which may have a substituent, more preferably an unsubstituted straight-chain or branched alkyl group of 1 to 10 carbon atoms.

The alkenyl group is a straight-chain, branched or cyclic alkenyl group which may have a substituent, preferably a straight-chain, branched or cyclic alkenyl group of 1 to 20 carbon atoms, which may have a substituent, more preferably an unsubstituted straight-chain or branched alkenyl group of 1 to 10 carbon atoms.

The aralkyl group is an aralkyl group which may have a straight-chain, branched or cyclic substituent, preferably an aralkyl group which may have a straight-chain, branched or cyclic substituent whose non-aromatic portion has 1 to 20 carbon atoms, more preferably an aralkyl group having a straight-chain or branched substituent whose non-aromatic portion has 1 to 10 carbon atoms.

The aryl group is an aryl group which may have a straight-chain, branched or cyclic substituent, more preferably an aryl group which may have a straight-chain, branched or cyclic substituent of 1 to 20 carbon atoms.

The alkoxyl group is a straight-chain, branched or cyclic alkoxyl group which may have a substituent, preferably a straight-chain, branched or cyclic alkoxyl group of 1 to 20 carbon atoms, which may have a substituent, more preferably an unsubstituted straight-chain or branched alkoxyl group of 1 to 10 carbon atoms.

The alkylthio group is a straight-chain, branched or cyclic alkylthio group which may have a substituent, preferably a straight-chain, branched or cyclic alkylthio group of 1 to 20 carbon atoms, which may have a substituent, more preferably an unsubstituted straight-chain or branched alkylthio group of 1 to 10 carbon atoms.

The halogen atom is, for example, a fluorine atom, a chlorine atom, a bromine atom or an iodine atom.

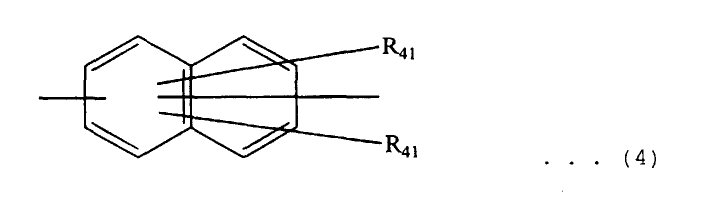

In the formula (4) representing a preferred group of R₁₁, each R₄₁ is independently a hydrogen atom or an alkyl group.

With regard to the bonding mode between the naphthalene ring R₁₁ and the two X₁₁ groups bonded to the naphthalene ring, the two X₁₁ groups may be bonded to the same benzene ring or may be bonded to different benzene rings. There is no specific limitation on the positional relation between the two X₁₁ groups. The alkyl group is a straight-chain, branched or cyclic alkyl group which may have a substituent, preferably a straight-chain, branched or cyclic alkyl group of 1 to 20 carbon atoms, which may have a substituent, more preferably an unsubstituted straight-chain or branched alkyl group of 1 to 10 carbon atoms.

In the formula (1), X₁₁ is preferably an oxygen atom or a sulfur atom, more preferably an oxygen atom.

In the formula (1), each R₁₂ is independently a hydrogen atom or an alkyl group. The alkyl group is a straight-chain, branched or cyclic alkyl group which may have a substituent, preferably a straight-chain, branched or cyclic alkyl group of 1 to 10 carbon atoms, which may have a substituent. Each R₁₂ is particularly preferably a hydrogen atom or a methyl group.

In the formula (1), each R₁₃ is independently a substituent containing a sulfur atom, and in the structure of the substituent, at least one sulfur atom is contained. Because of the sulfur atom contained in the substituent, the refractive index of the monomer can be easily controlled, so that such a monomer is preferable to the conventional monomers. The substituent R₁₃ is, for example, a substituent represented by the following formula (5) or (6).

R₁₃ₐ-O- (5)

R_{13b}-S- (6)

In the formula (5), R₁₃ₐ is a monovalent organic group containing at least one sulfur atom, and in the formula (6), R_{13b} is a monovalent organic group which may contain a sulfur atom.

In the above formula, the substituent R₁₃ₐ is preferably an alkyl group, an aralkyl group, an aryl group or an acyl group, which contains at least one sulfur atom and may have a substituent. The substituent R₁₃ₐ may be a heterocyclic ring-containing group.

In the above formula, the substituent R_{13b} is preferably an alkyl group, an aralkyl group, an aryl group or an acyl group, which may have a substituent.

When the substituent R₁₃ₐ or R_{13b} contains a sulfur atom, the refractive index of the monomer represented by the formula (1) tends to increase.

The substituent R₁₃ is preferably a chain or cyclic alkoxyl group containing a sulfur atom, an aralkyloxy group containing a sulfur atom, an aryloxy group containing a sulfur atom, an acyloxy group containing a sulfur atom, an aralkylthio group which may contain a sulfur atom, an arylthio group which may contain a sulfur atom, or an acylthio group which may contain a sulfur atom.

In the formula (1), each R₁₄ is independently a hydrogen atom or an alkyl group, preferably a hydrogen atom or a methyl group.

Examples of the polymerizable monomers represented by the formula (1) include the later-described compounds No. 1 to No. 349. Details of the process for preparing those compounds are described in EP1057808A2 (laid open on Dec. 6, 2000). The present invention is not limited to those compounds.

Of the above compounds, particularly preferable are:
1,3-bis(3-phenylthio-2-acryloyloxypropyloxy)benzene,
1,3-bis(3-phenylthio-2-methacryloyloxypropyloxy)benzene,
4,4'-bis(3-phenylthio-2-acryloyloxypropyloxy)-3,3',5,5'-tetramethylbiphenyl,
1,3-{2'-acryloyloxy-3'-[2-(1,3-dithiolan-2-yl)ethylthio]propyloxy}benzene,
2,2-bis[4-(2'-acryloyloxy-3'-phenylthiopropyloxy)phenyl]propane,
2,2-bis(4-{2-acryloyloxy-3-[2-(1,3-dithiolan-2-yl)ethylthio]propyloxy}phenyl)propane,
3,3',5,5'-tetramethyl-4,4'-bis{2-acryloyloxy-3-[2-(1,3-dithiolan-2-yl)ethylthio]propyloxy}biphenyl,
1,1-bis[4-(2-acryloyloxy-3-phenylthiopropyloxy)phenyl]methane,
1,1-bis{[2-(2-acryloyloxy-3-phenylthiopropyloxy)phenyl]methane,
1-[2-(2-acryloyloxy-3-phenylthiopropyloxy)phenyl]-1-[4-(2-acryloyloxy-3-phenylthiopropyloxy)phenyl]methane, and
2,7-bis(2'-acryloyloxy-3'-phenylthiopropyloxy)naphthalene.

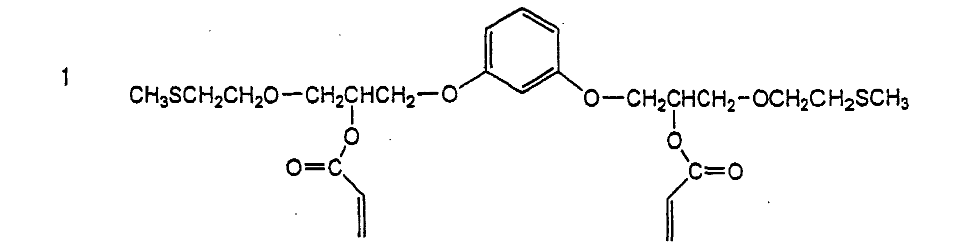

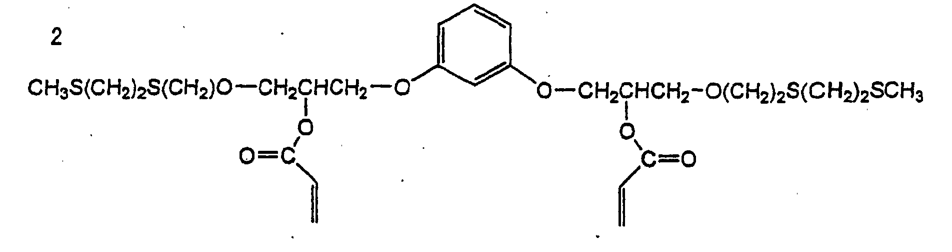

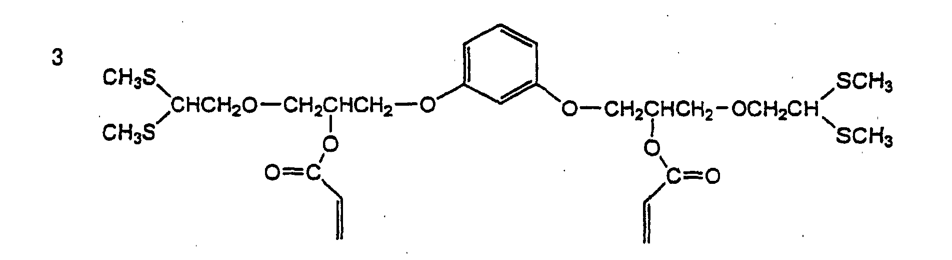

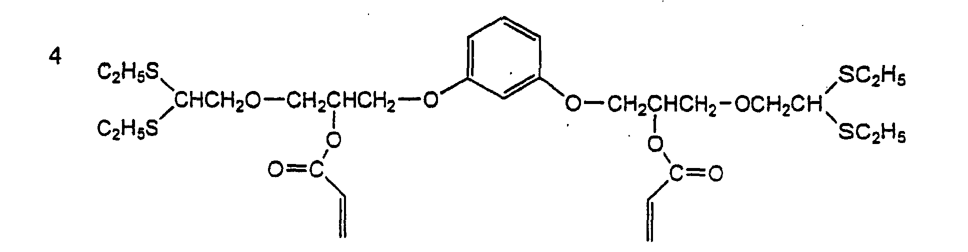

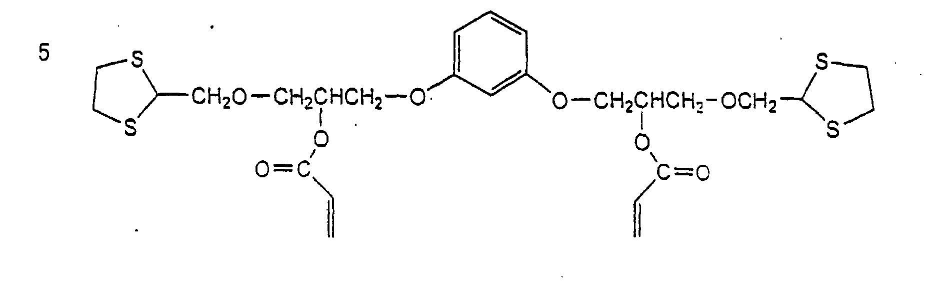

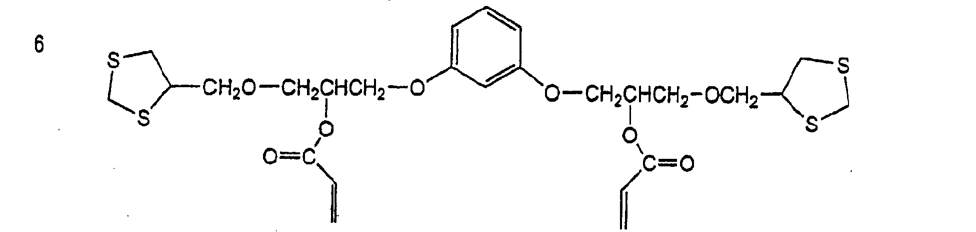

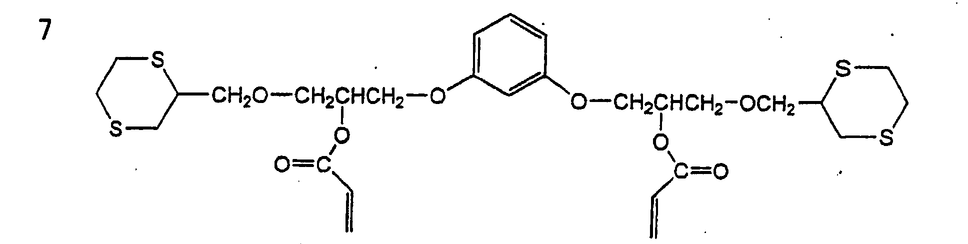

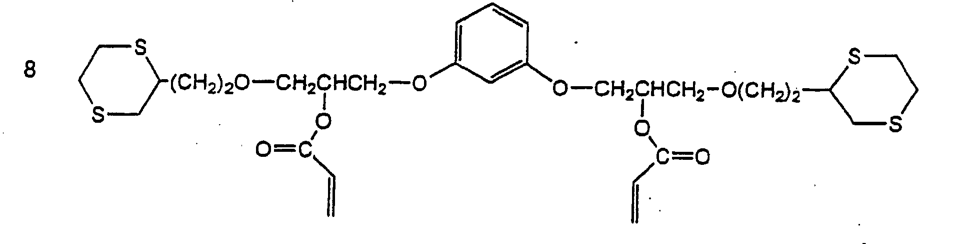

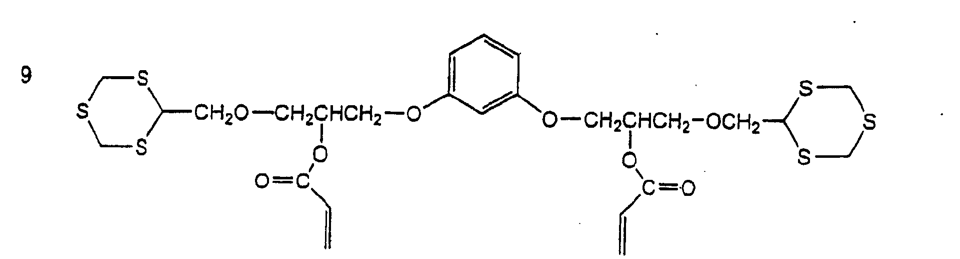

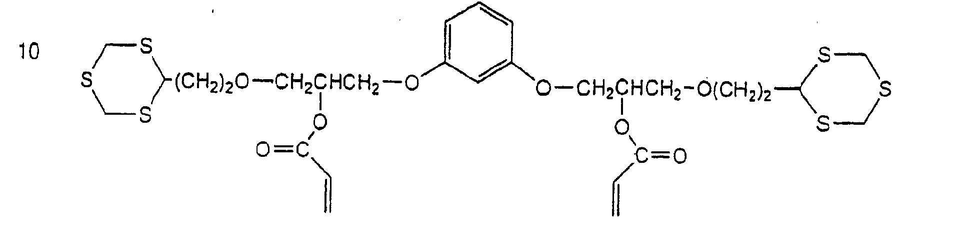

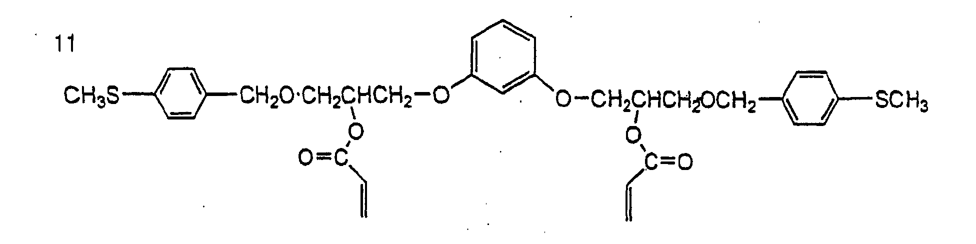

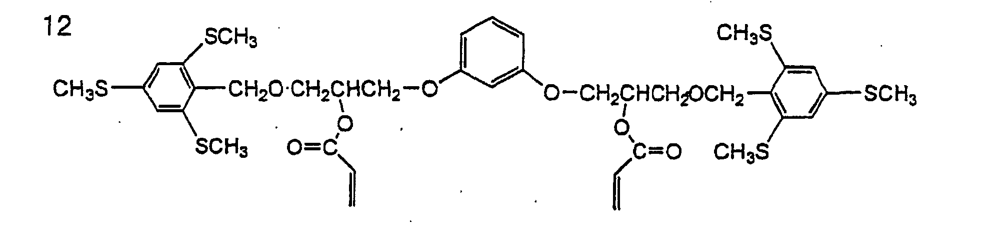

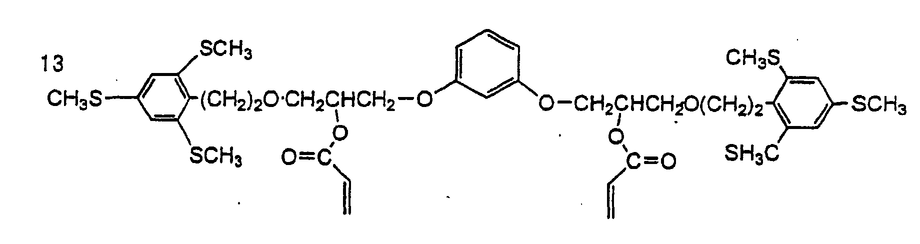

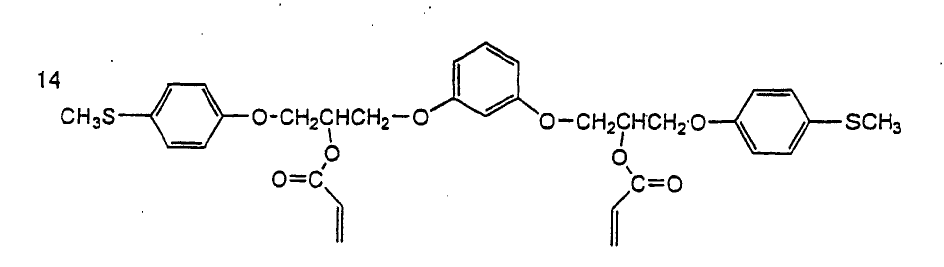

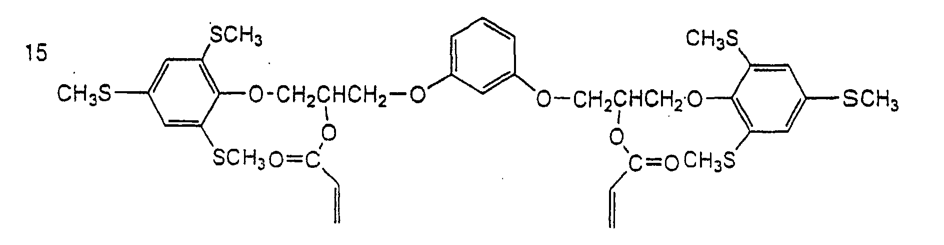

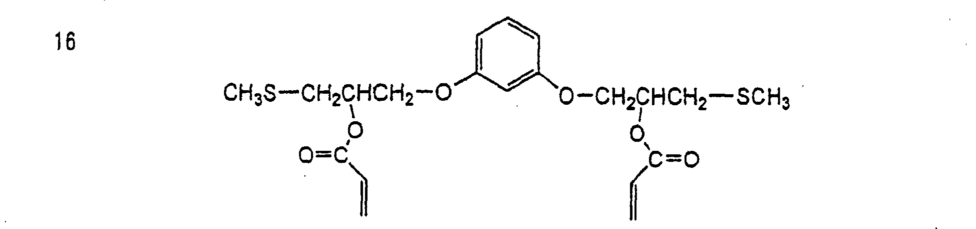

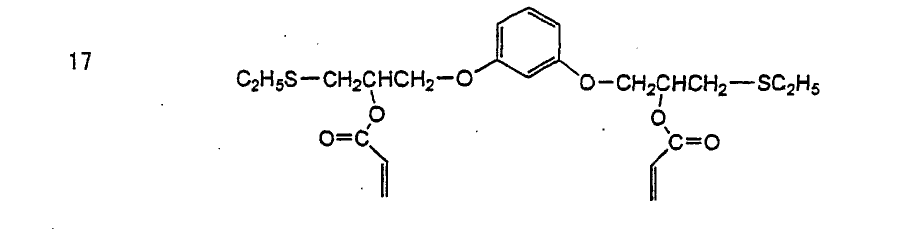

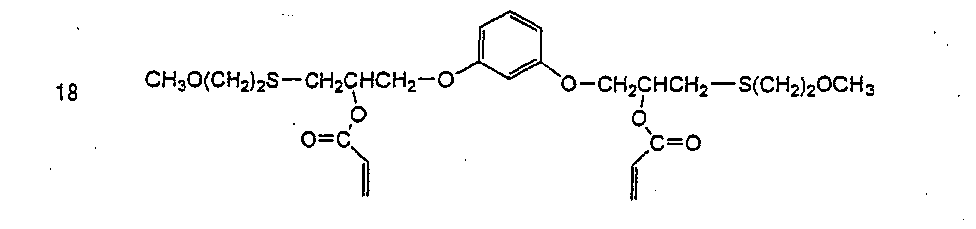

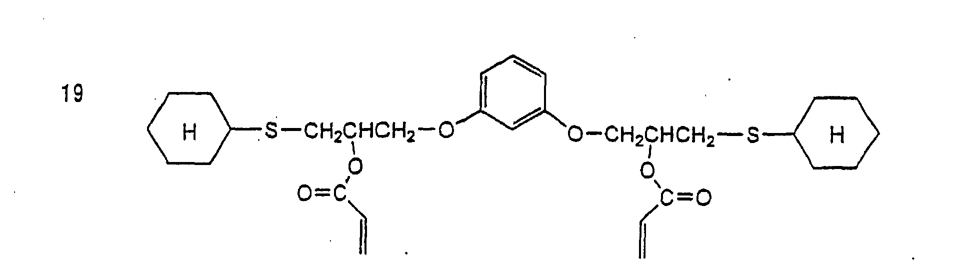

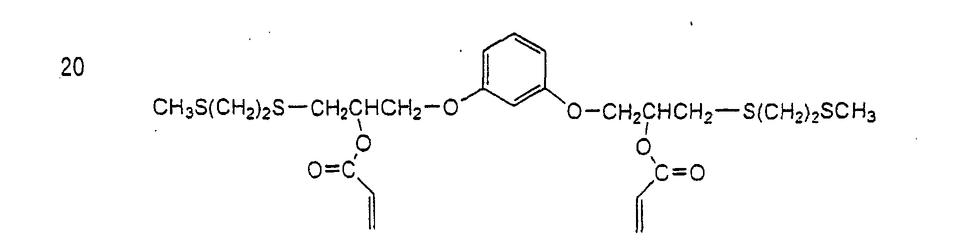

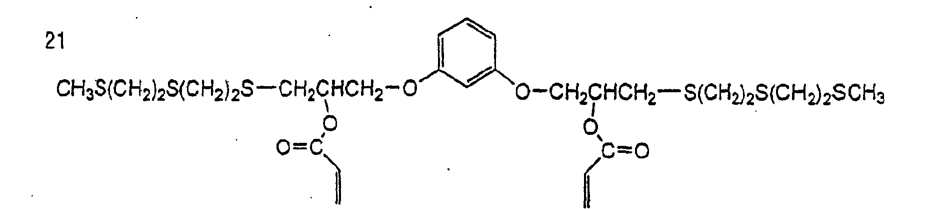

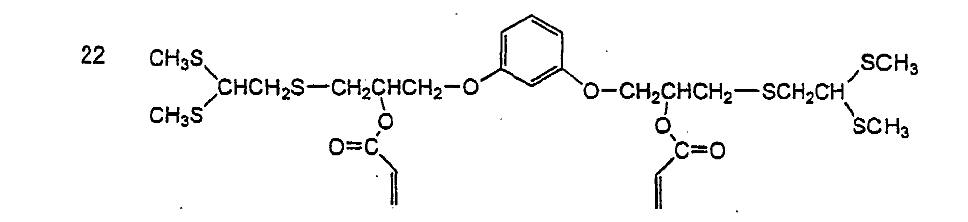

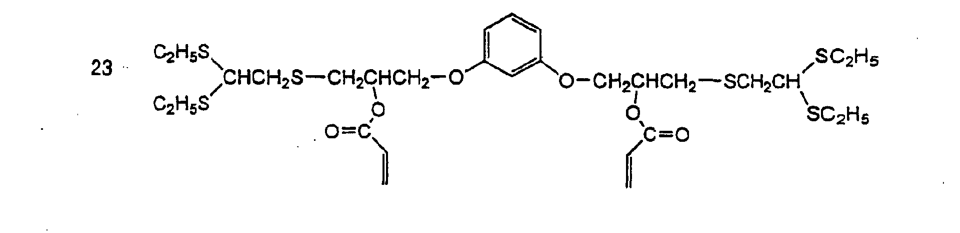

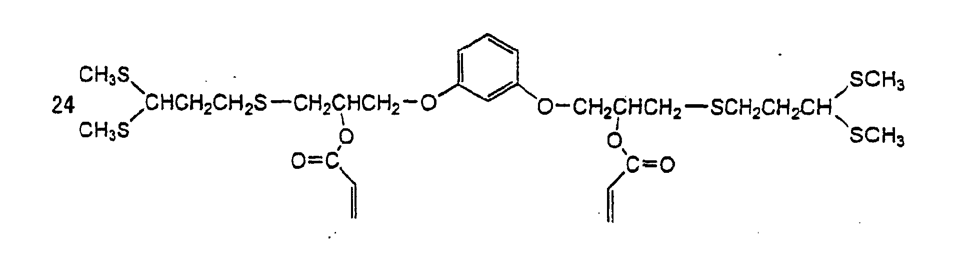

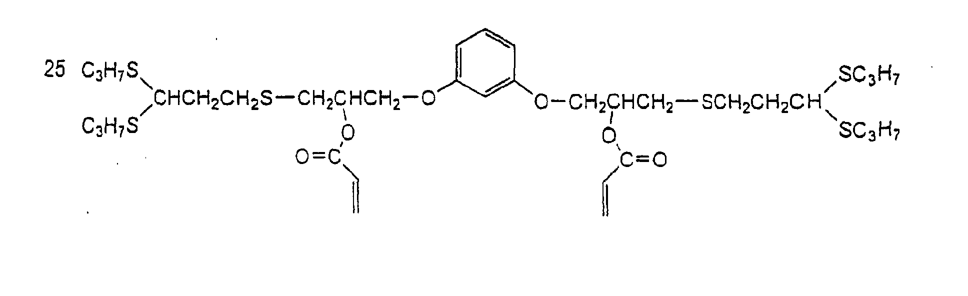

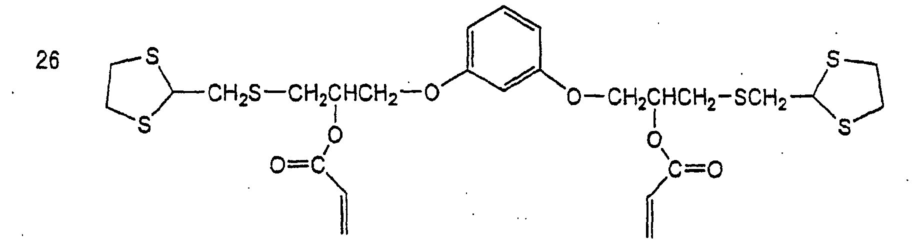

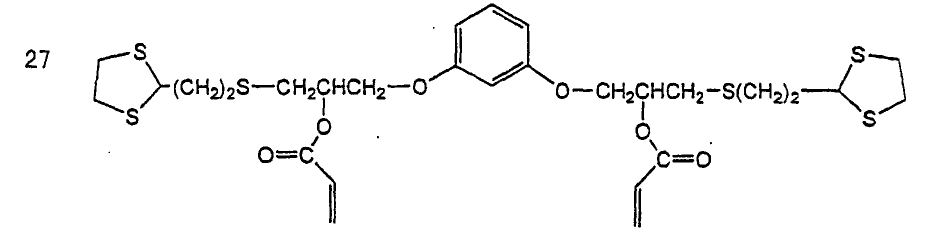

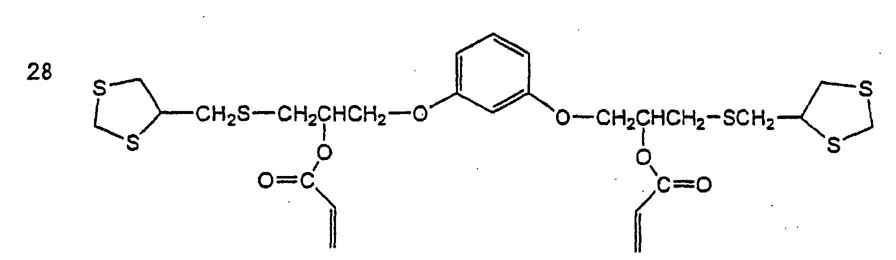

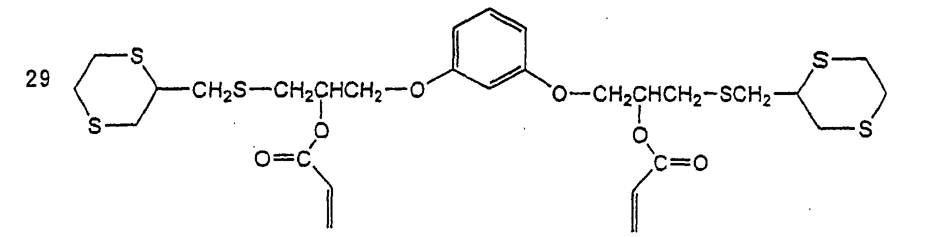

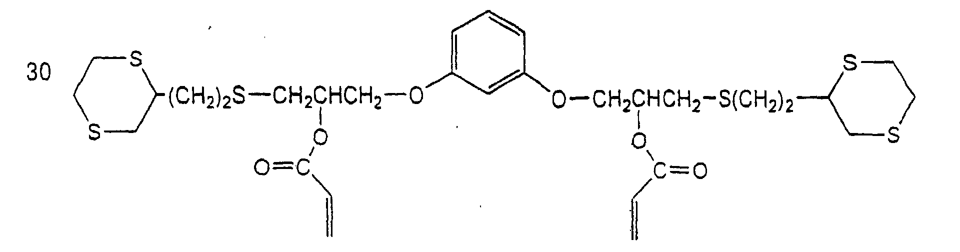

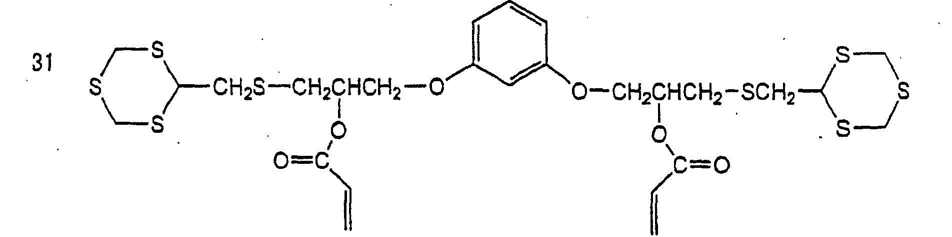

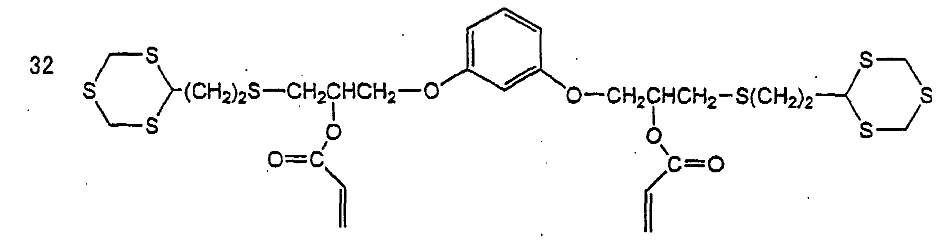

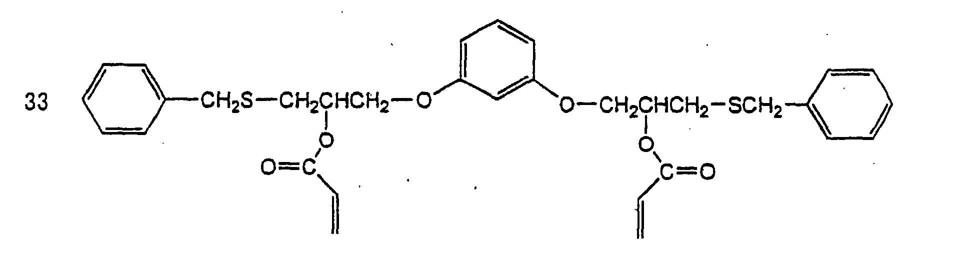

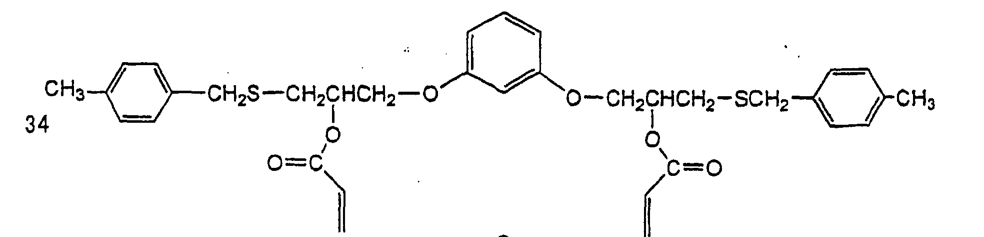

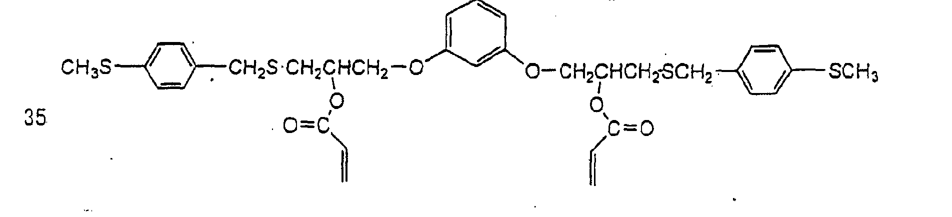

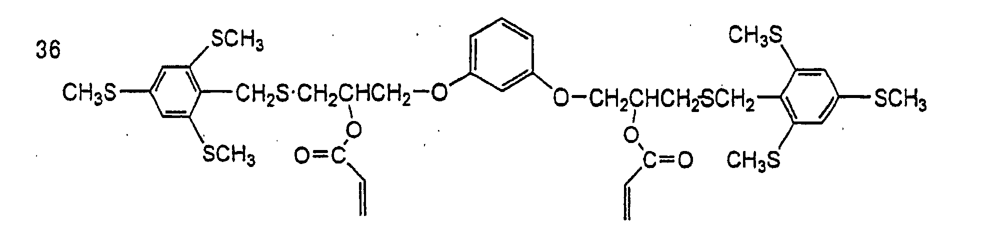

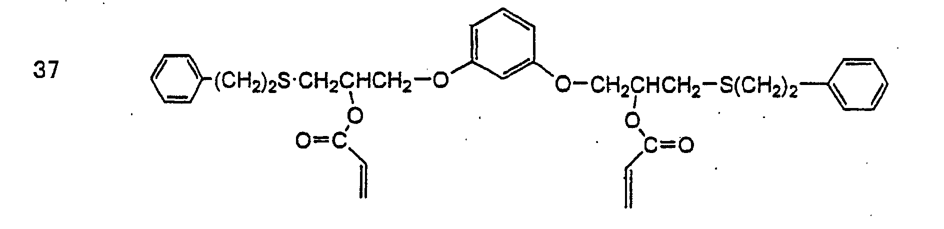

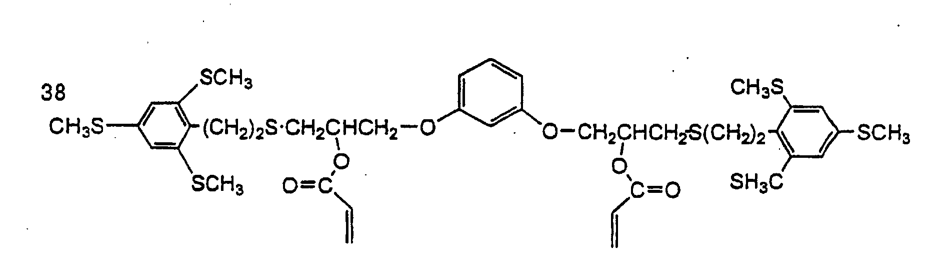

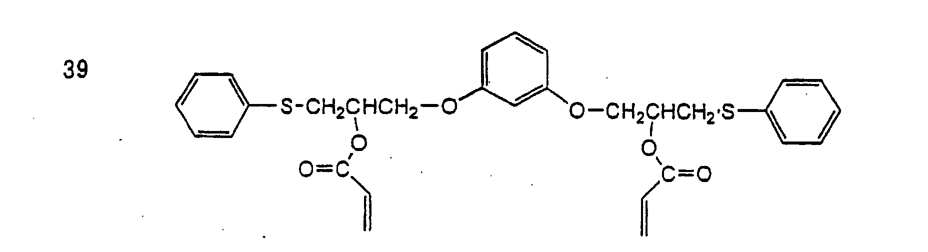

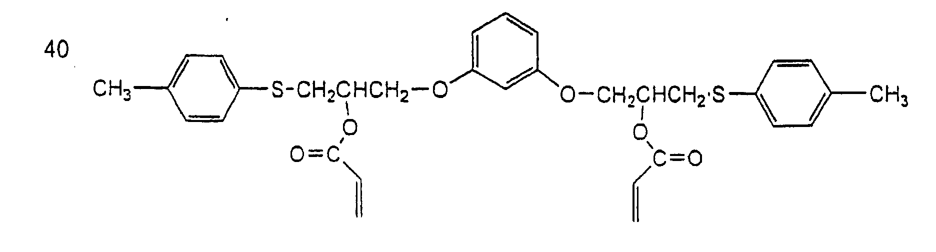

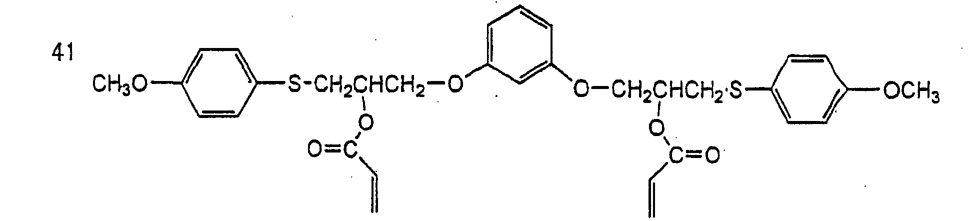

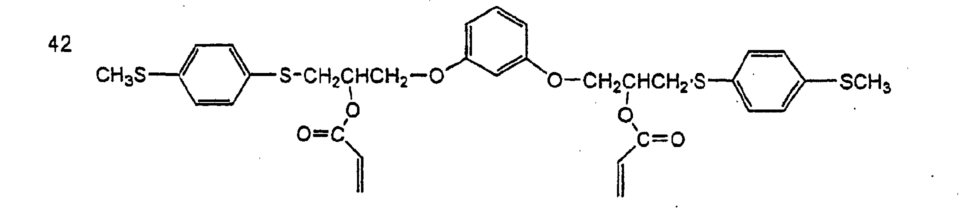

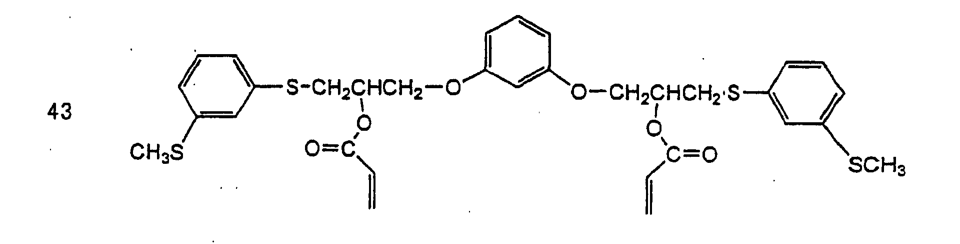

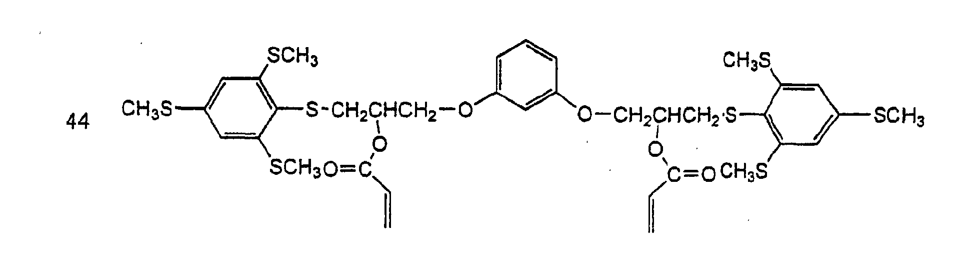

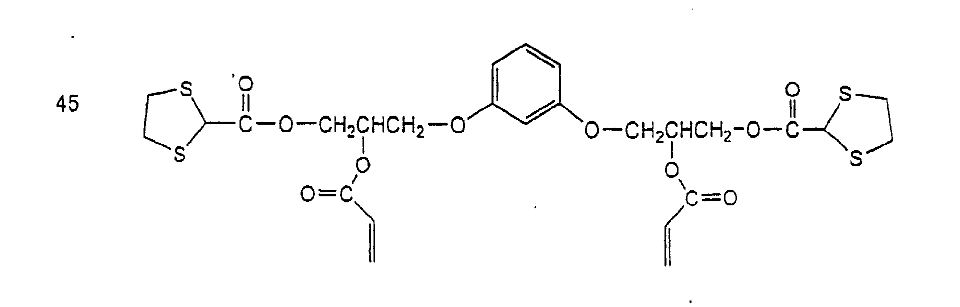

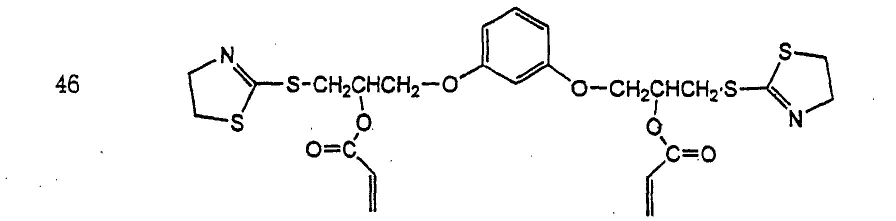

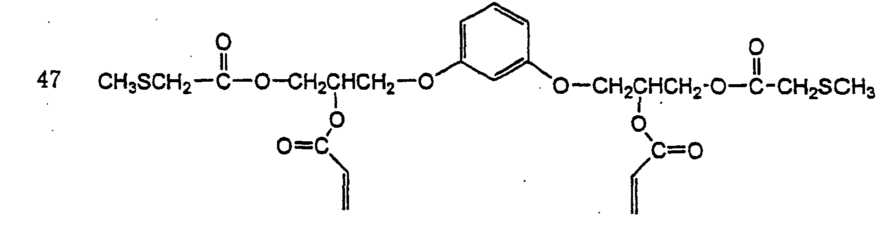

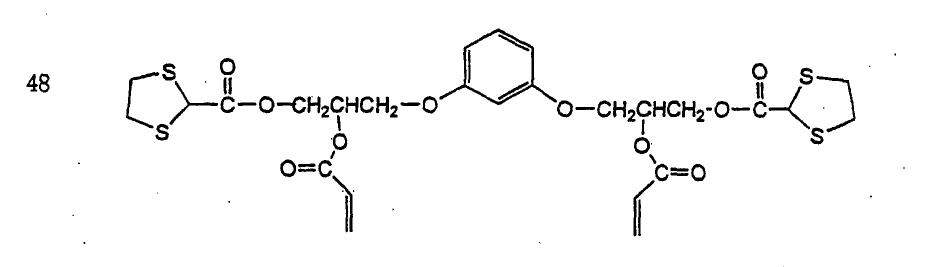

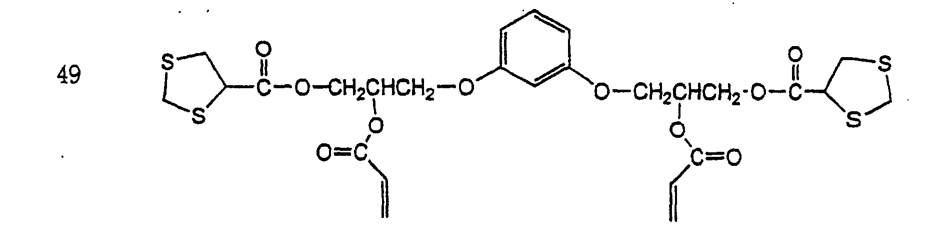

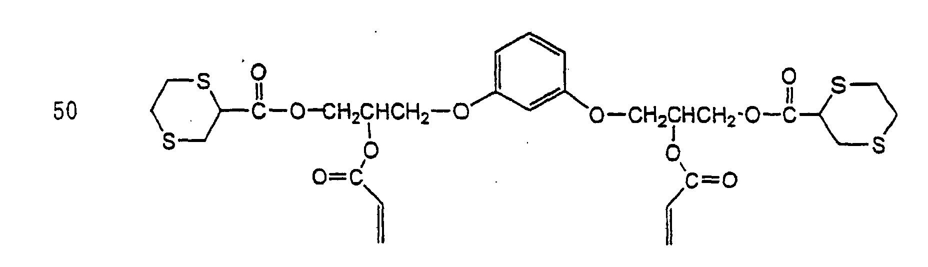

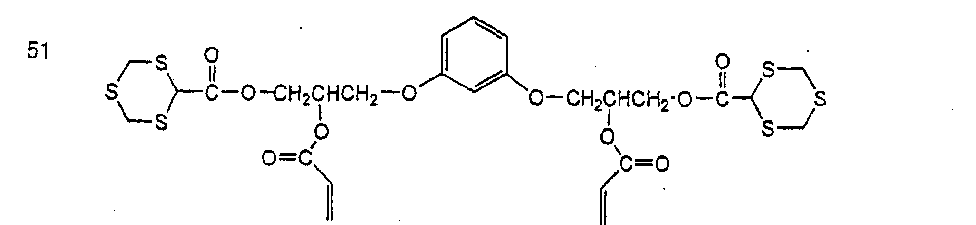

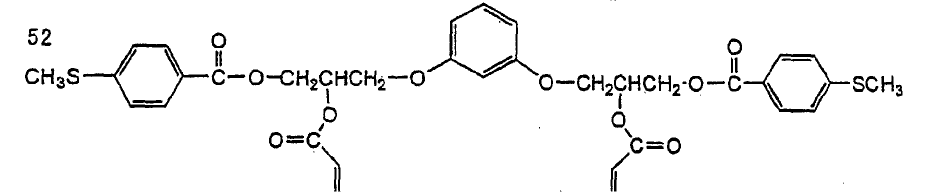

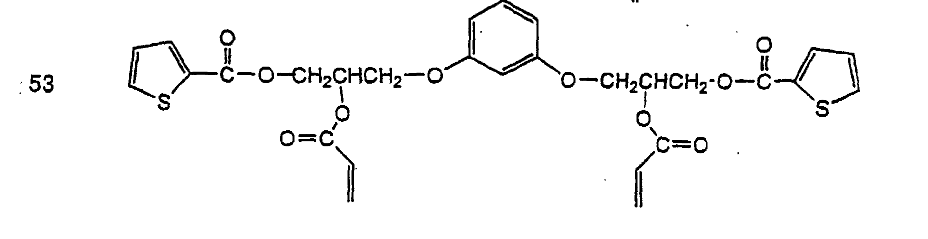

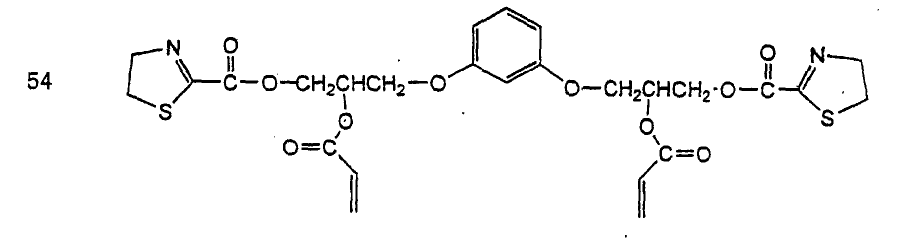

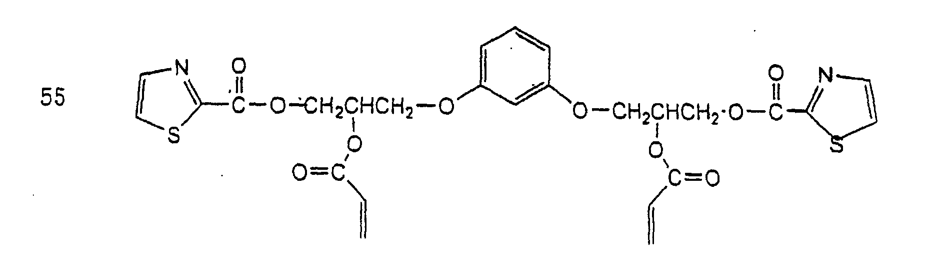

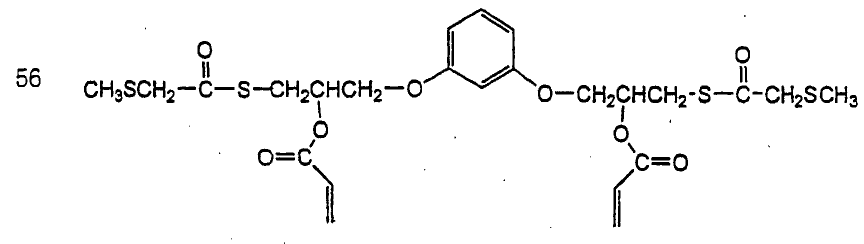

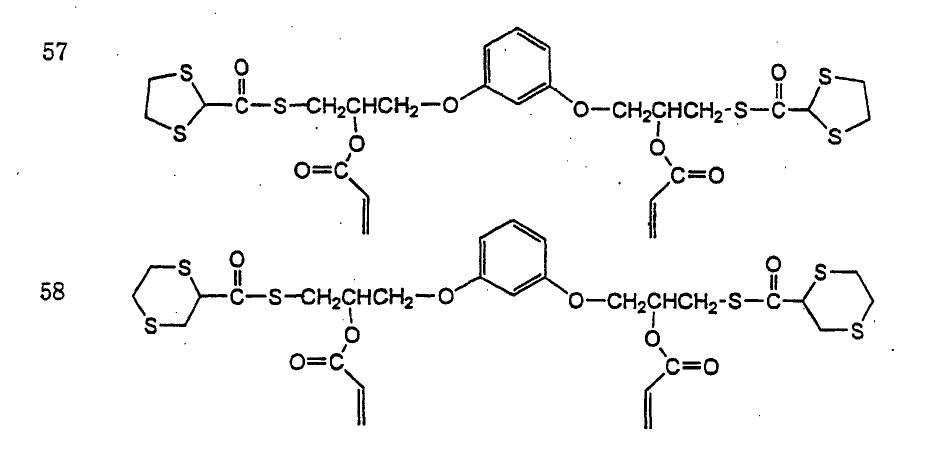

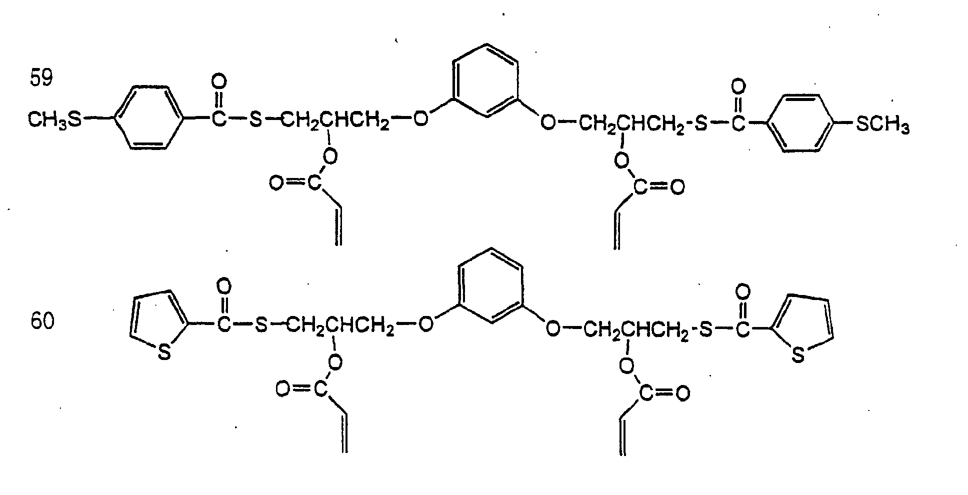

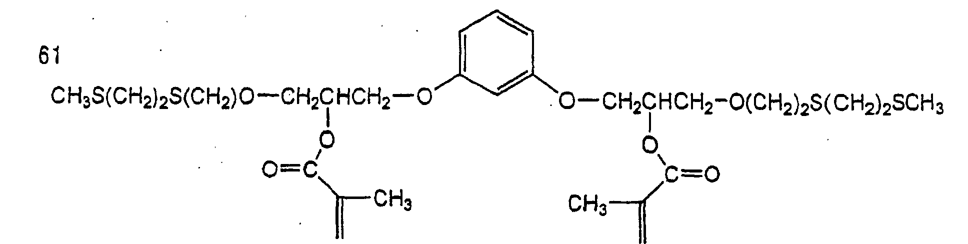

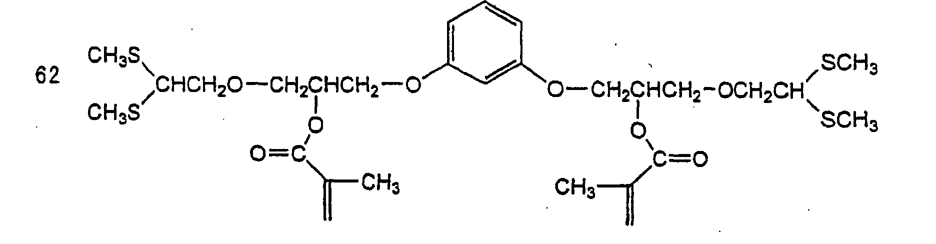

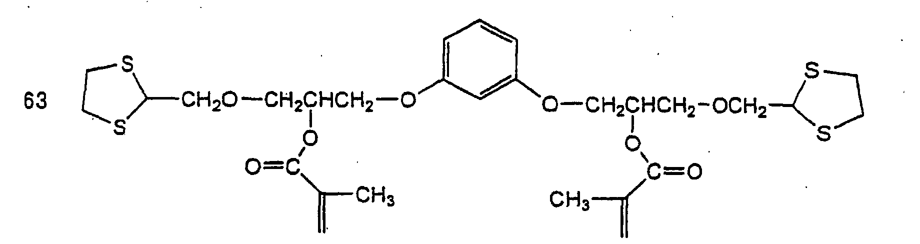

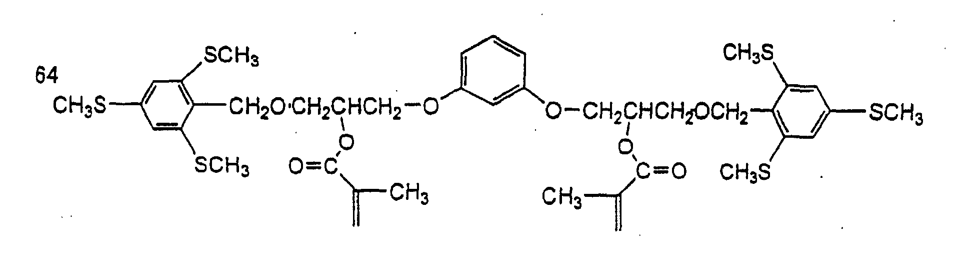

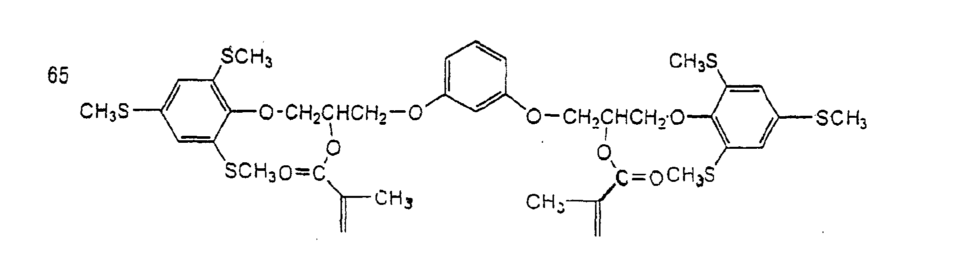

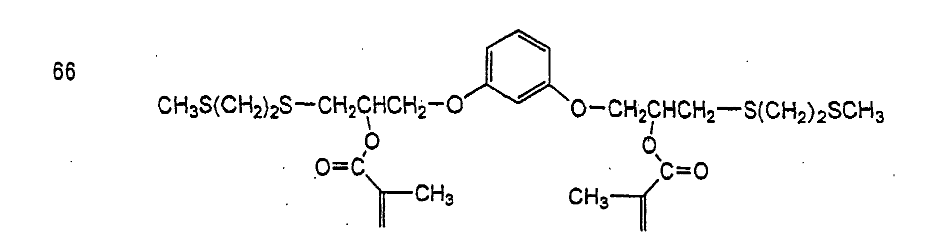

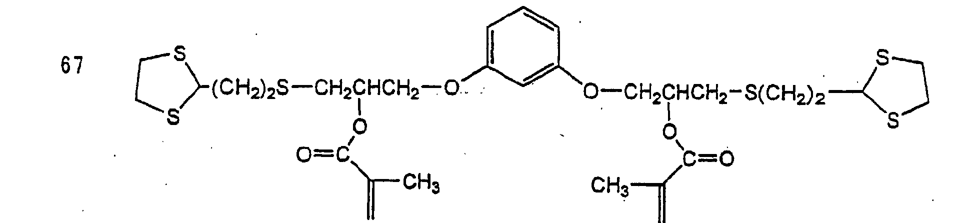

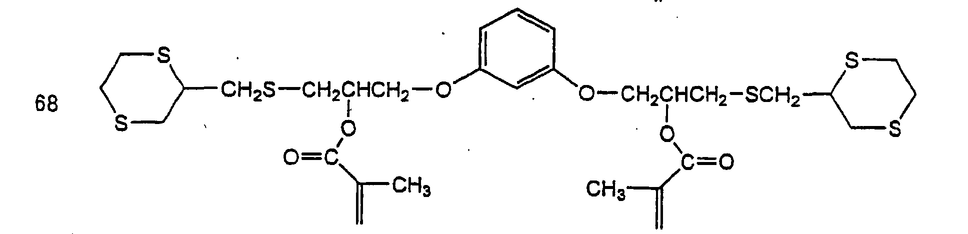

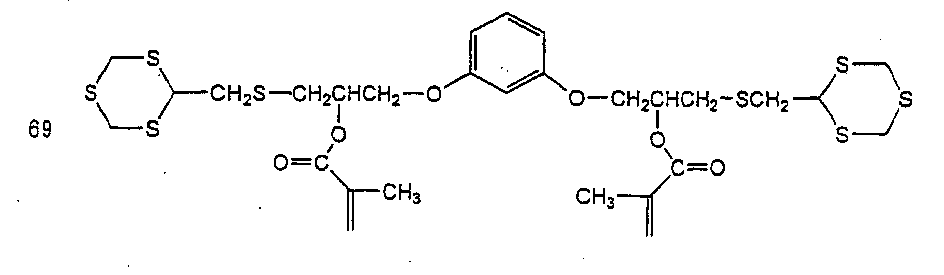

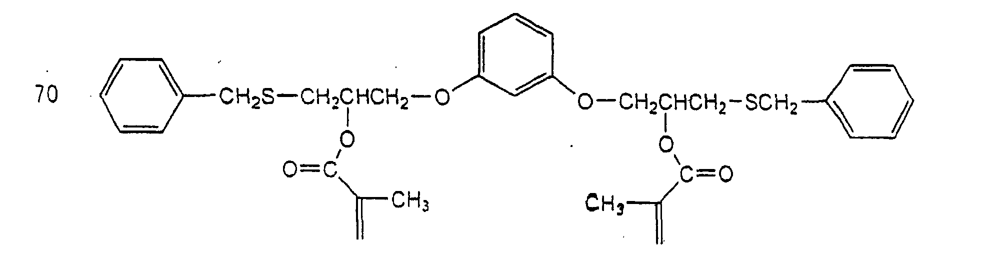

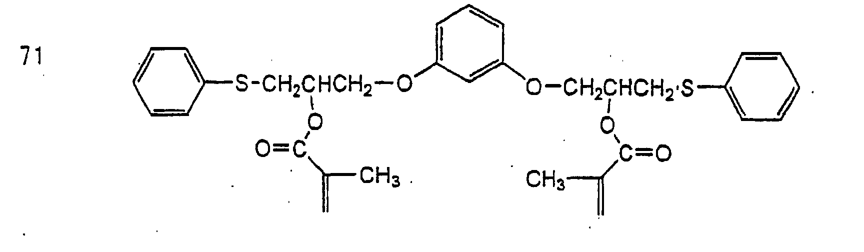

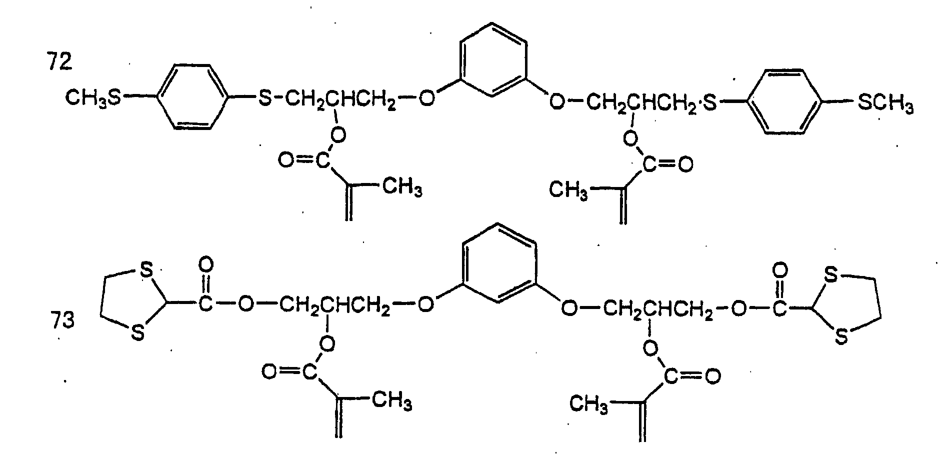

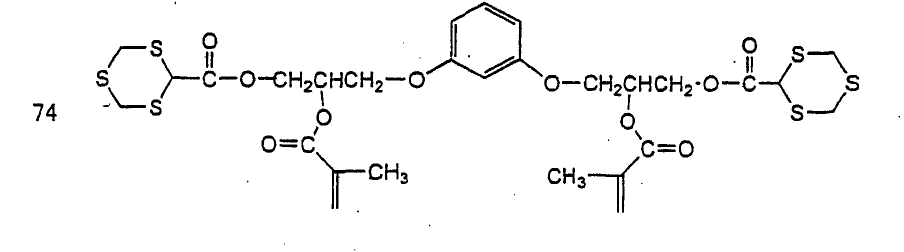

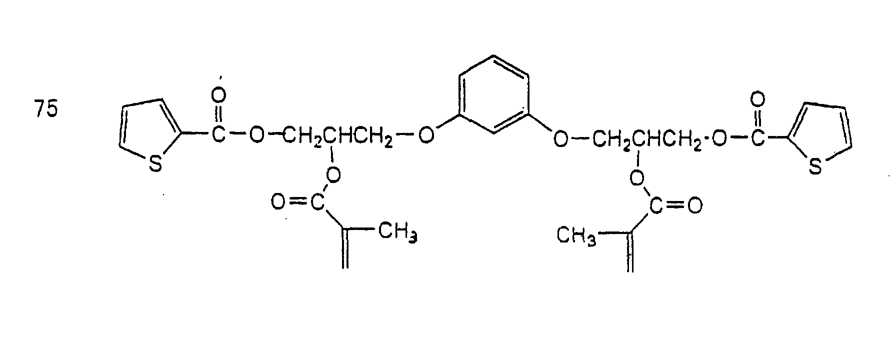

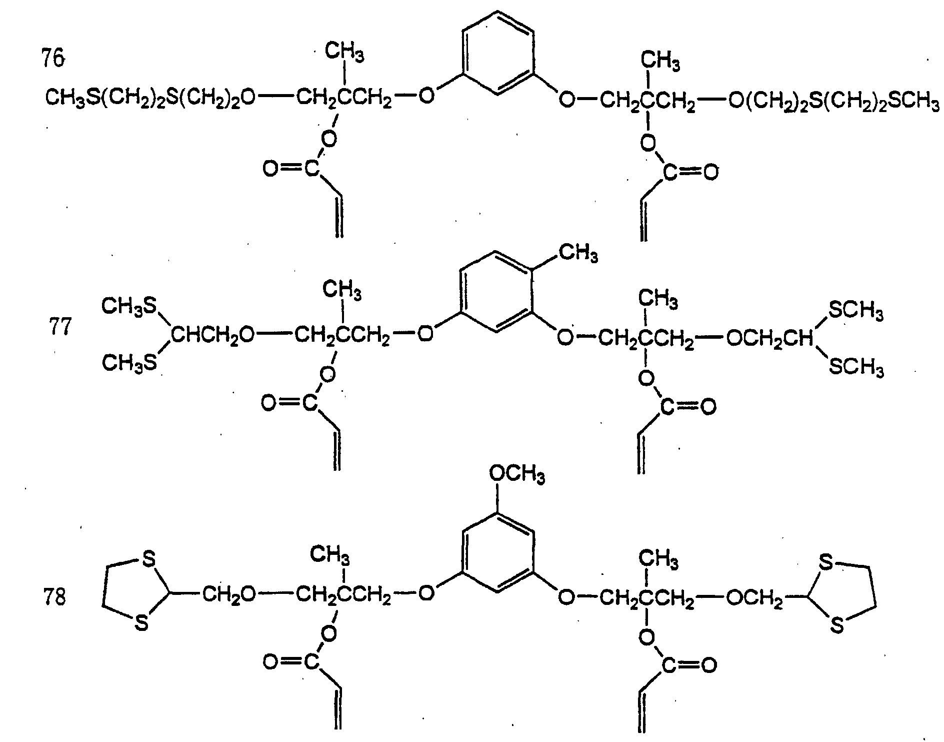

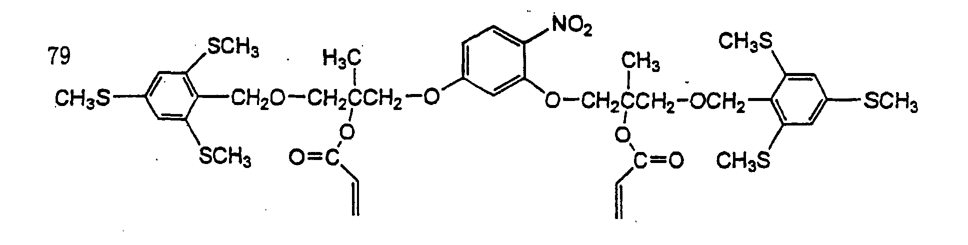

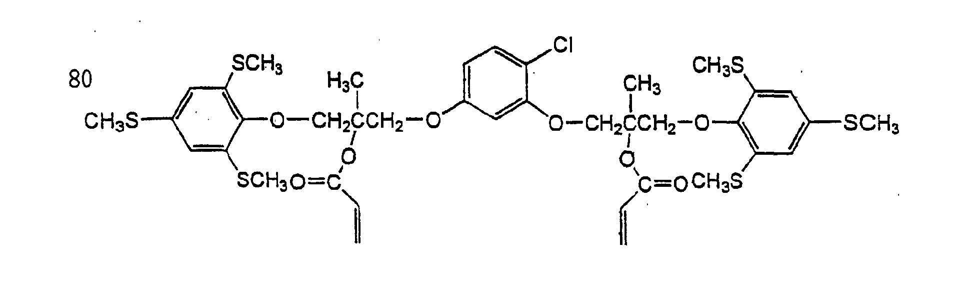

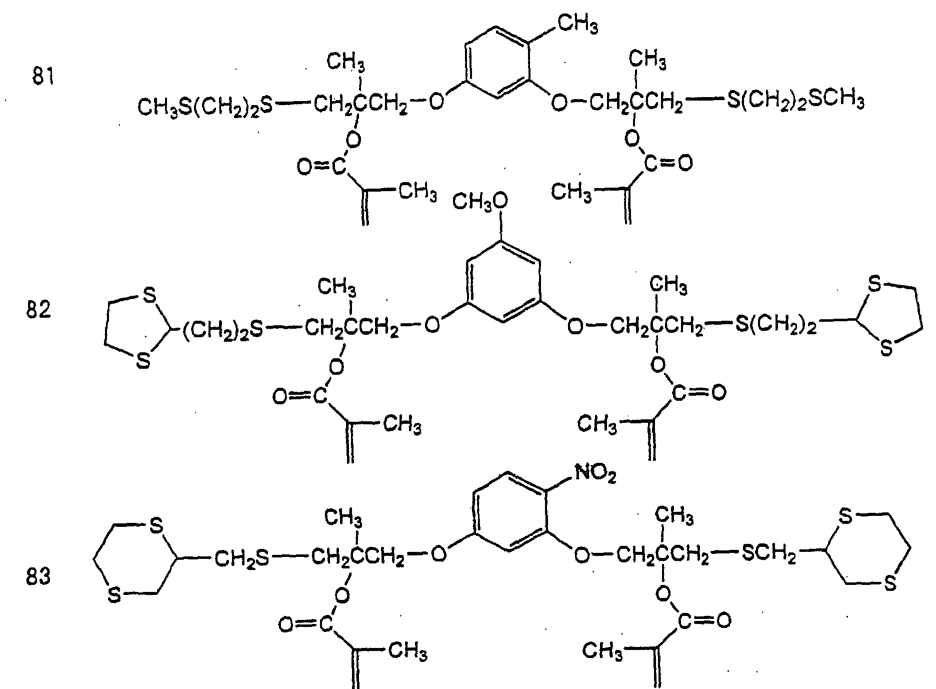

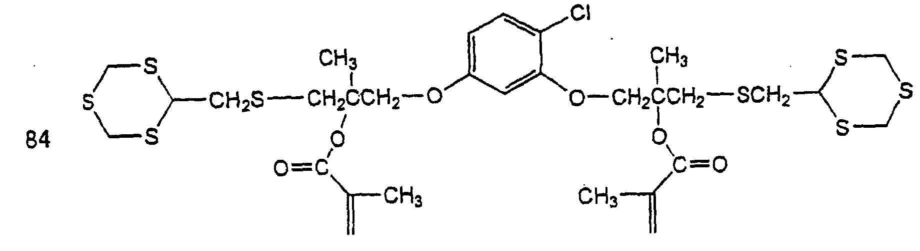

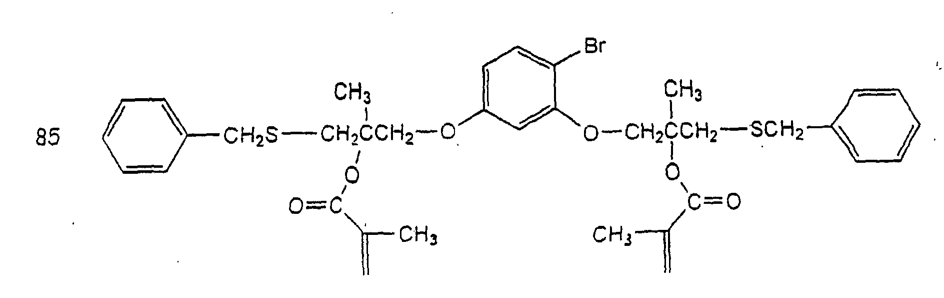

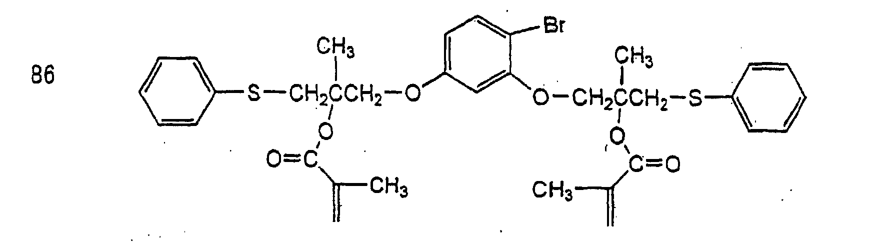

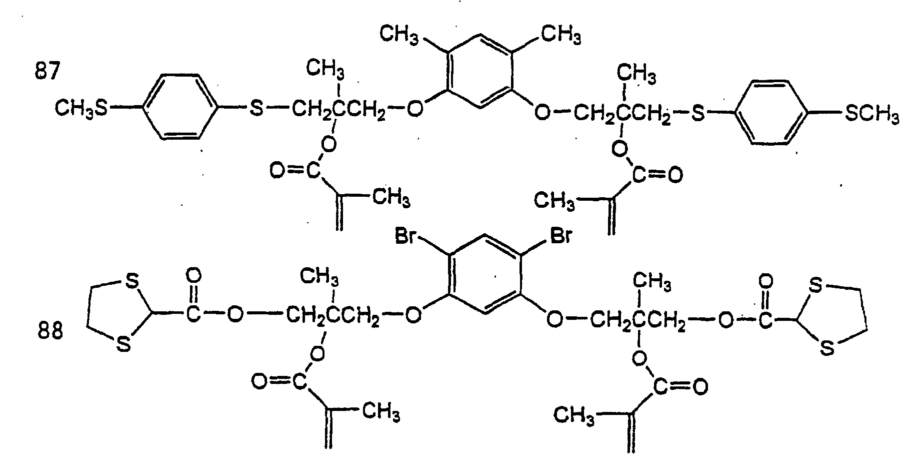

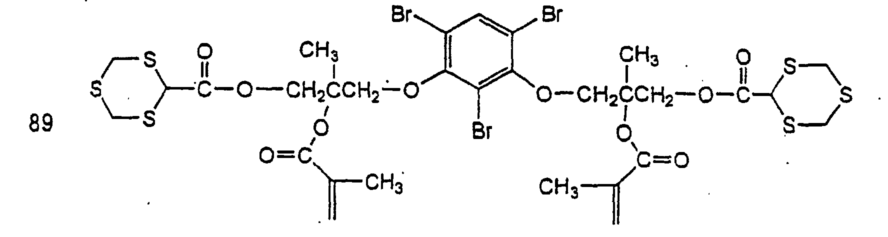

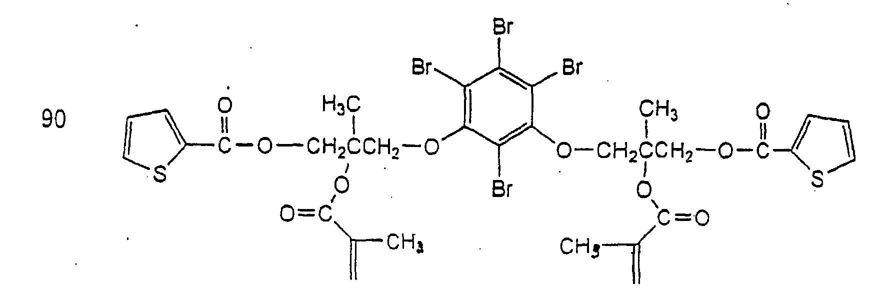

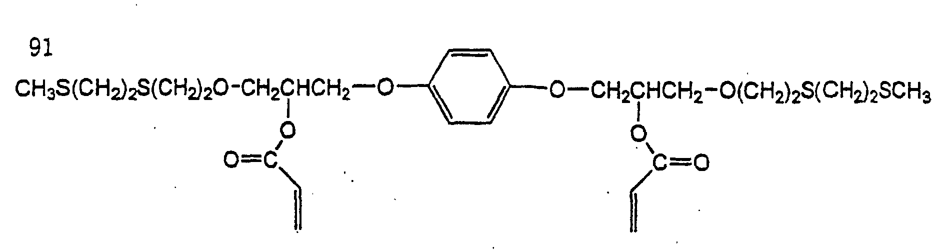

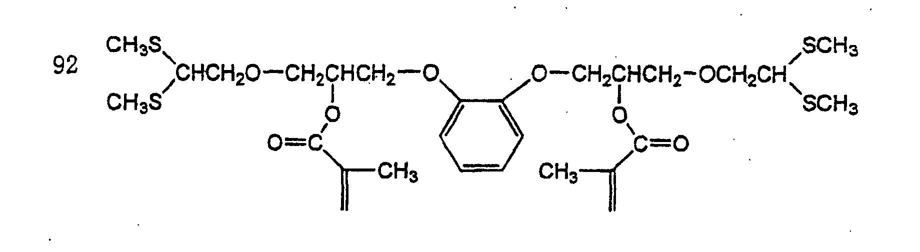

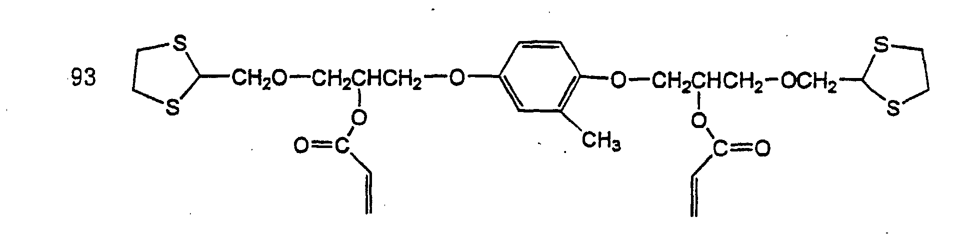

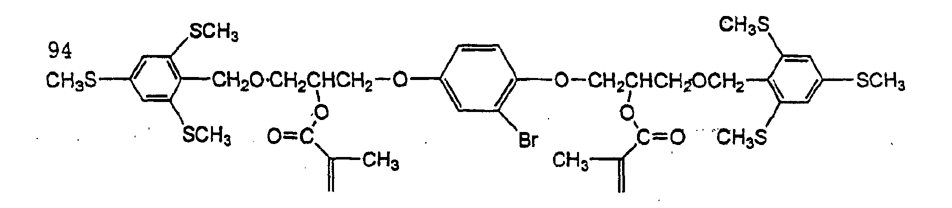

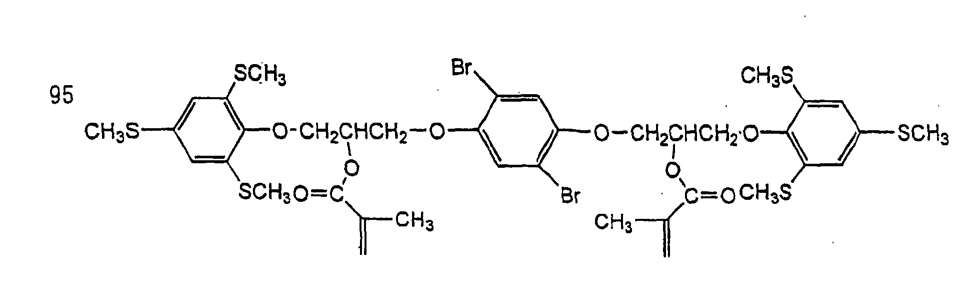

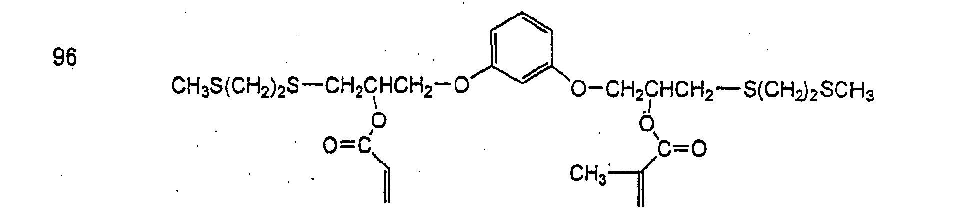

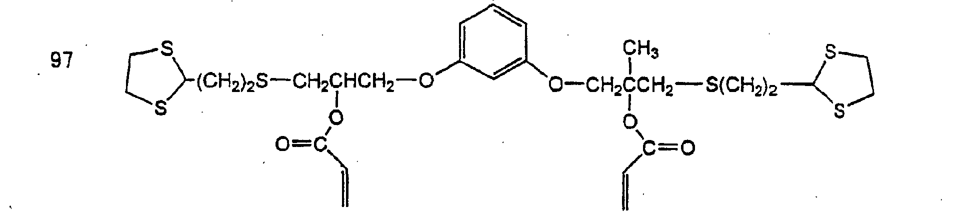

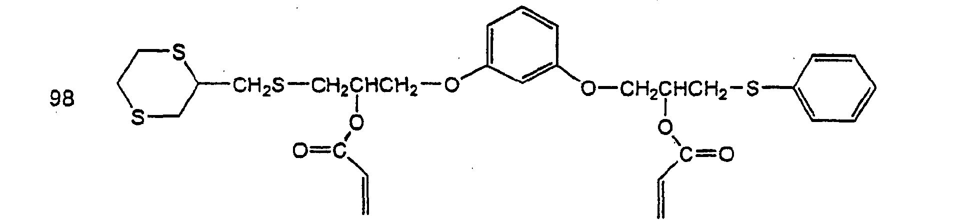

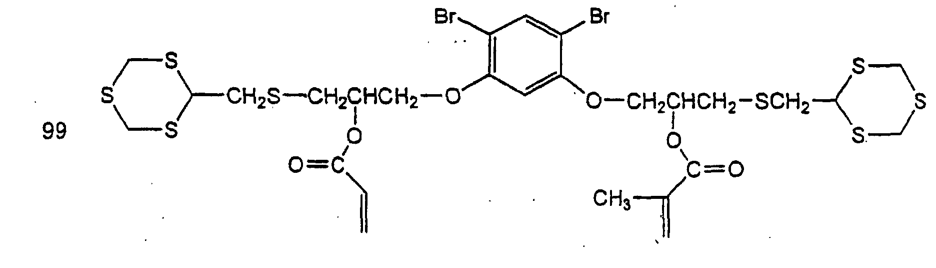

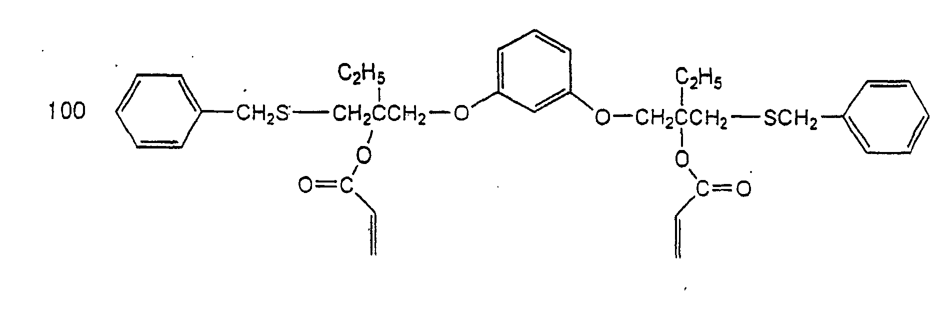

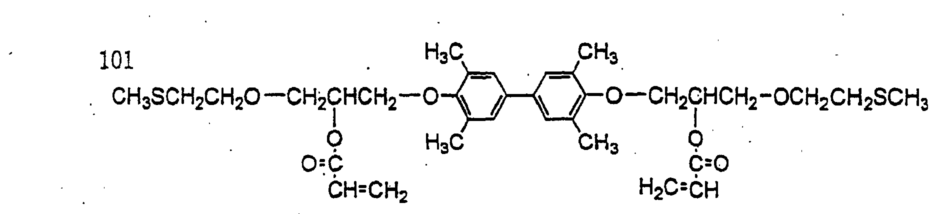

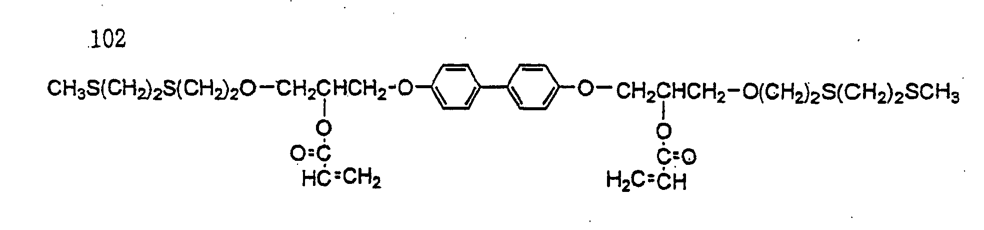

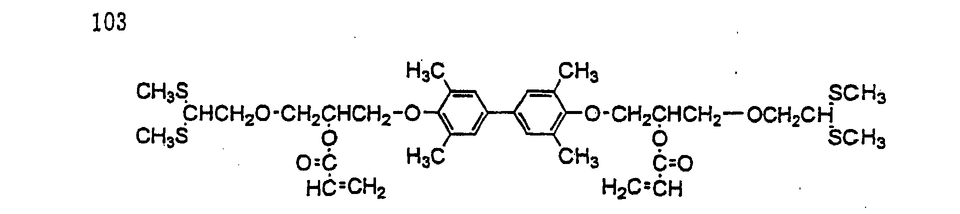

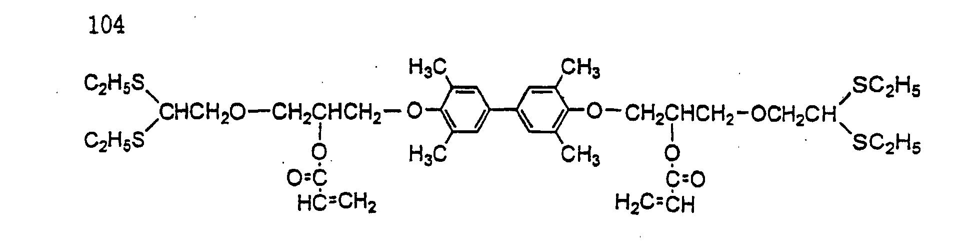

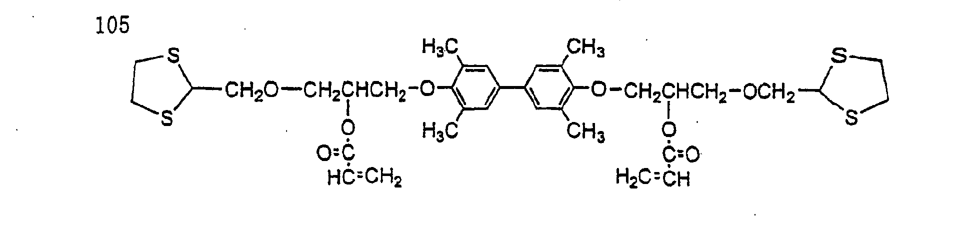

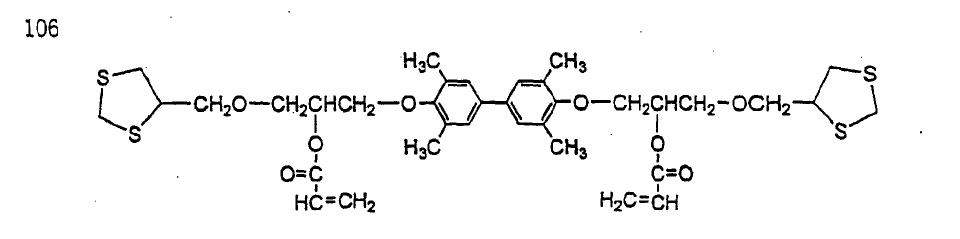

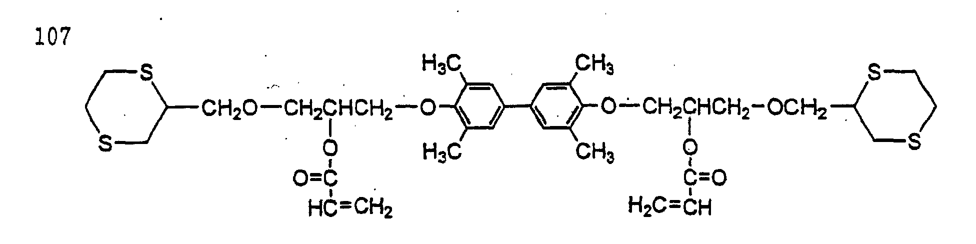

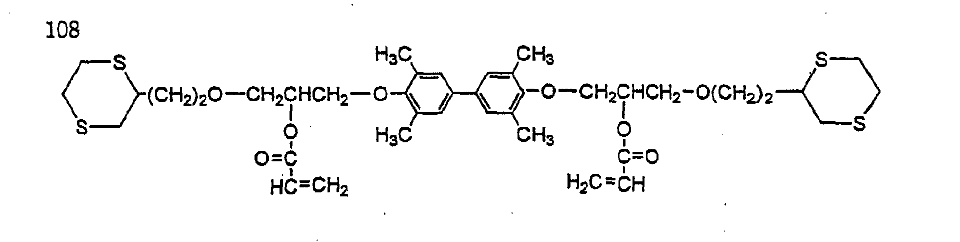

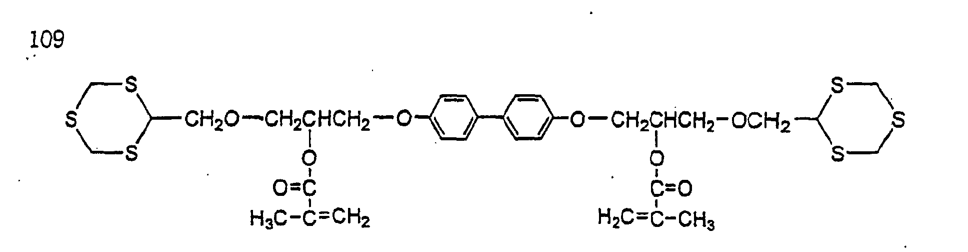

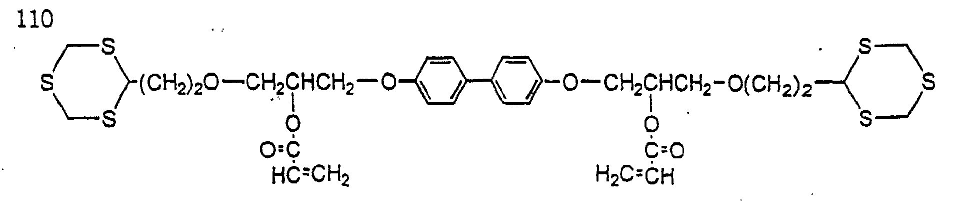

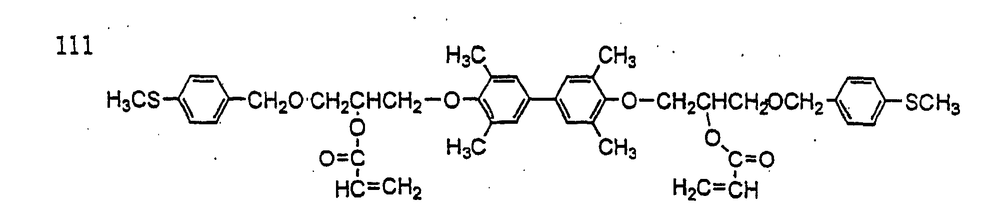

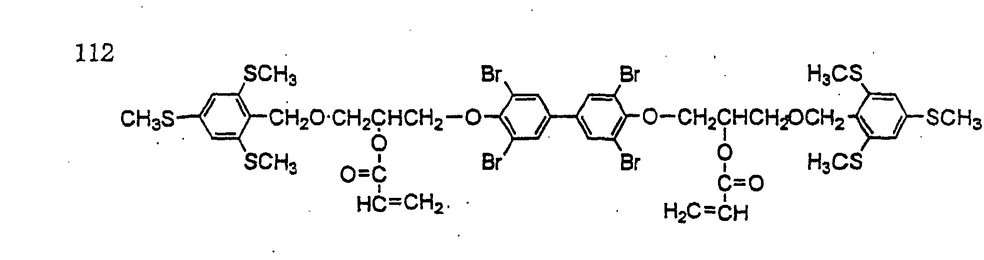

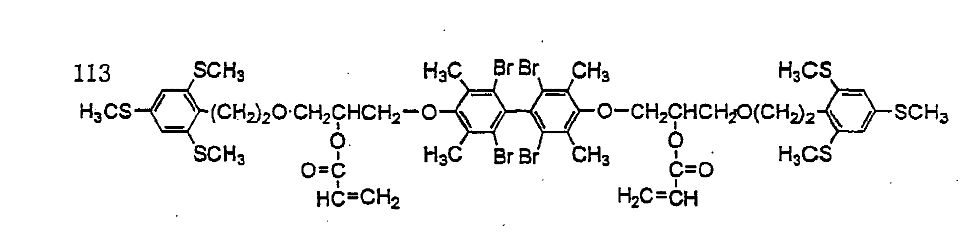

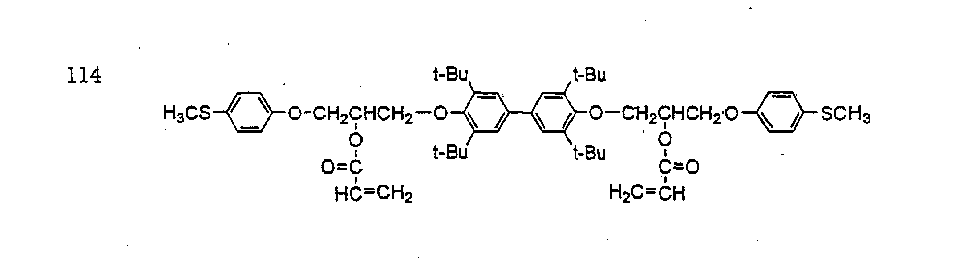

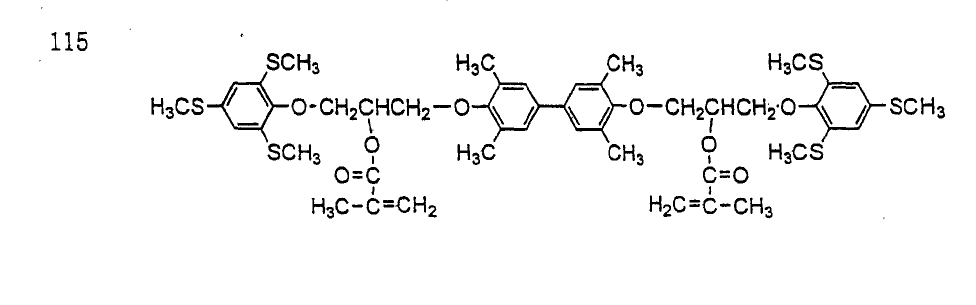

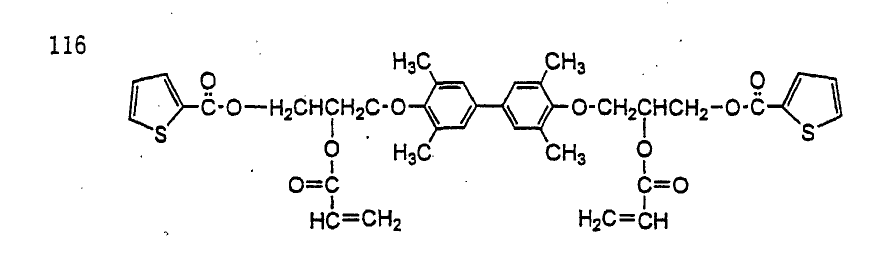

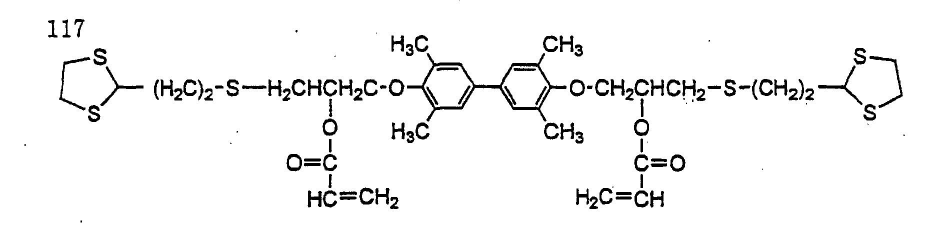

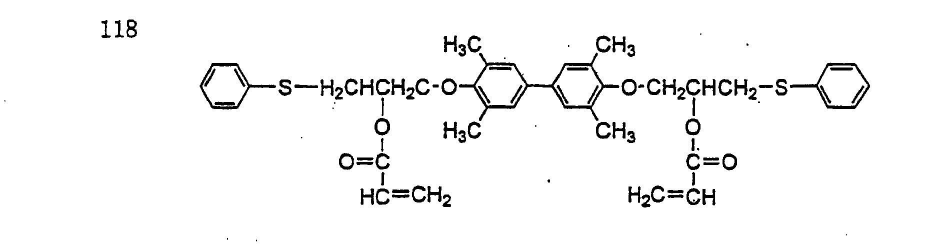

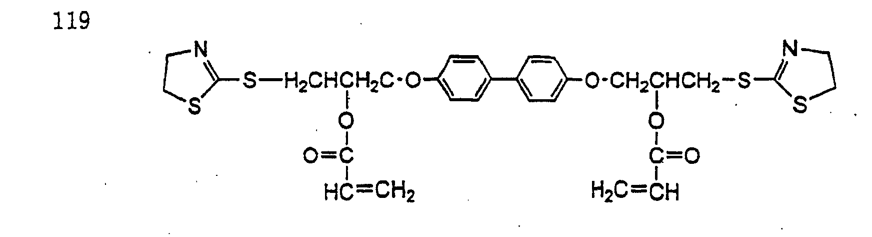

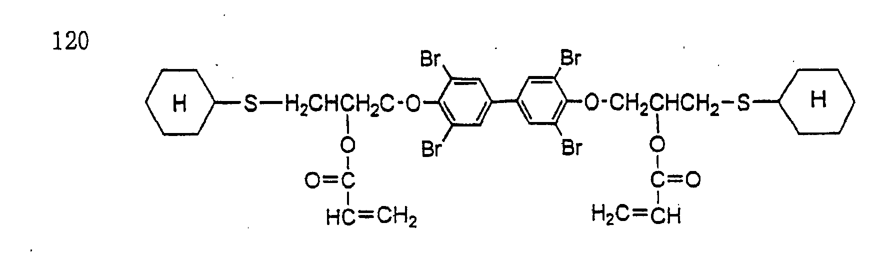

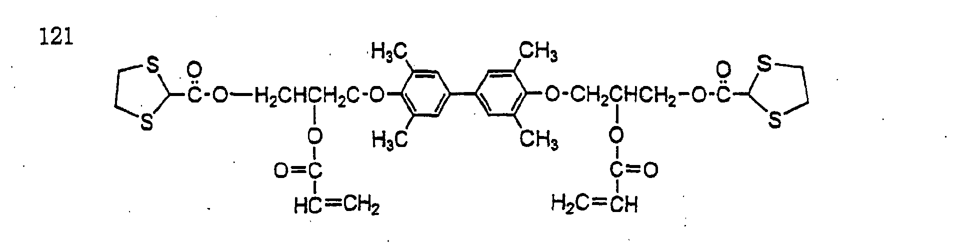

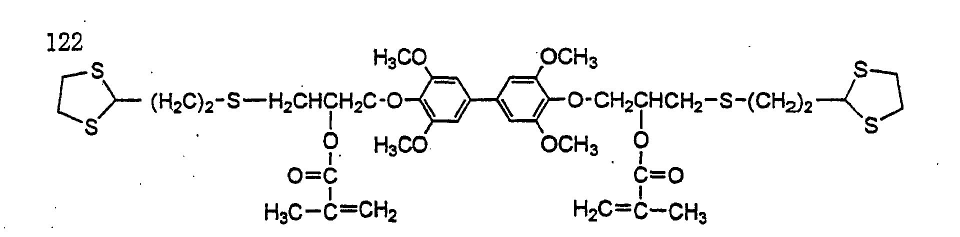

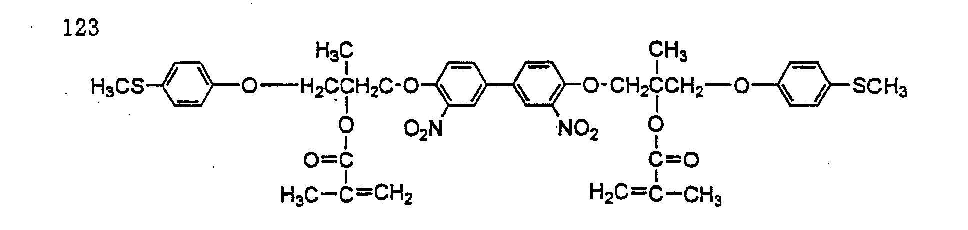

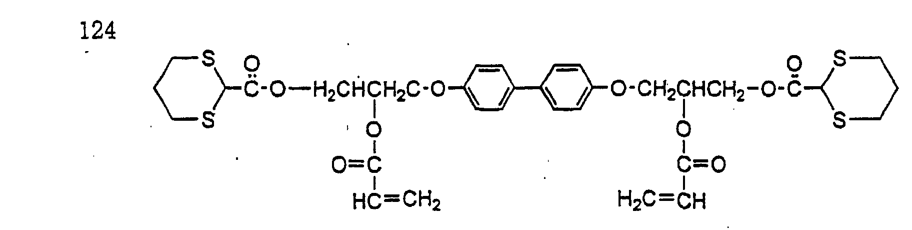

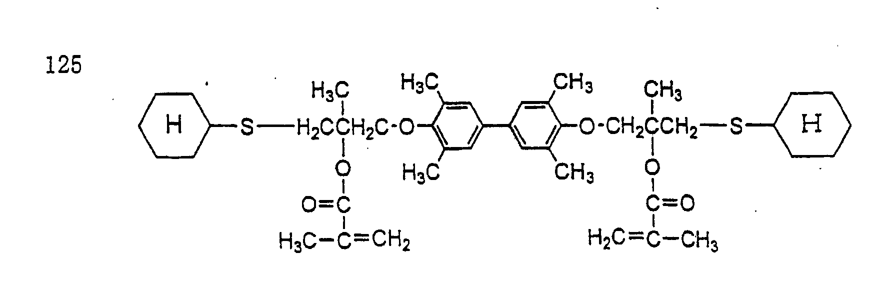

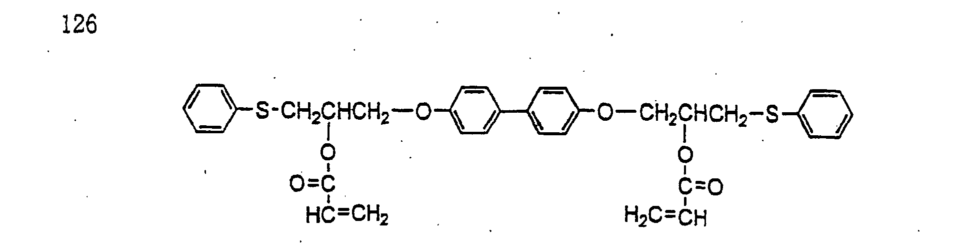

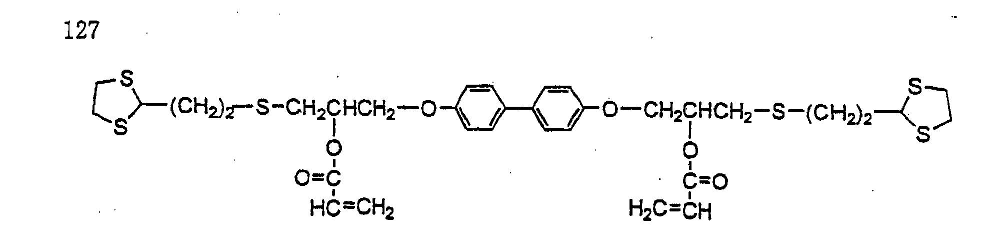

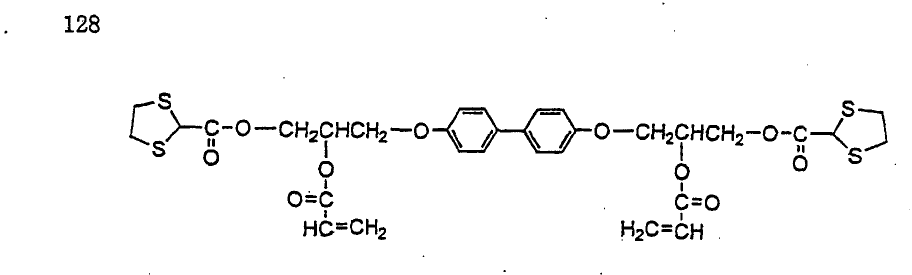

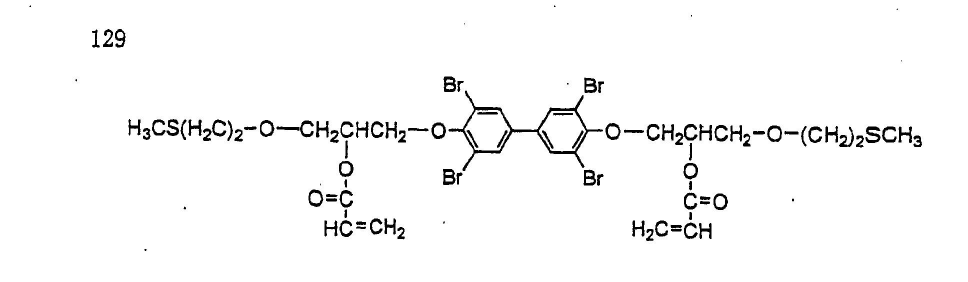

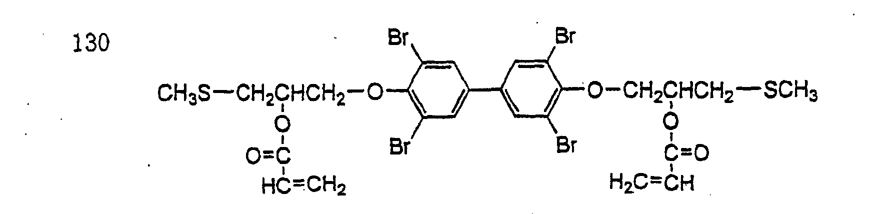

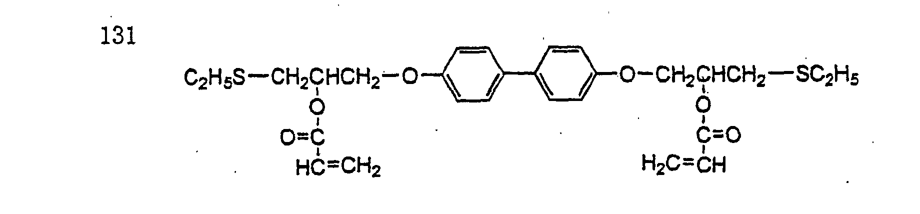

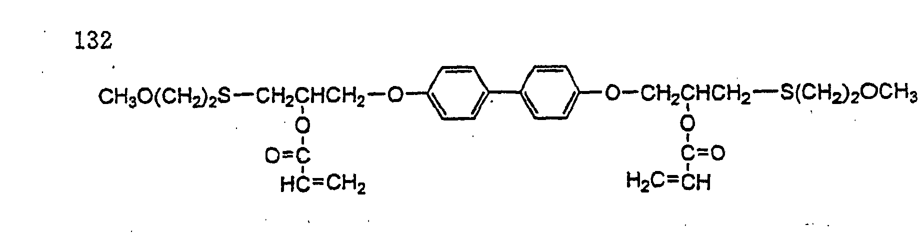

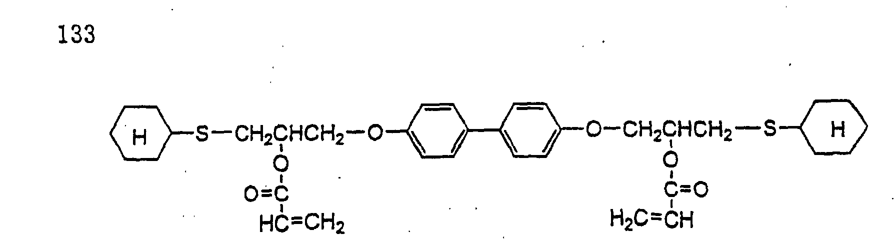

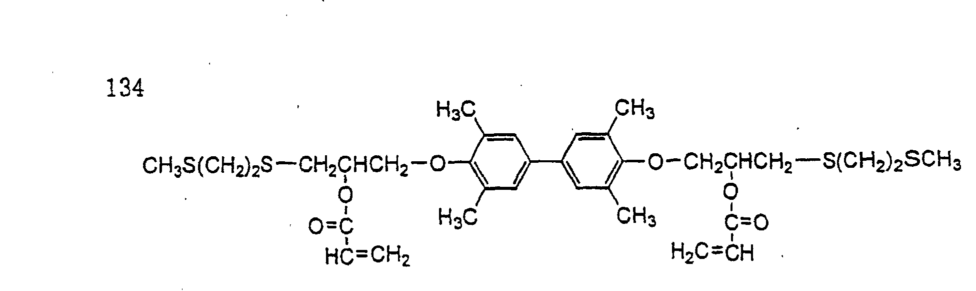

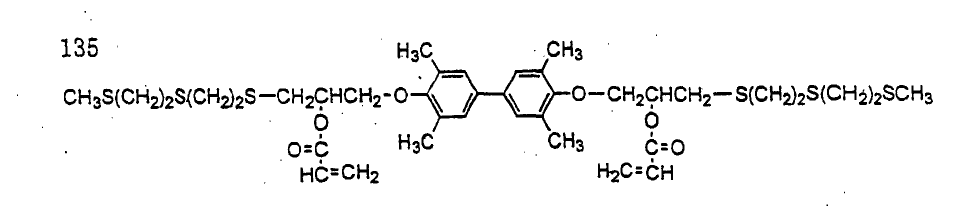

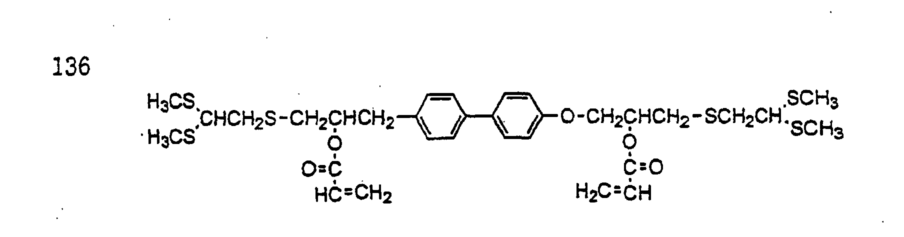

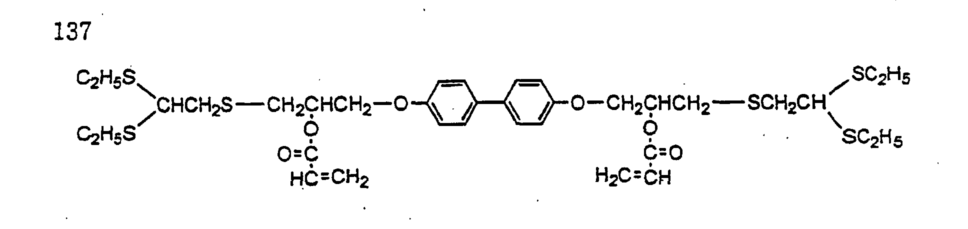

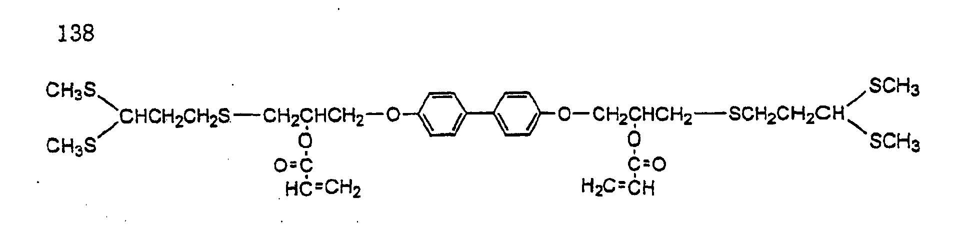

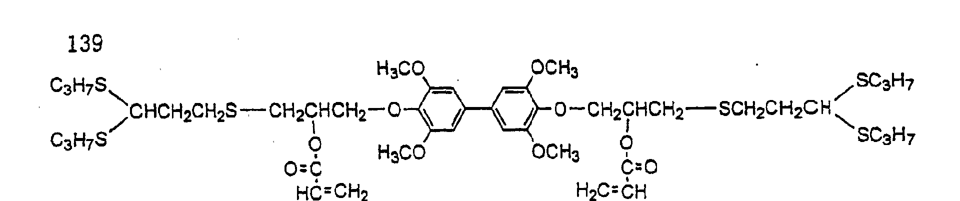

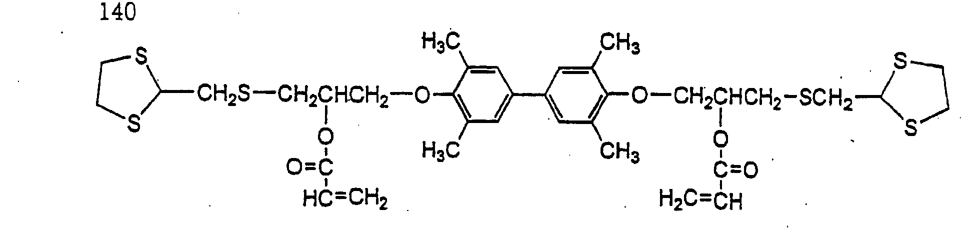

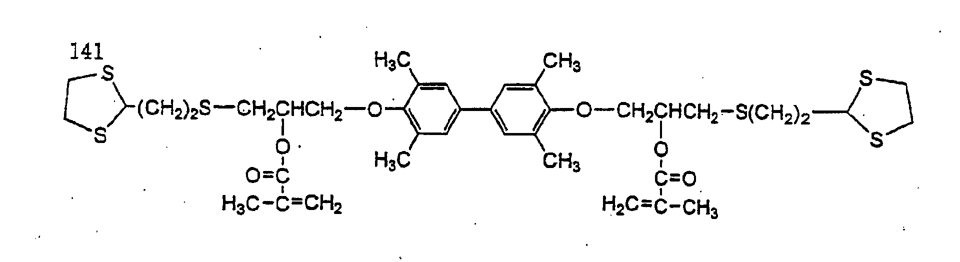

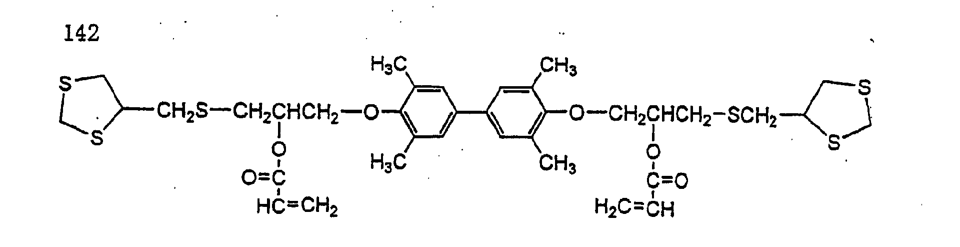

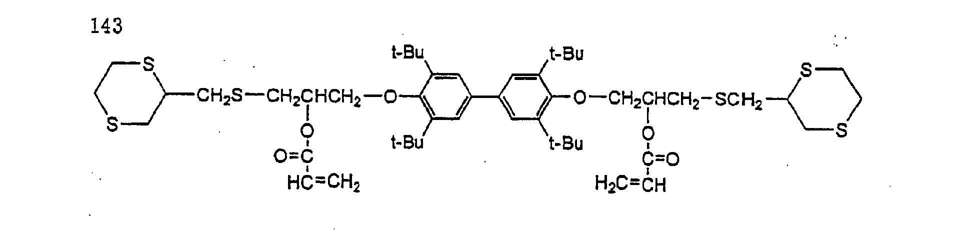

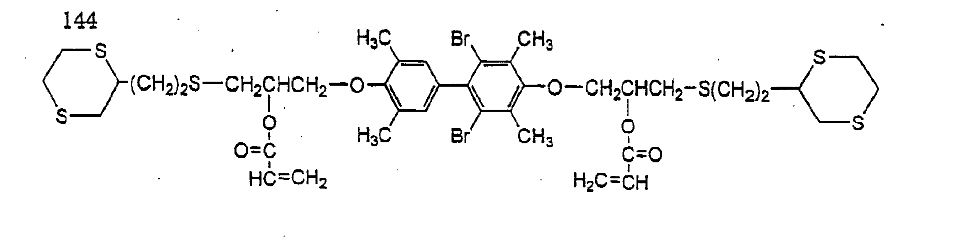

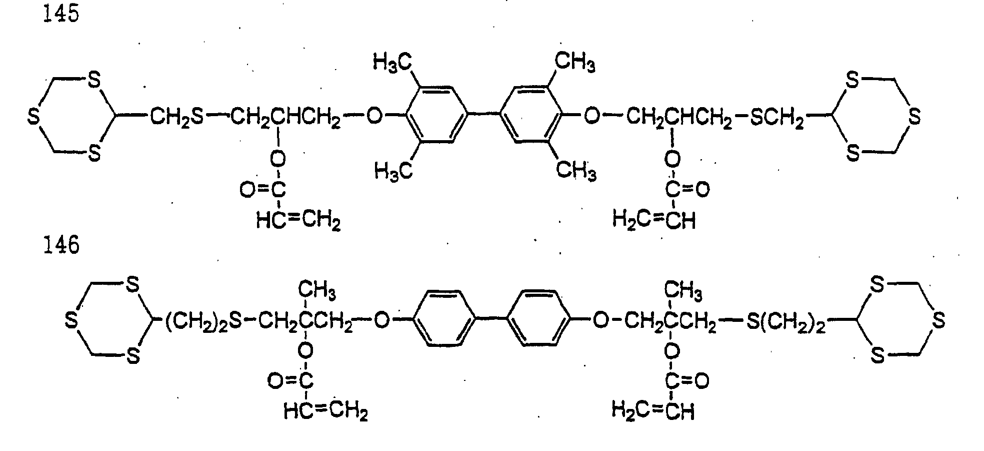

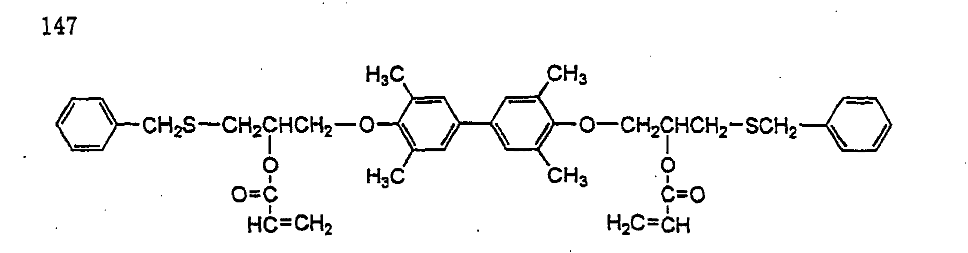

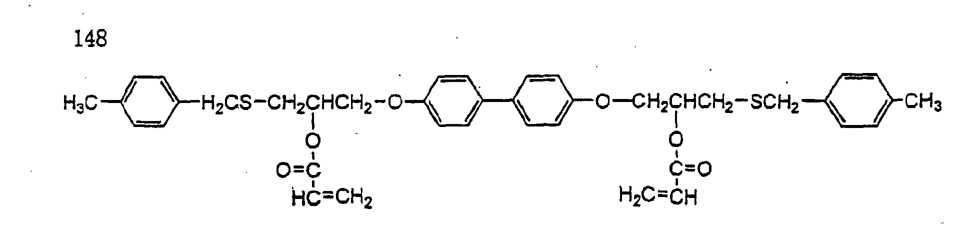

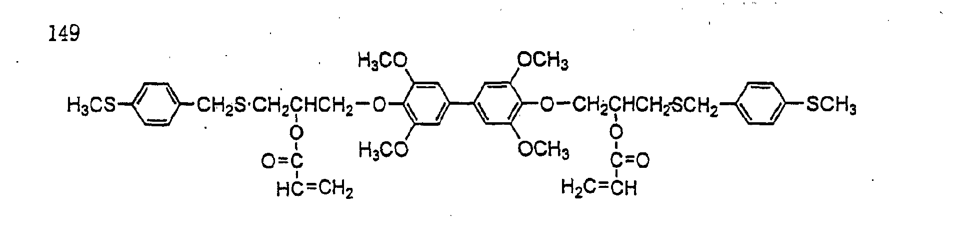

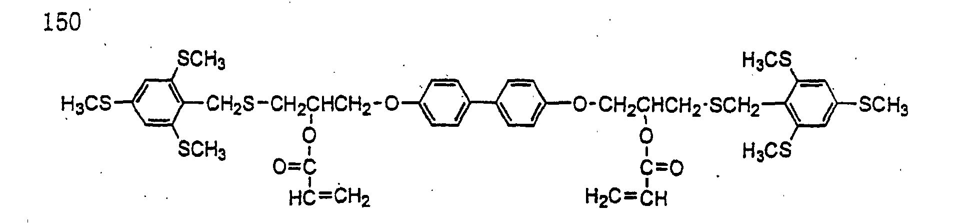

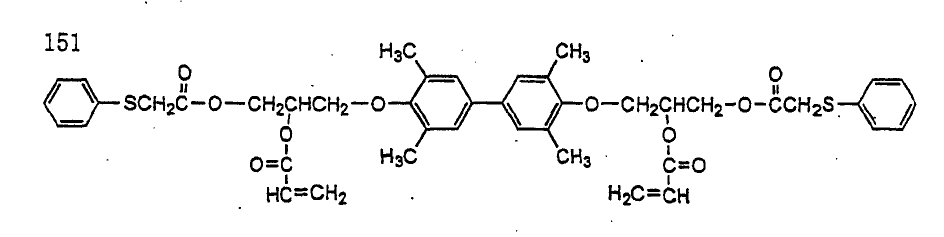

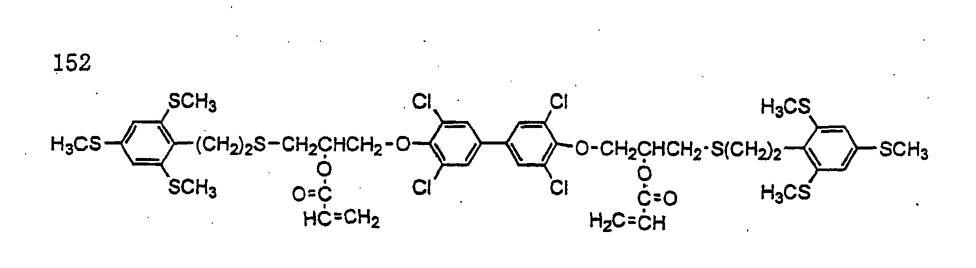

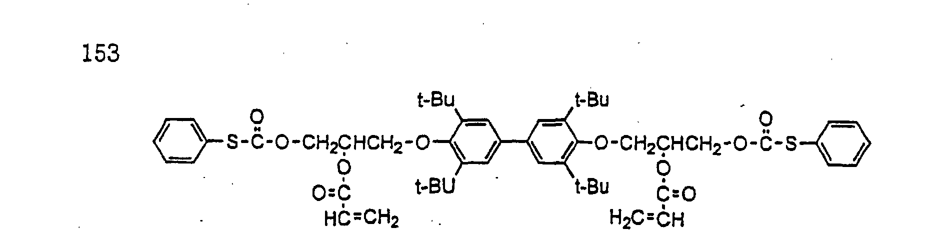

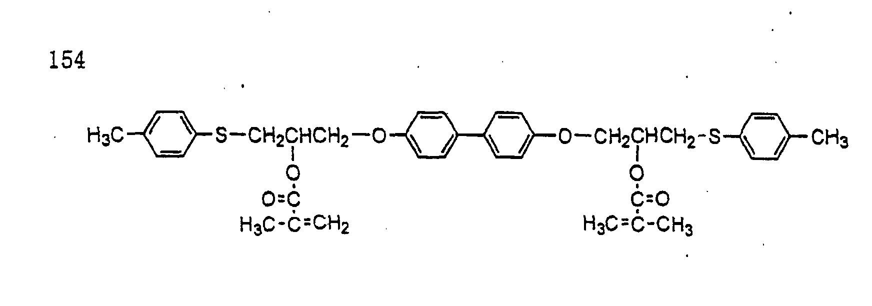

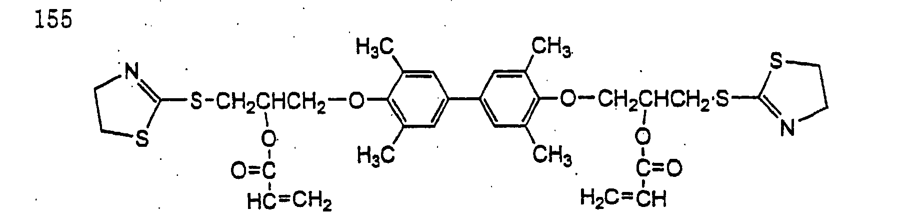

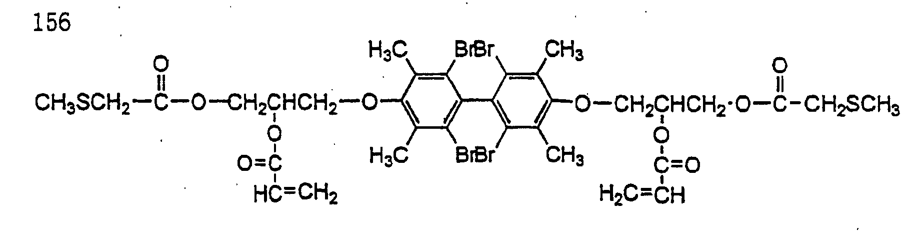

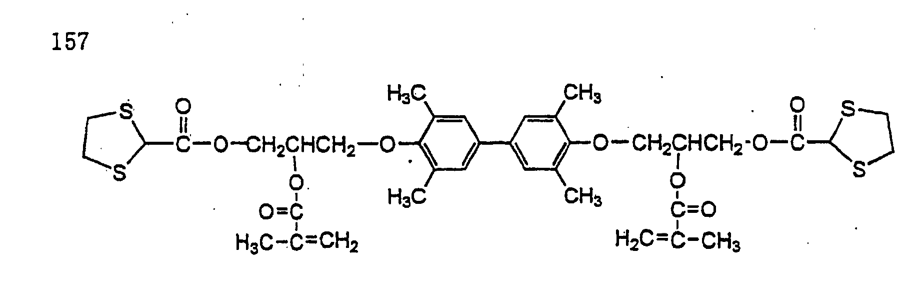

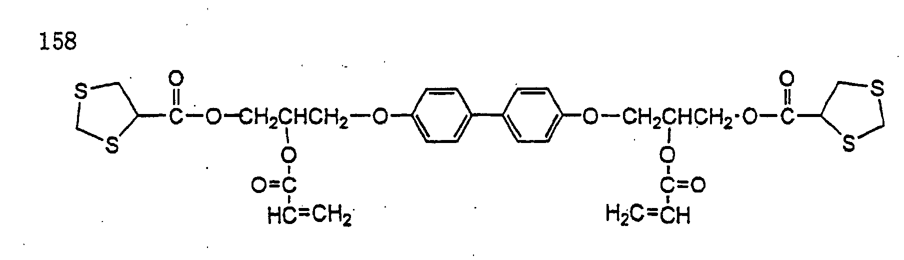

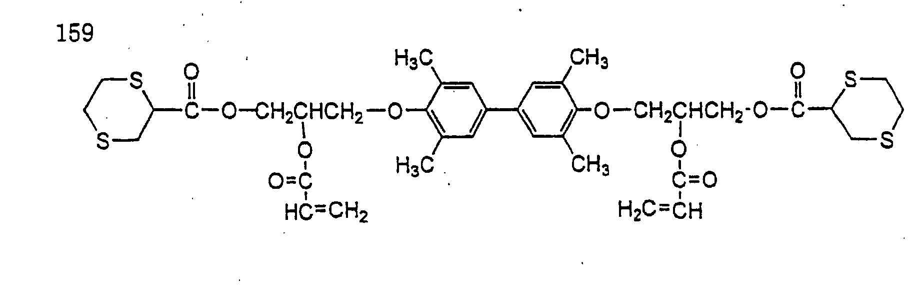

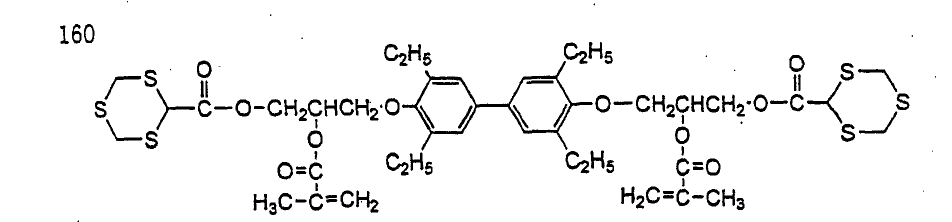

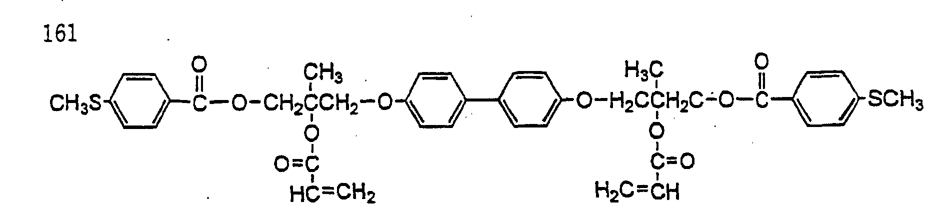

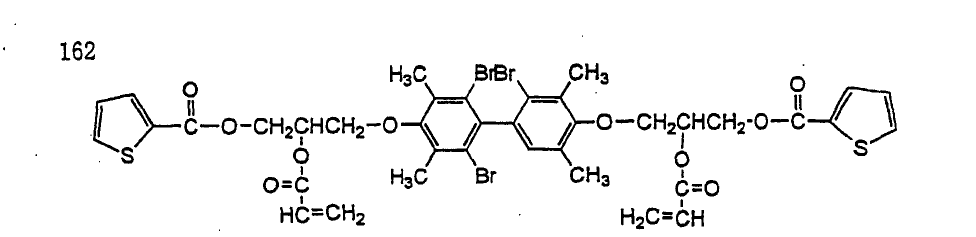

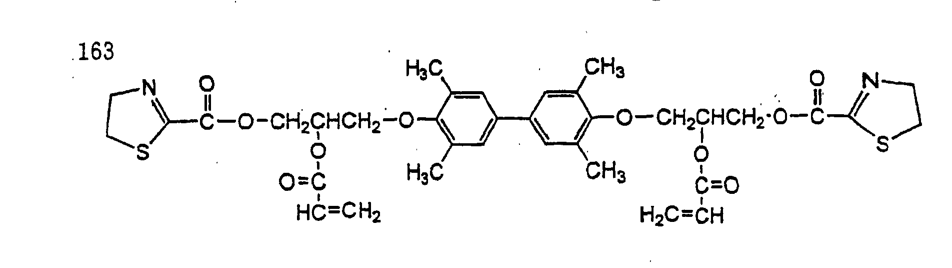

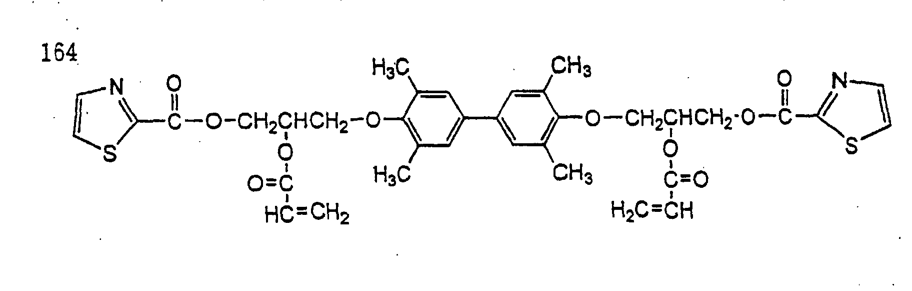

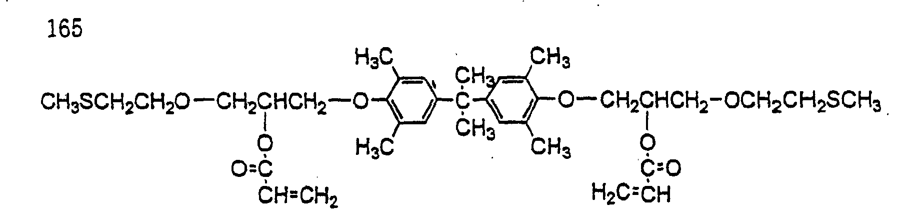

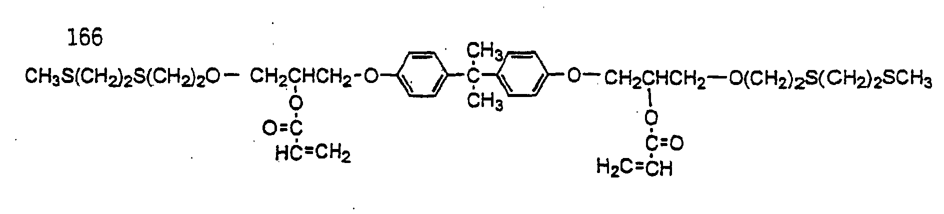

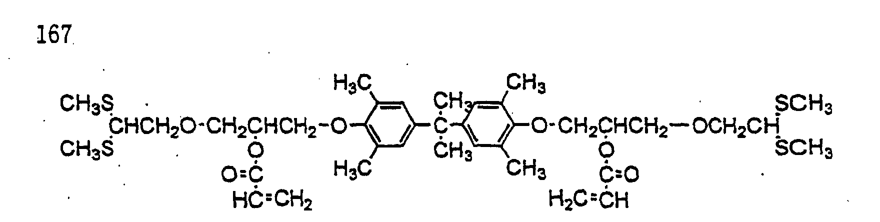

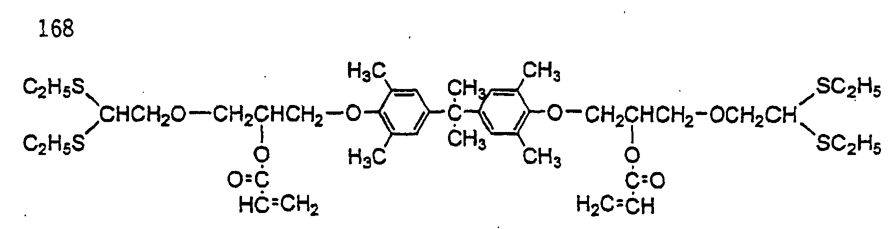

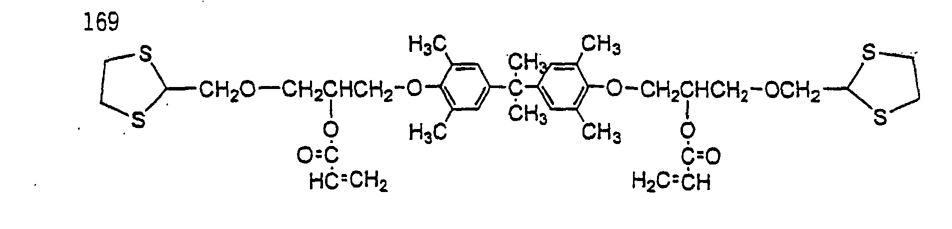

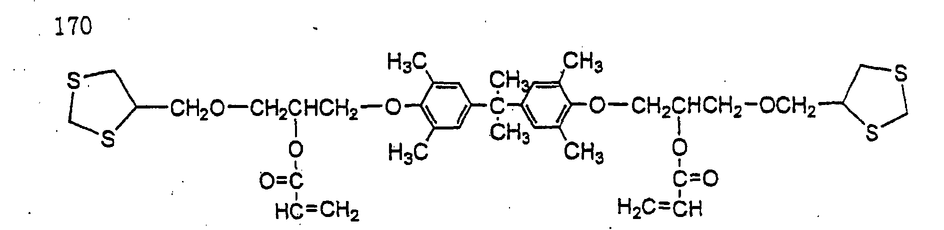

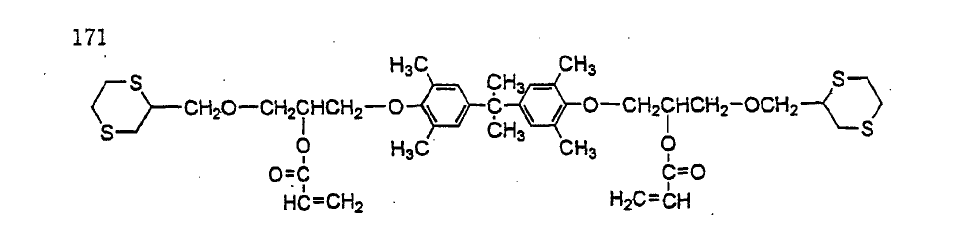

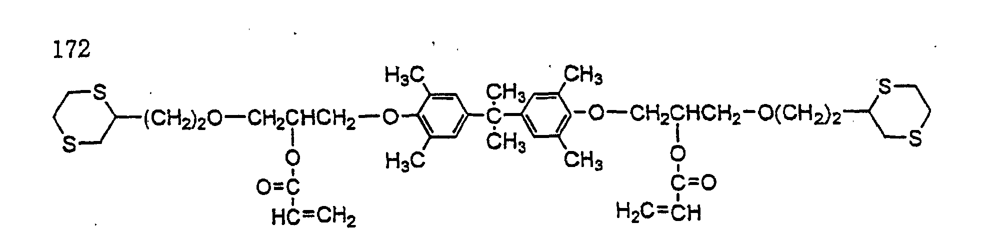

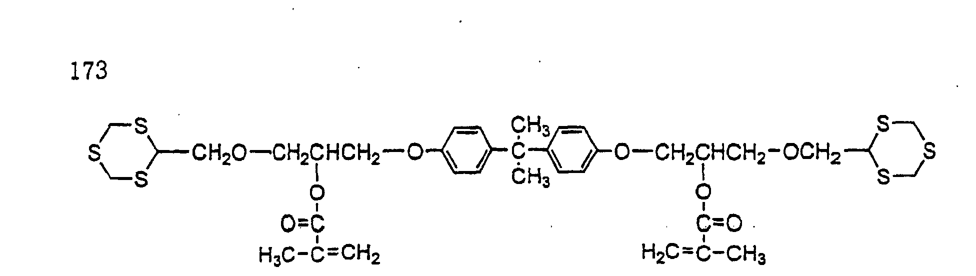

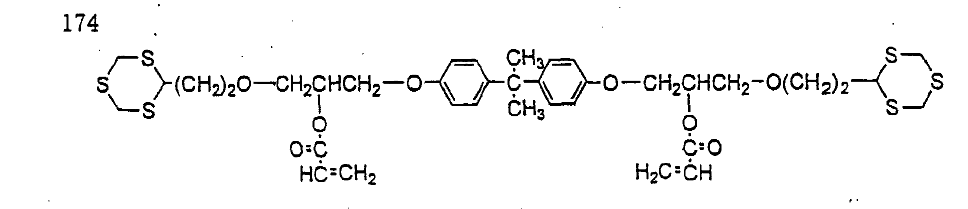

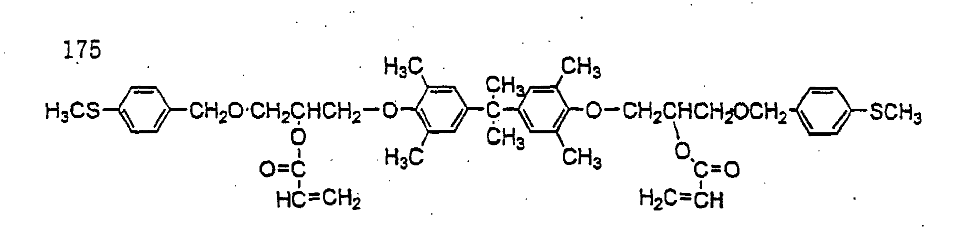

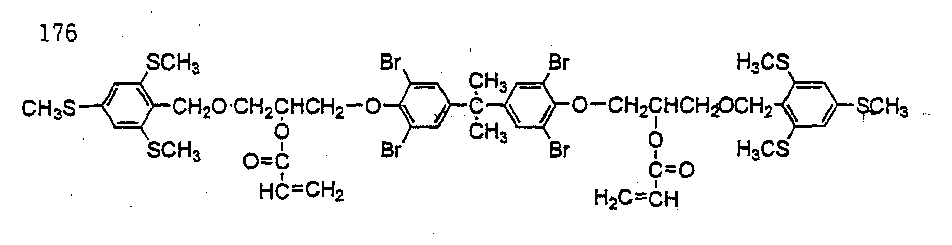

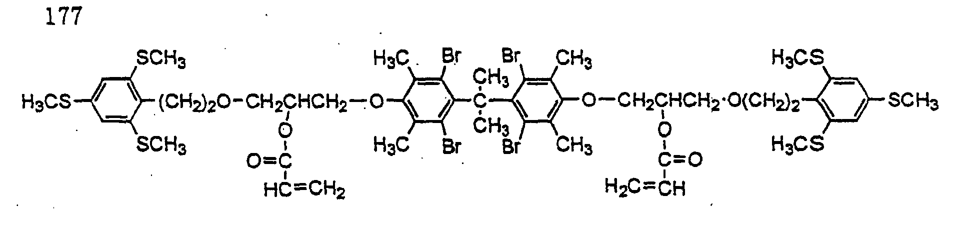

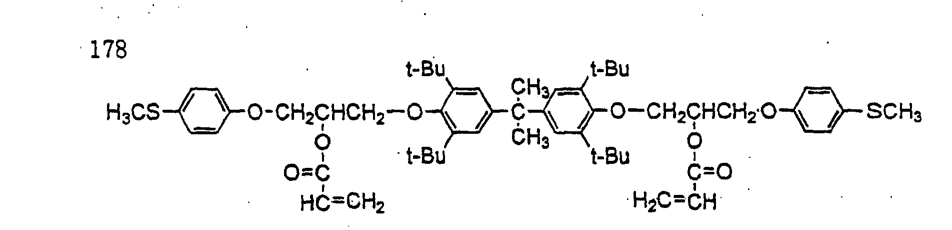

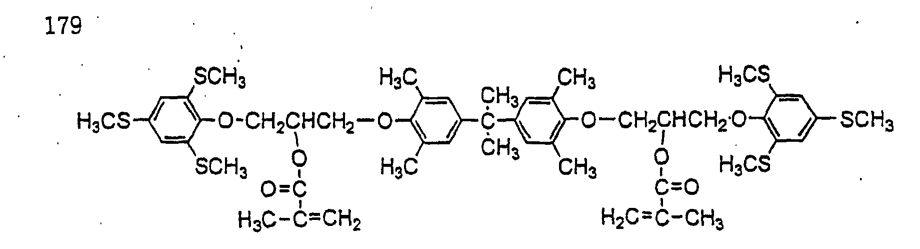

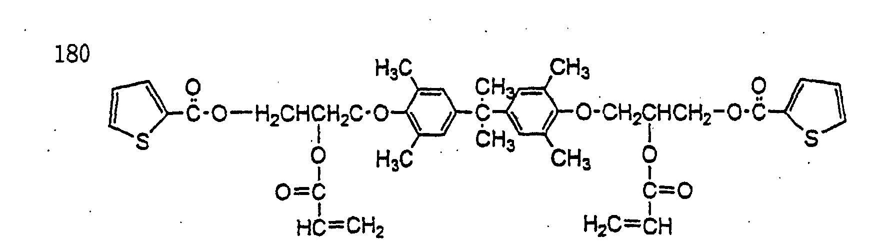

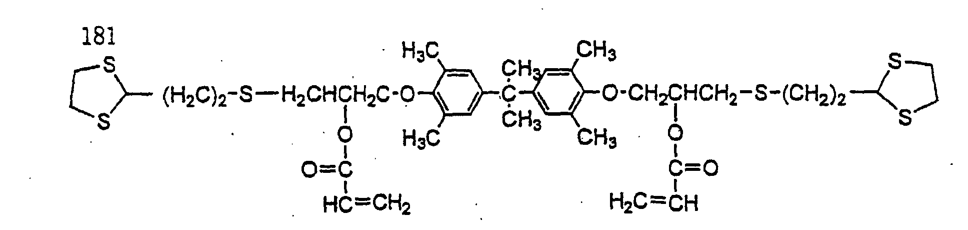

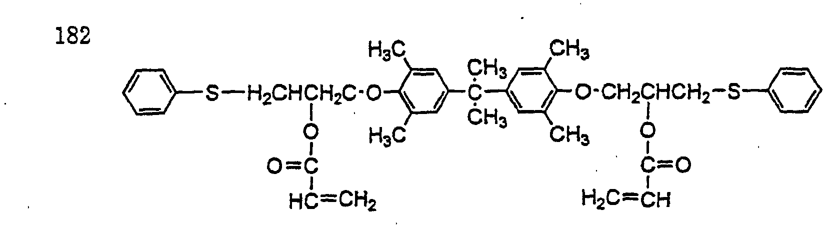

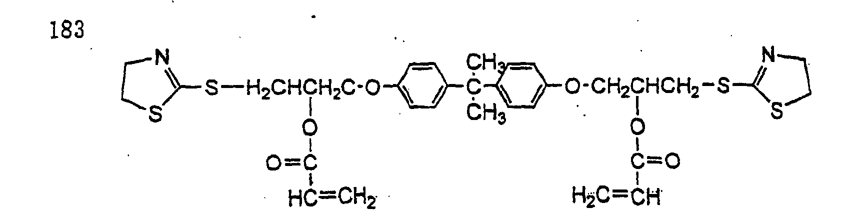

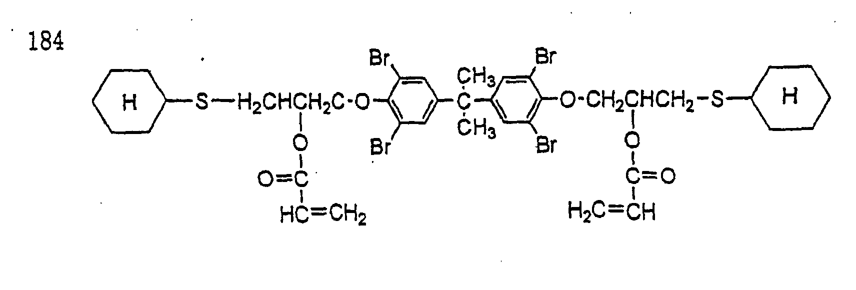

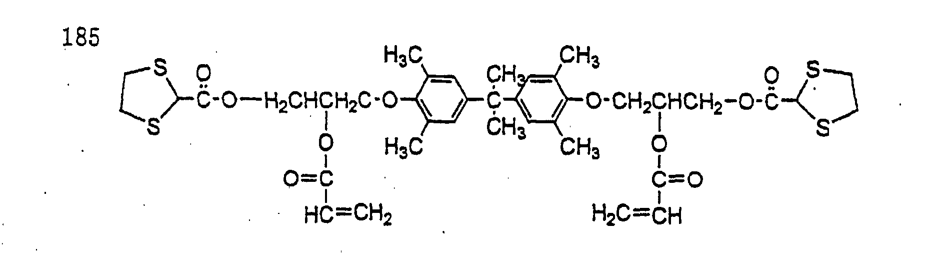

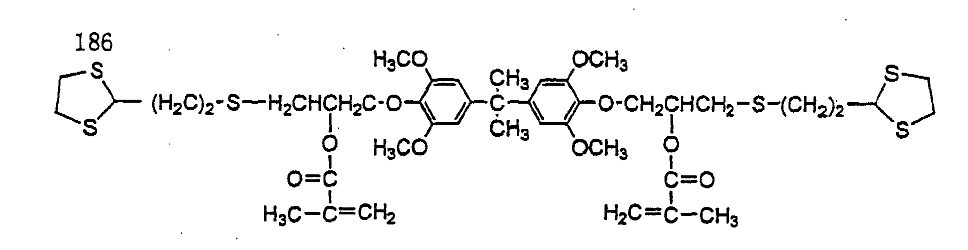

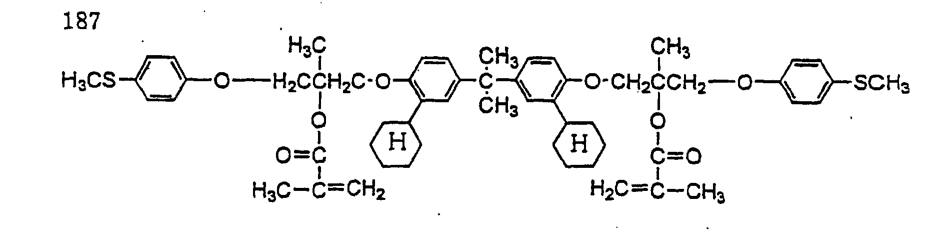

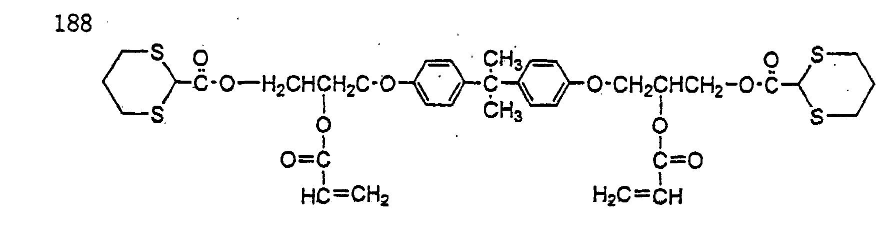

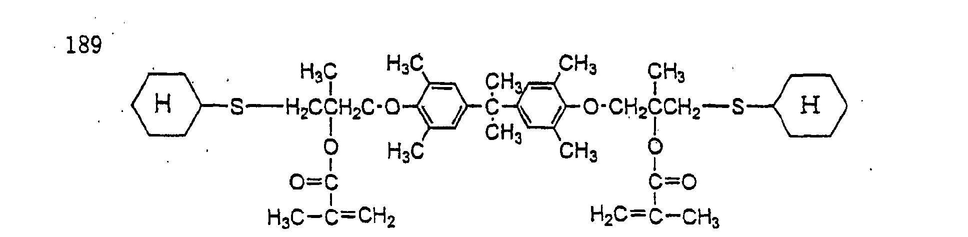

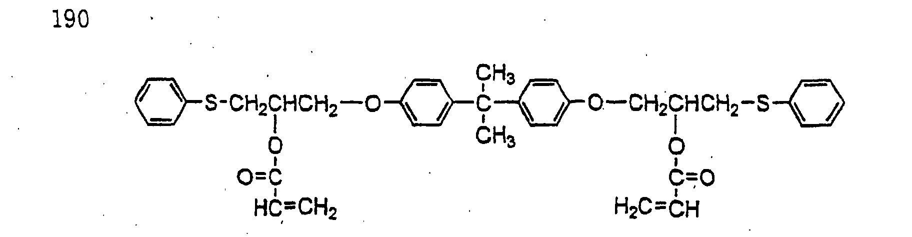

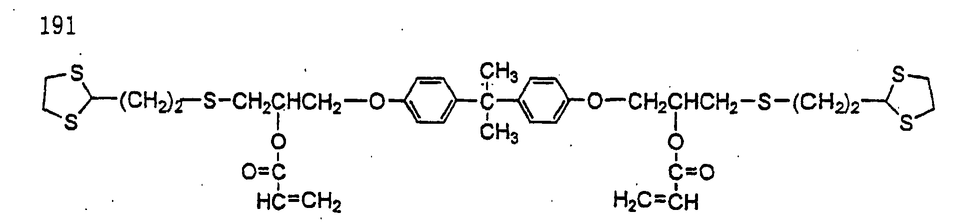

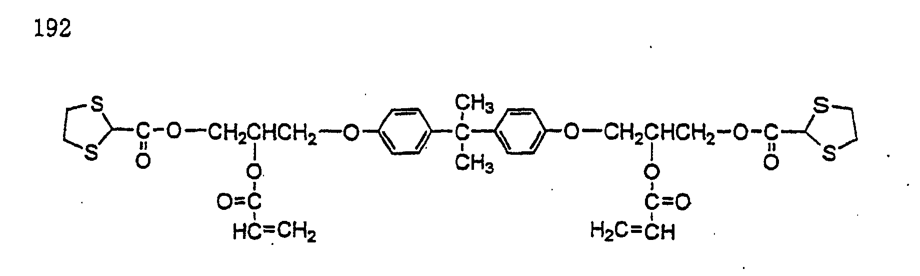

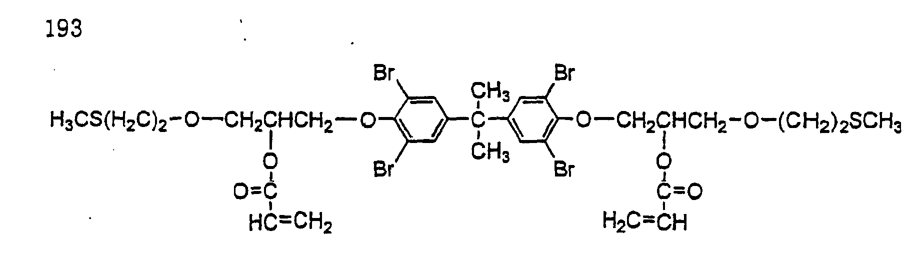

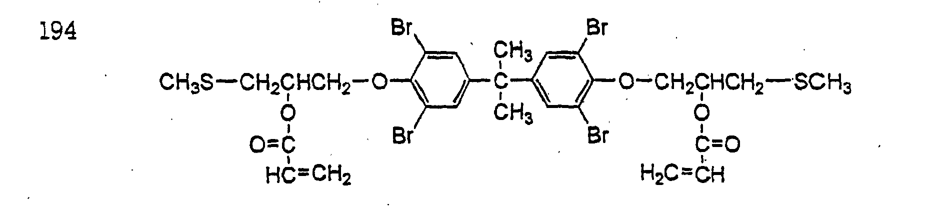

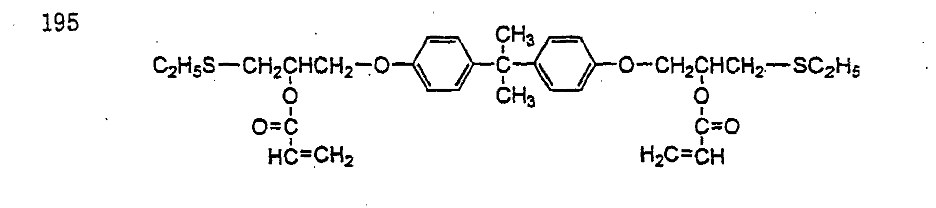

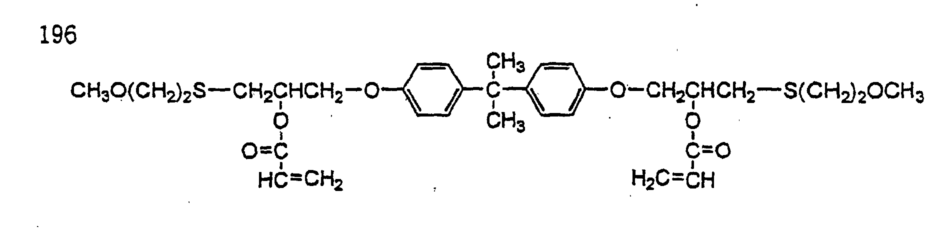

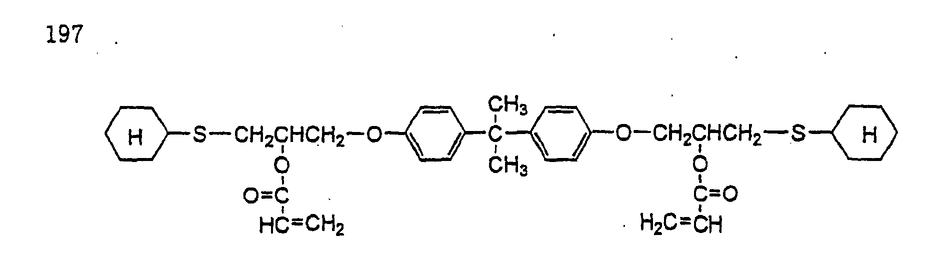

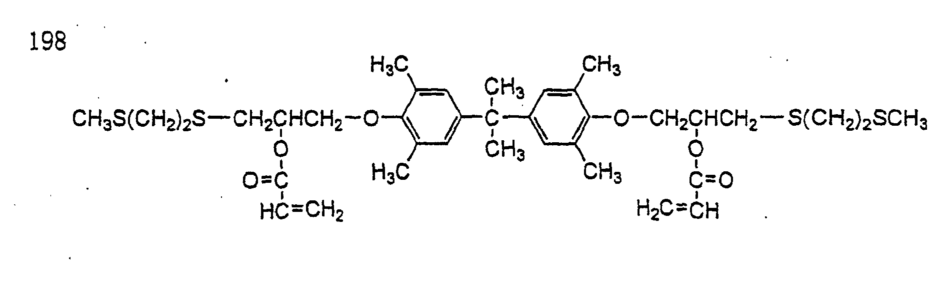

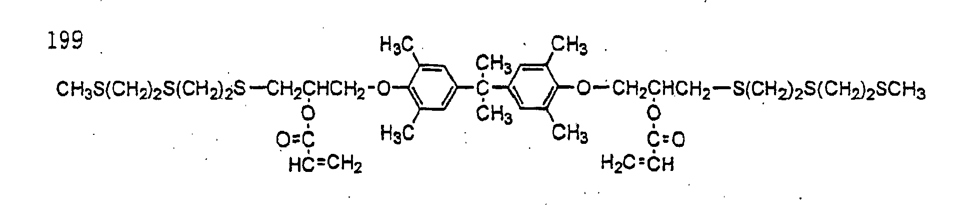

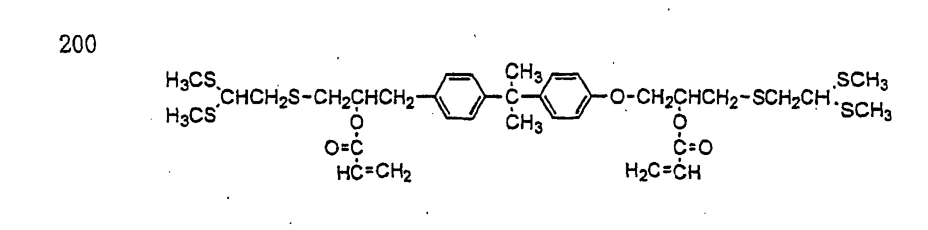

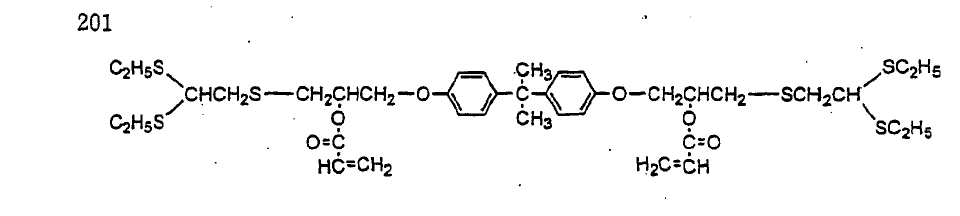

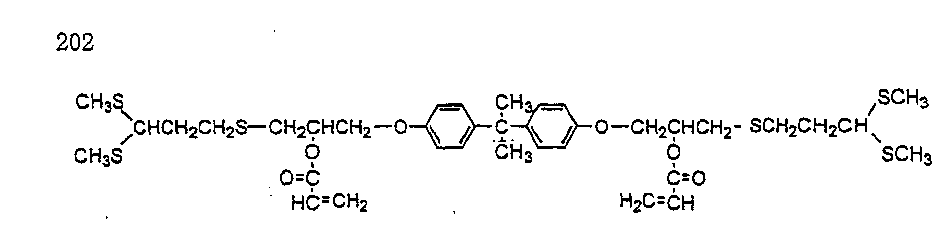

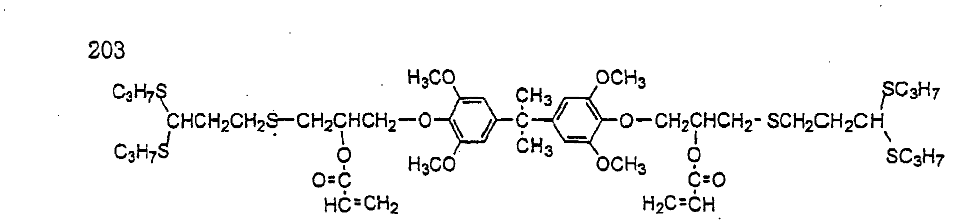

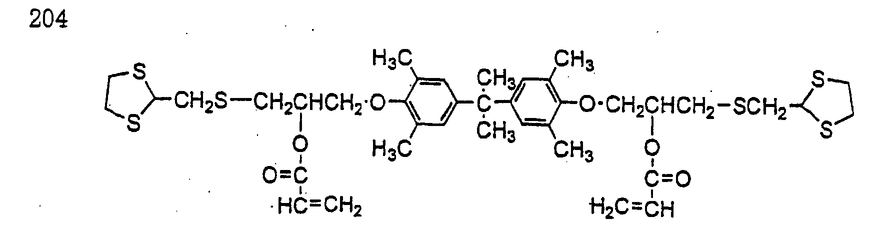

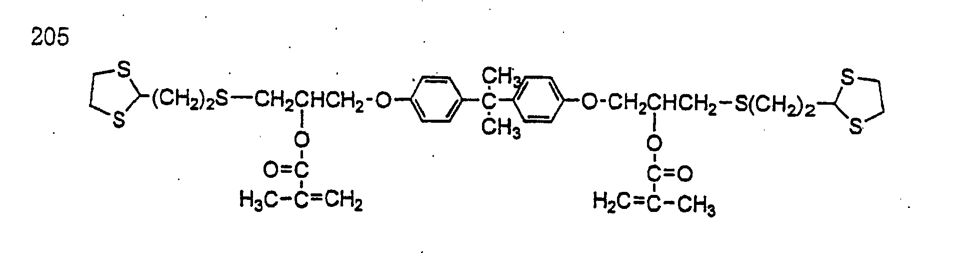

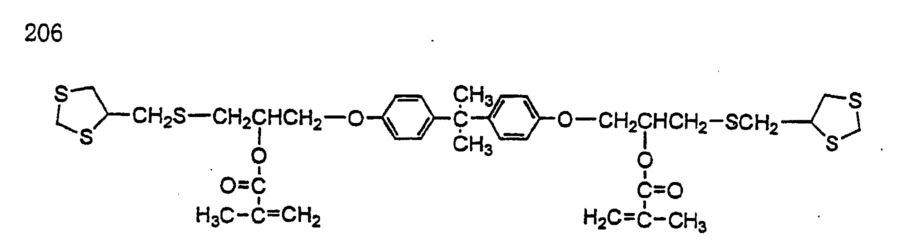

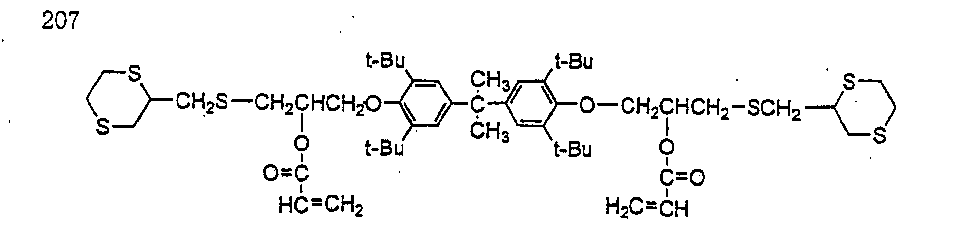

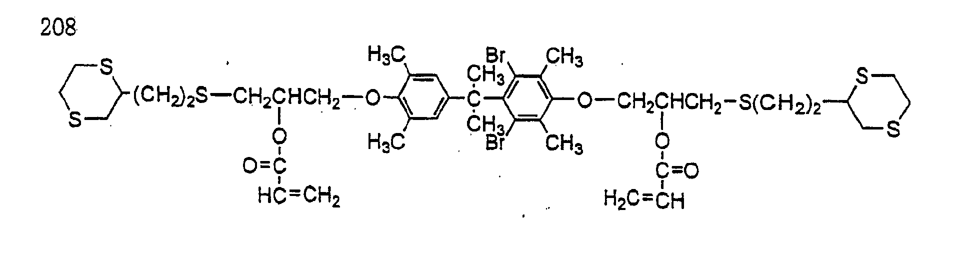

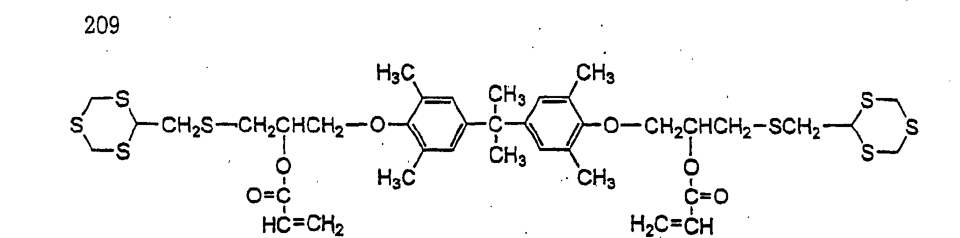

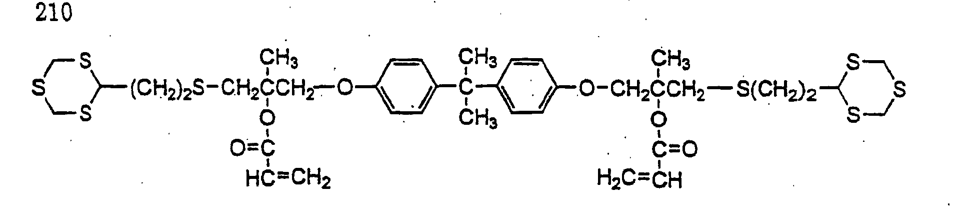

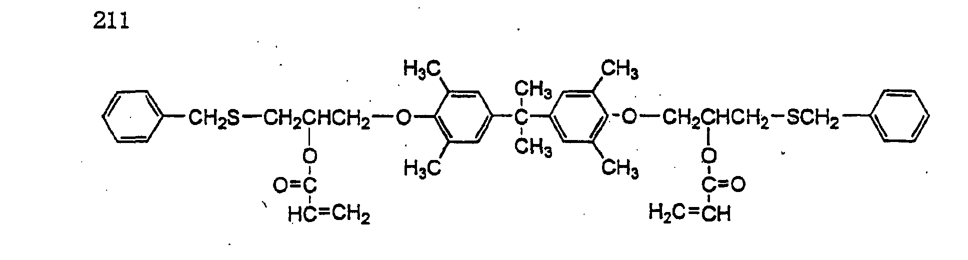

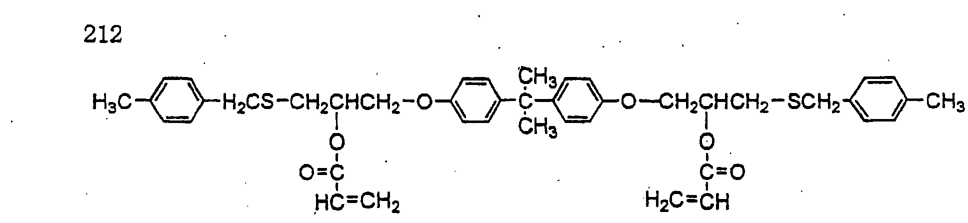

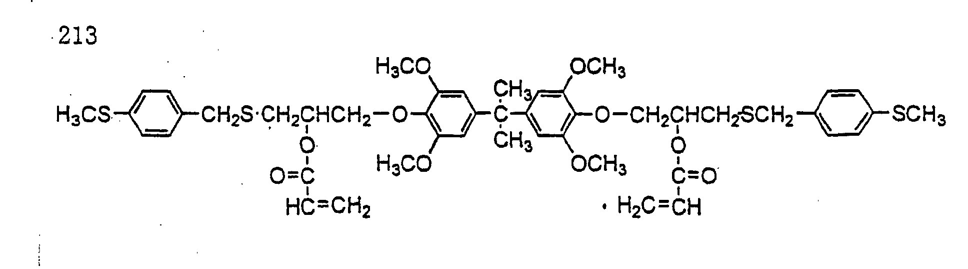

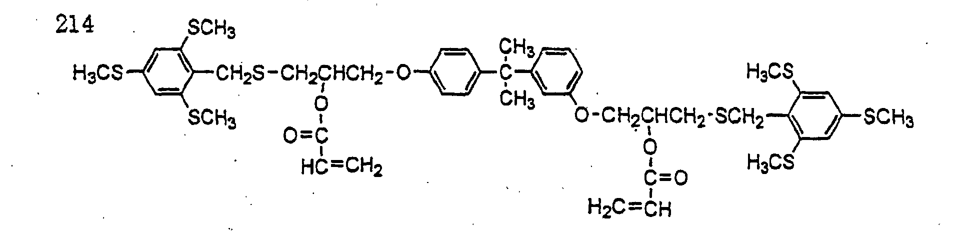

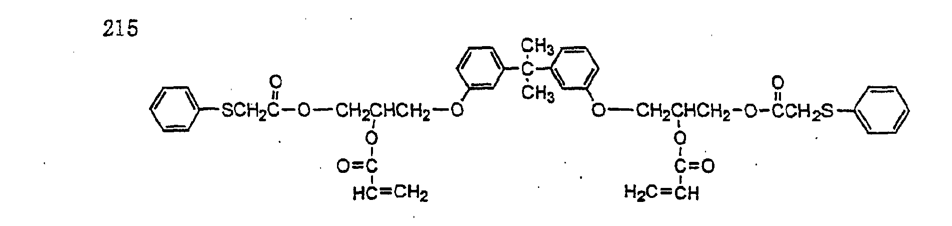

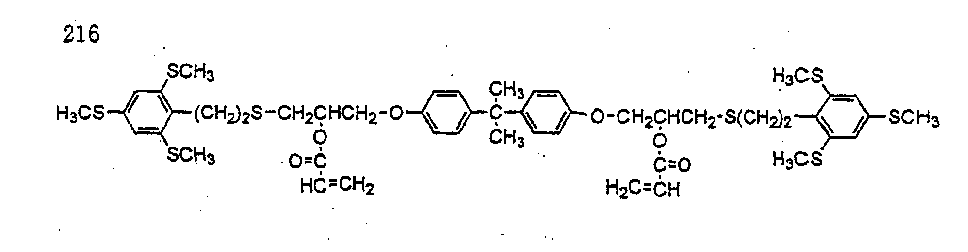

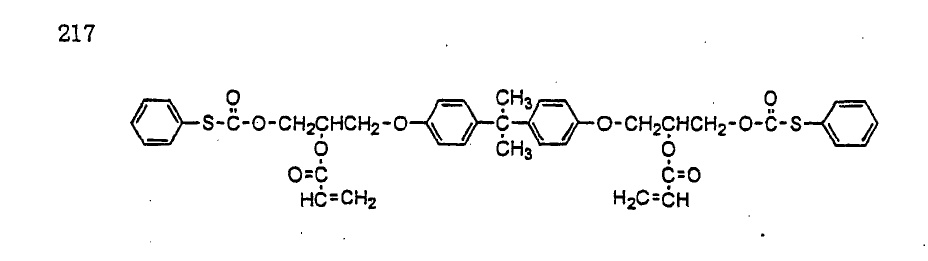

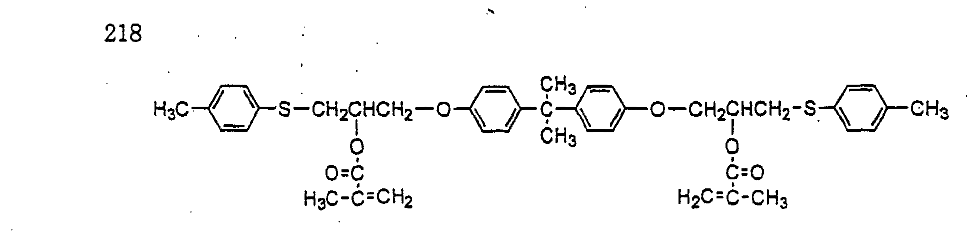

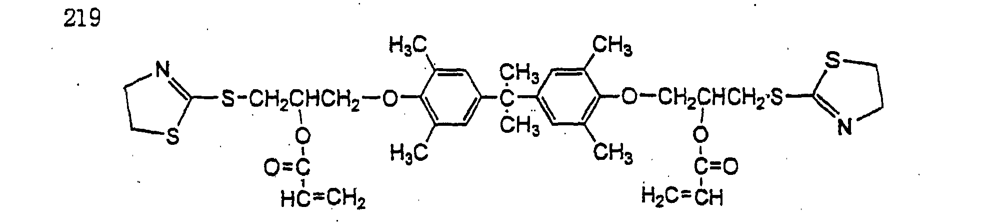

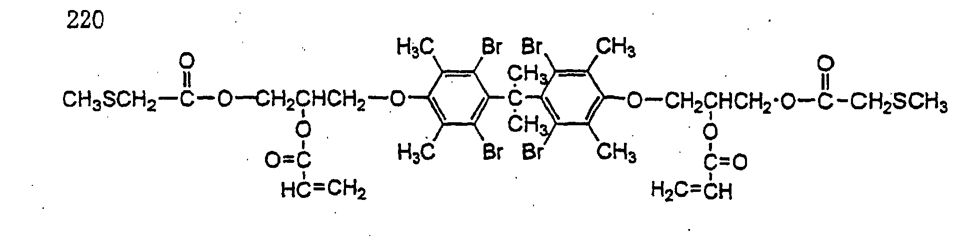

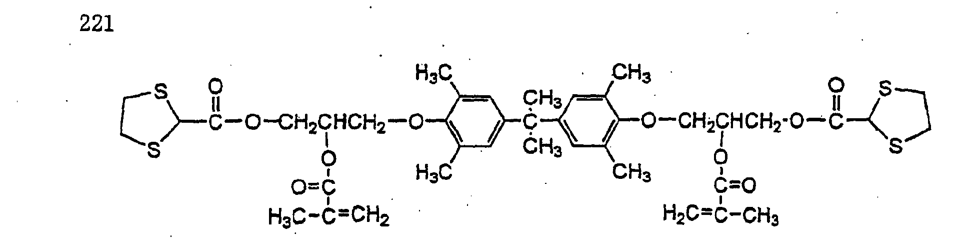

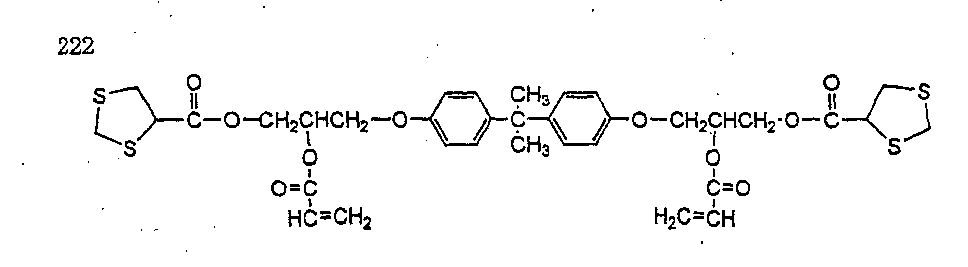

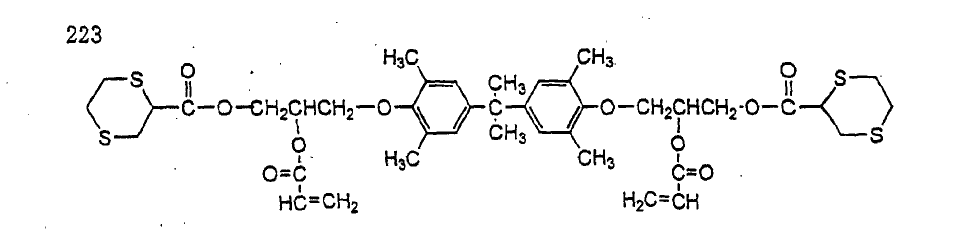

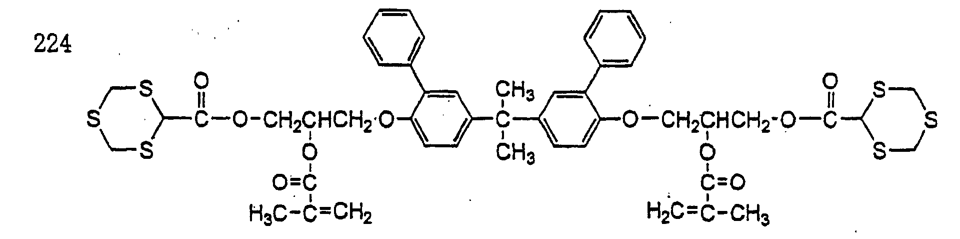

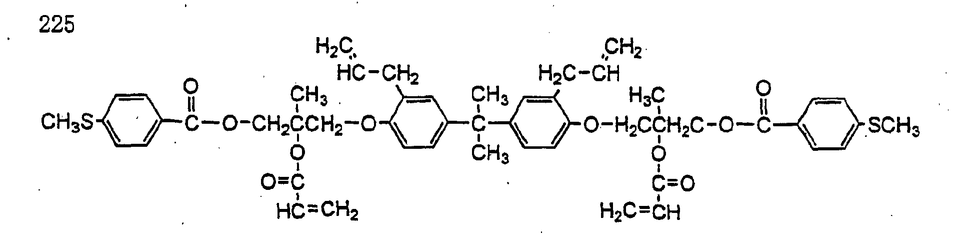

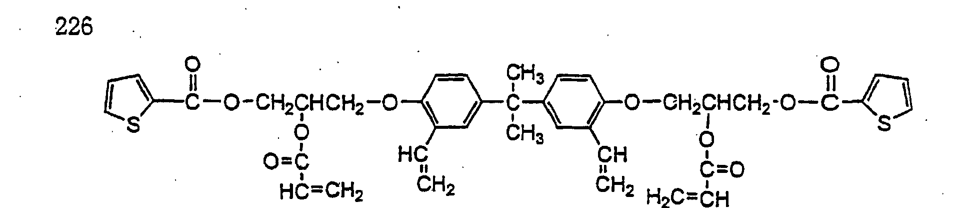

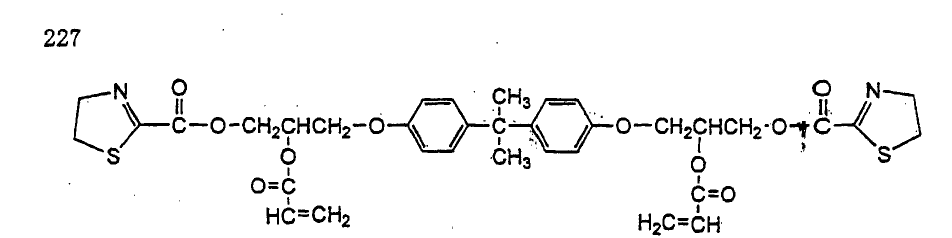

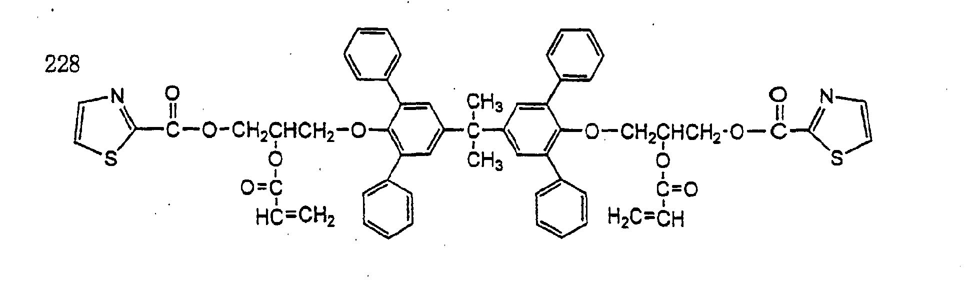

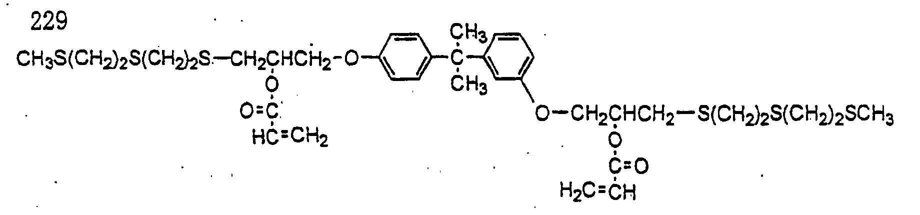

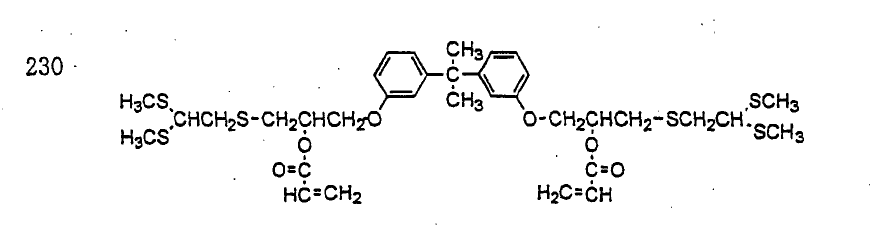

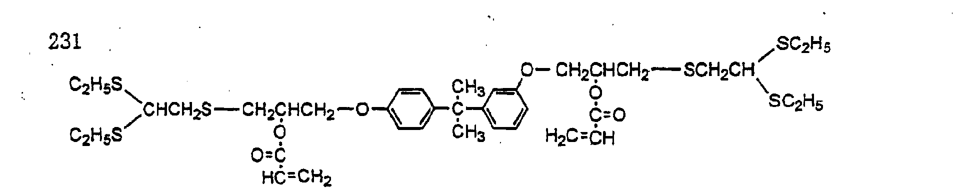

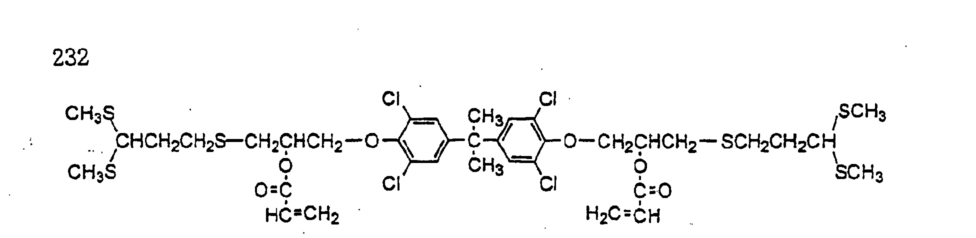

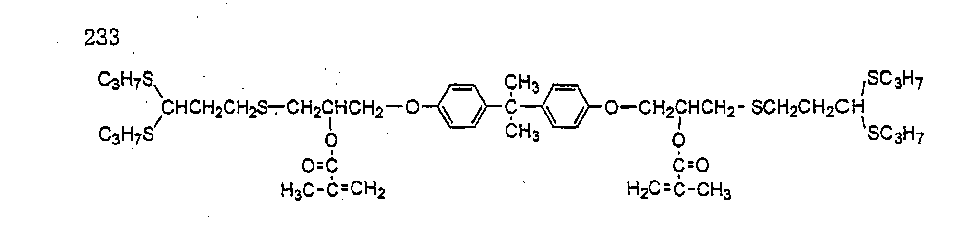

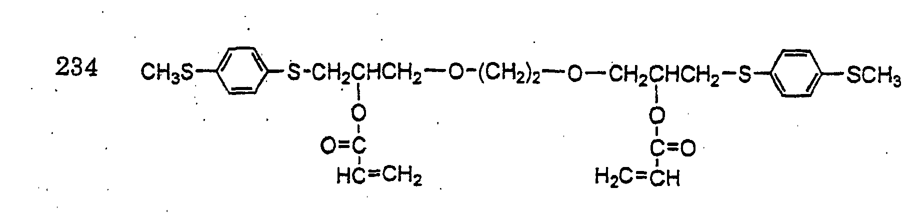

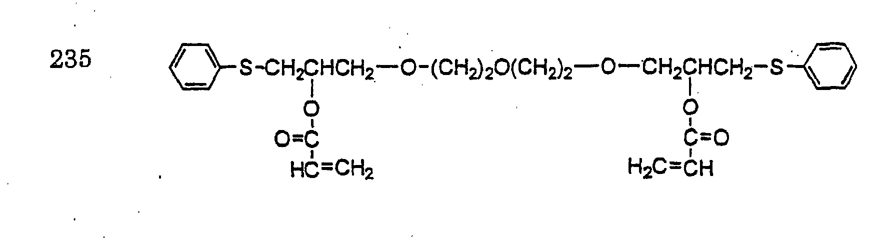

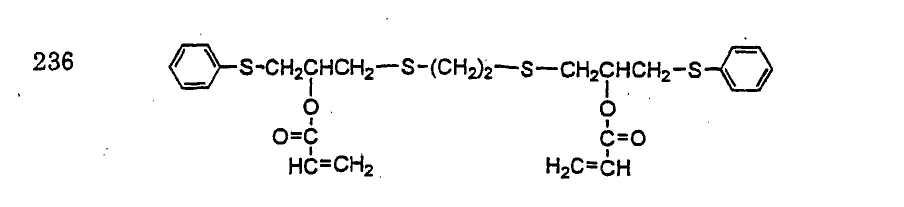

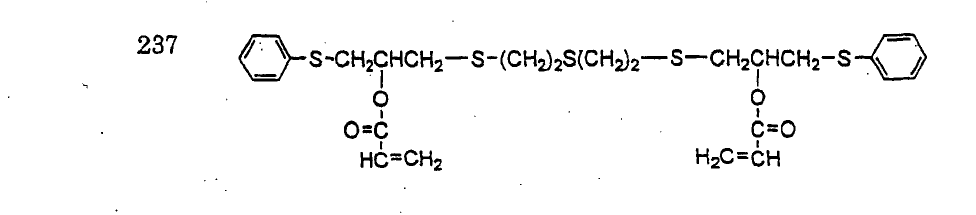

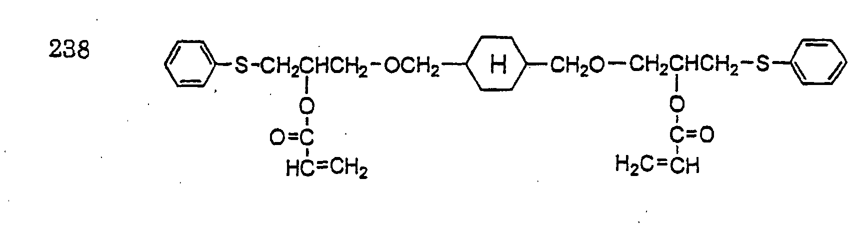

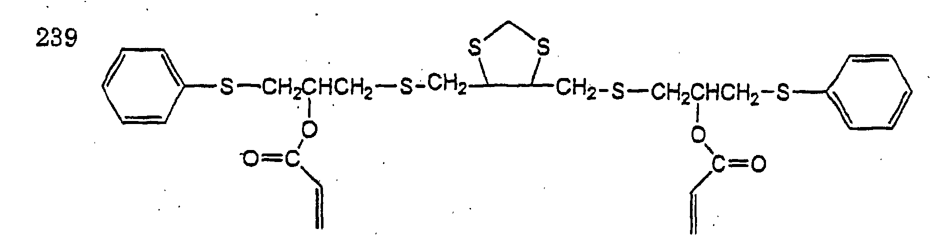

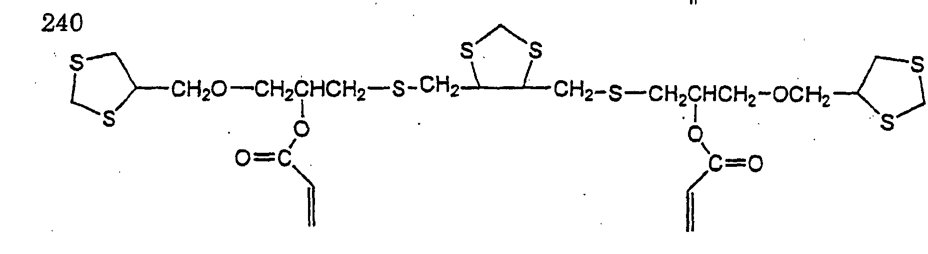

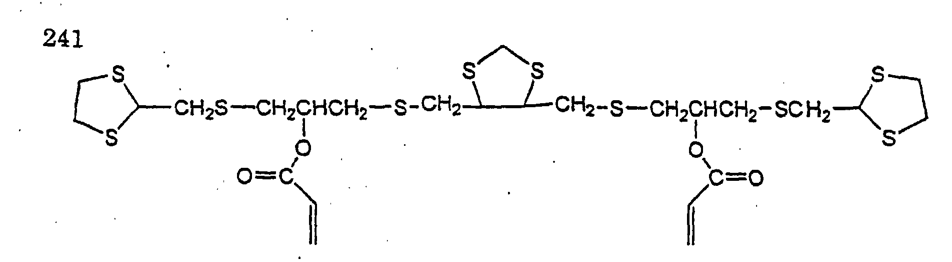

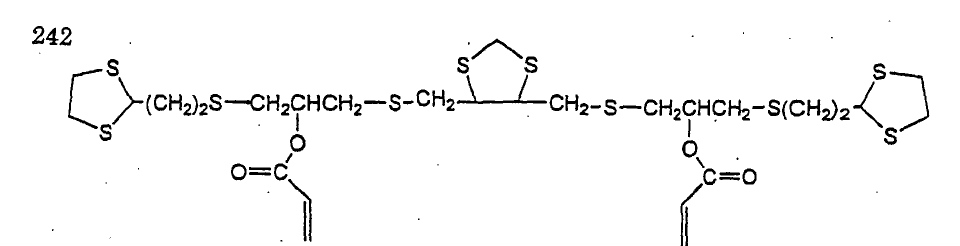

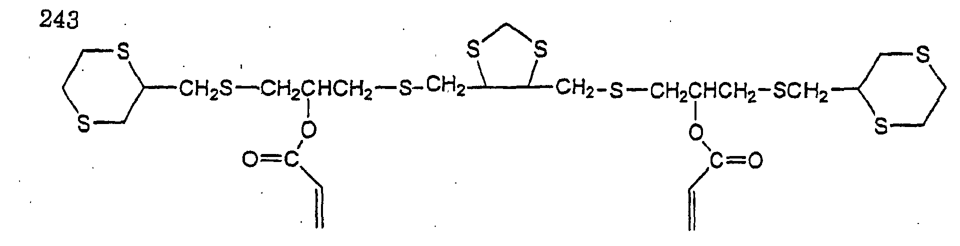

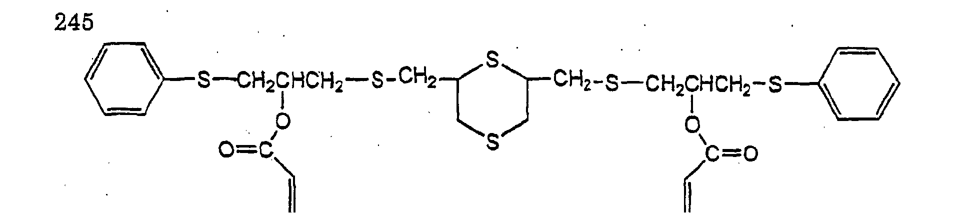

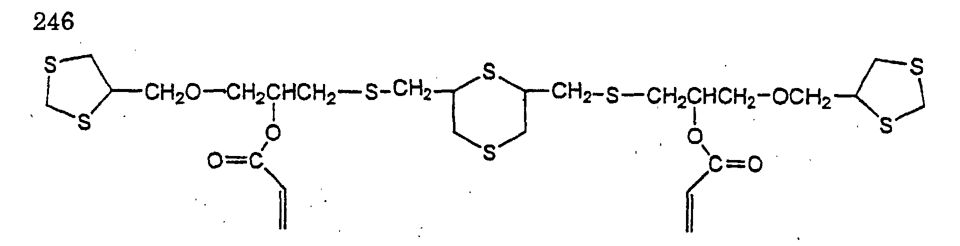

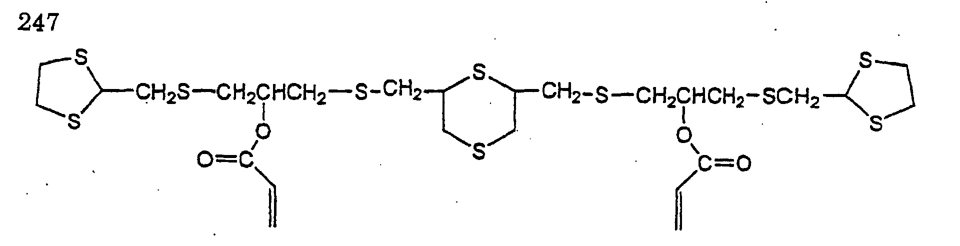

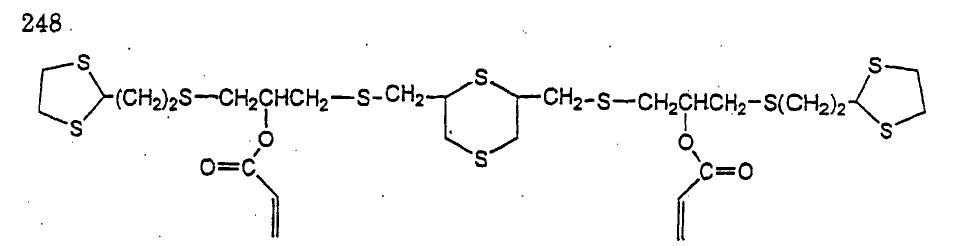

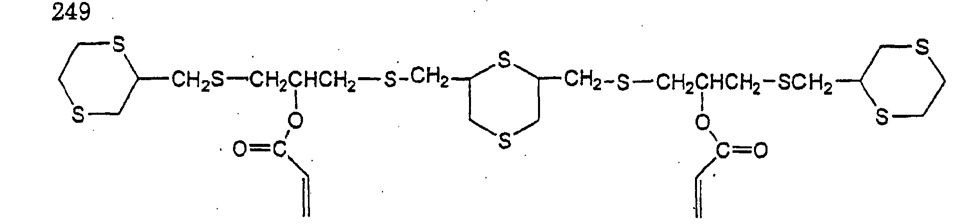

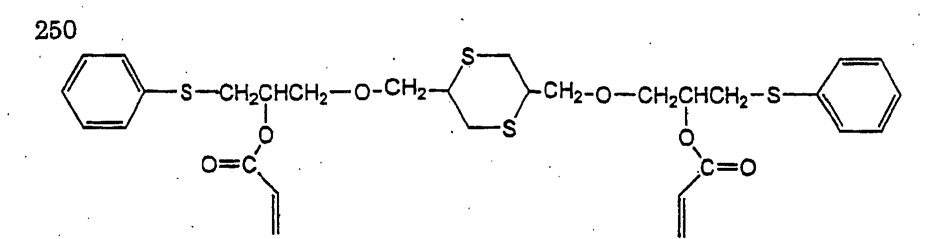

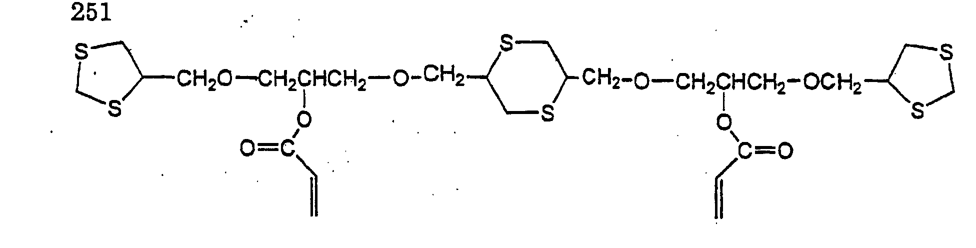

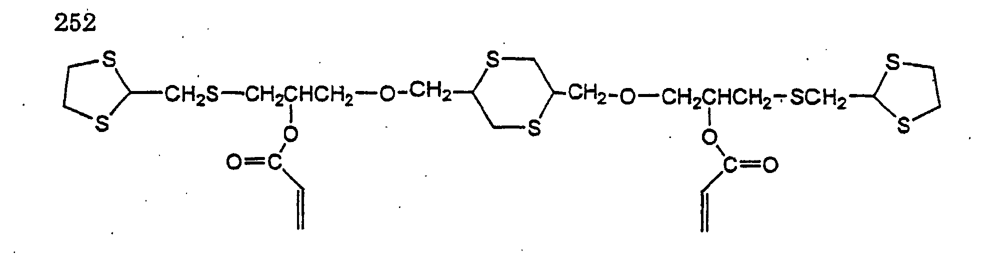

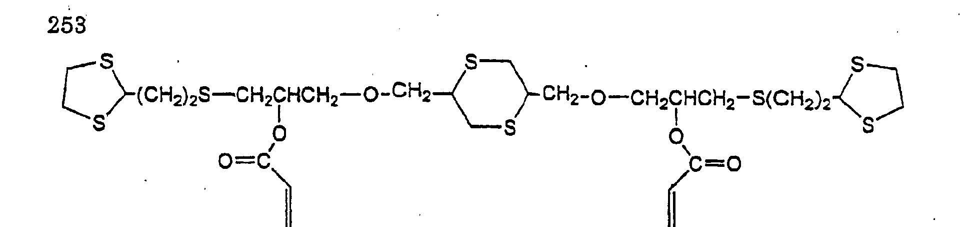

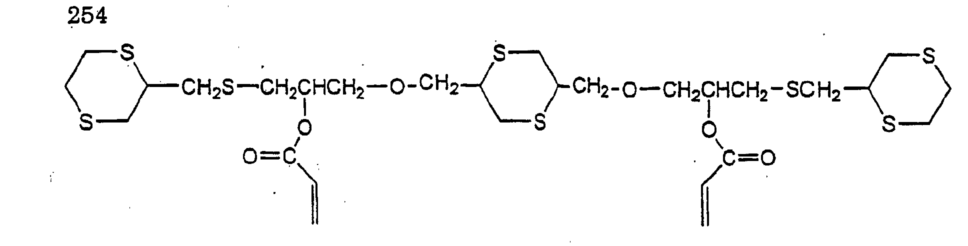

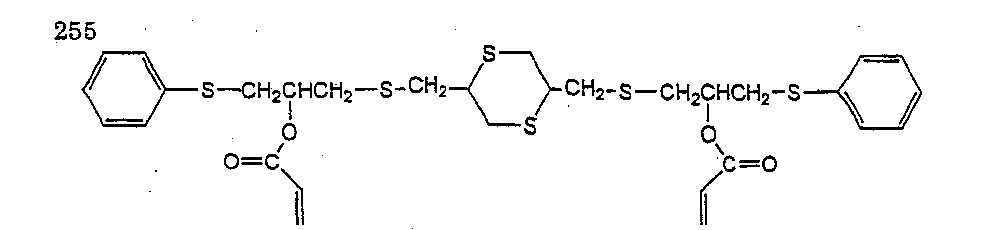

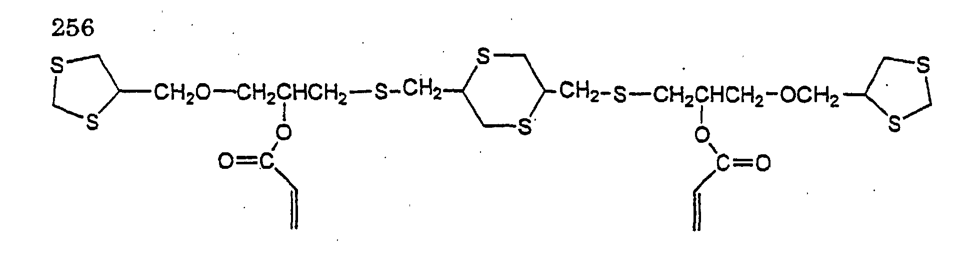

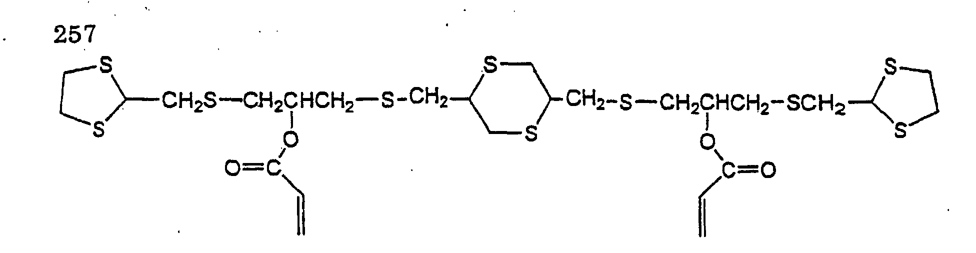

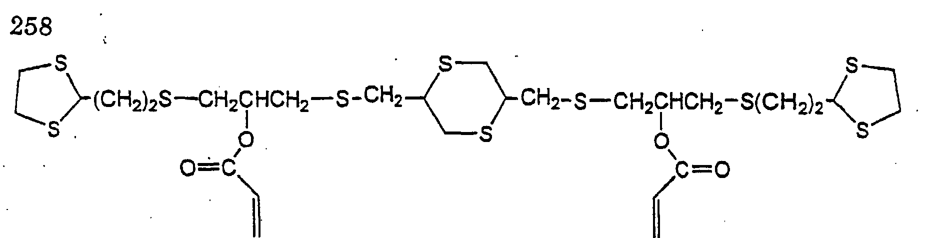

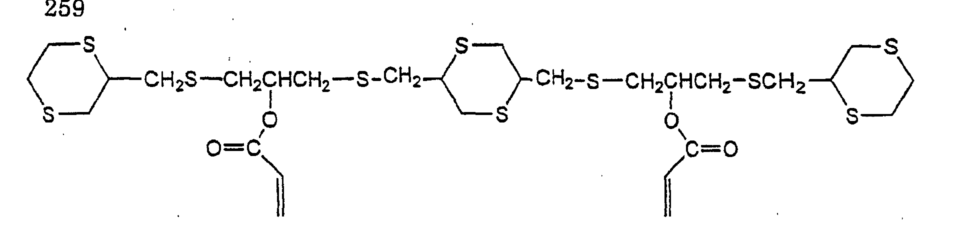

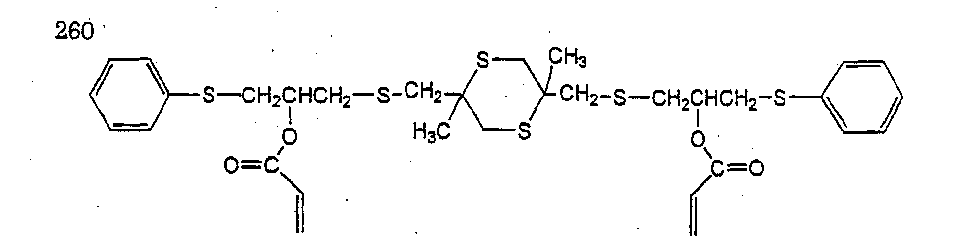

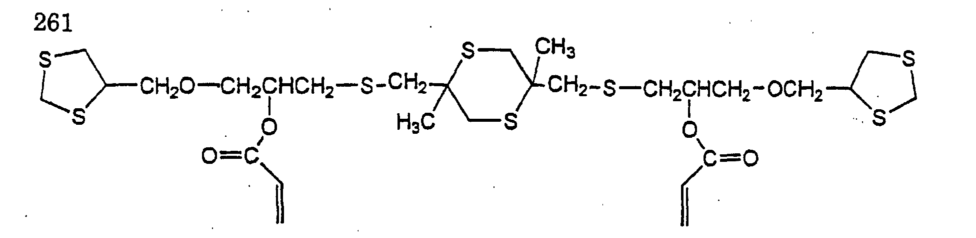

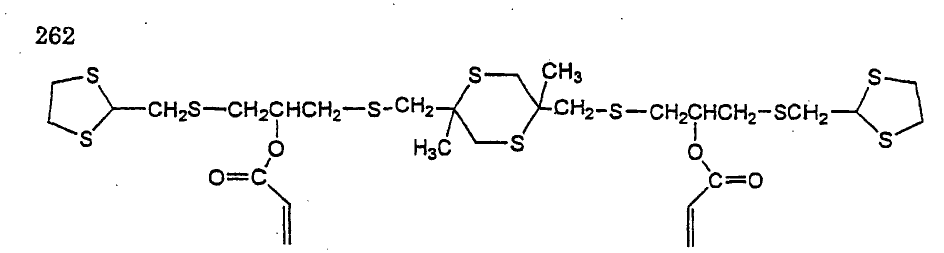

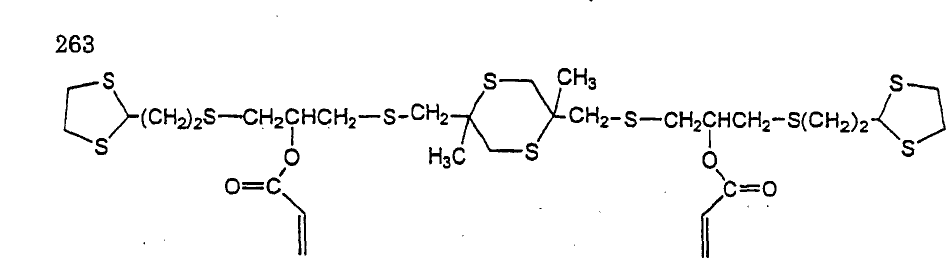

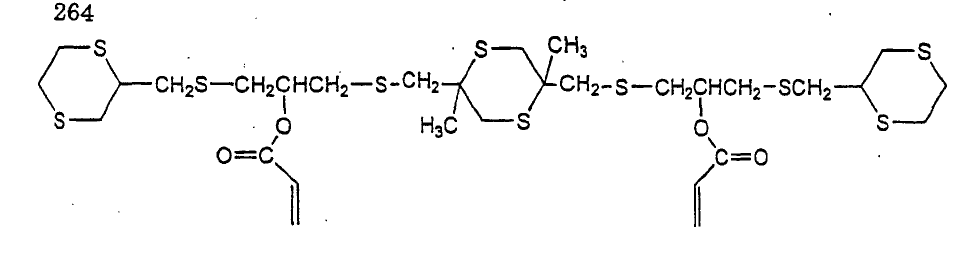

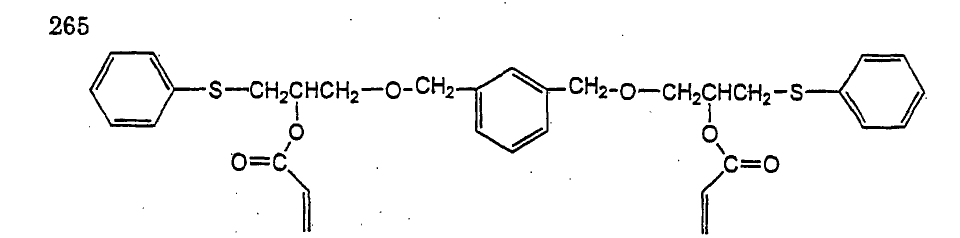

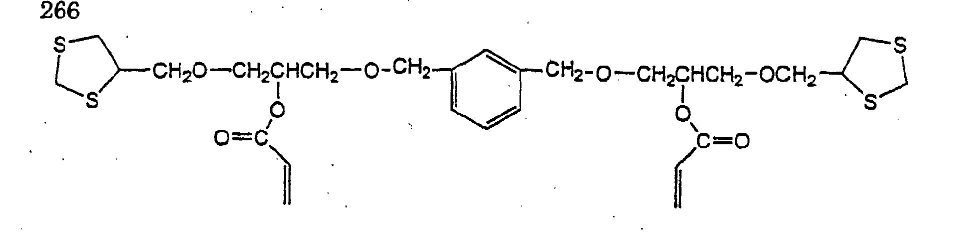

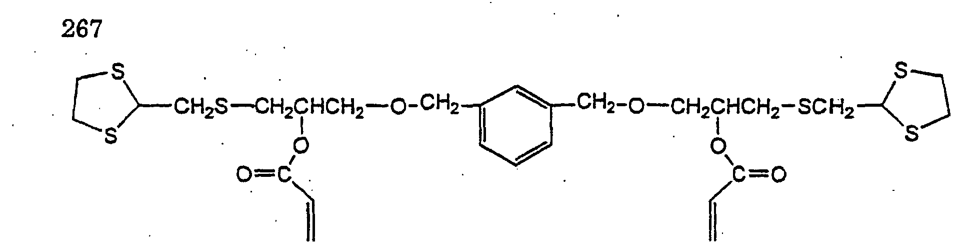

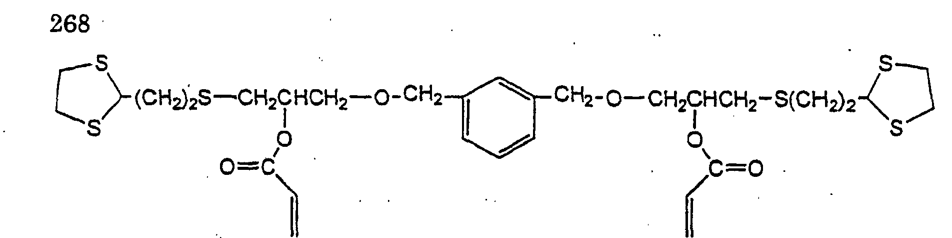

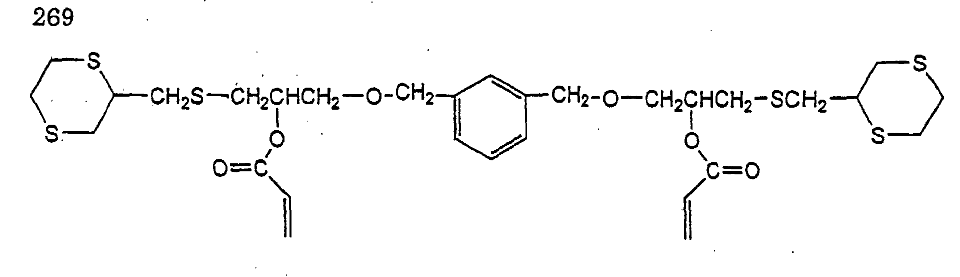

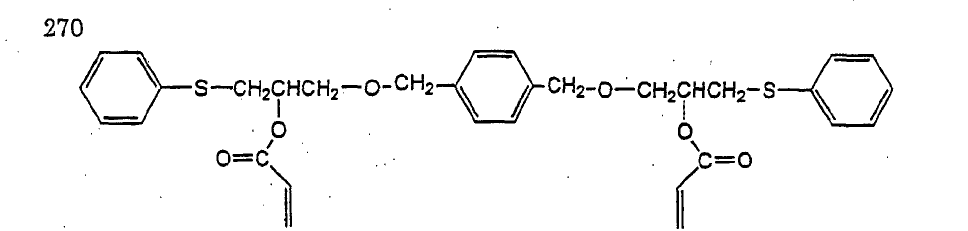

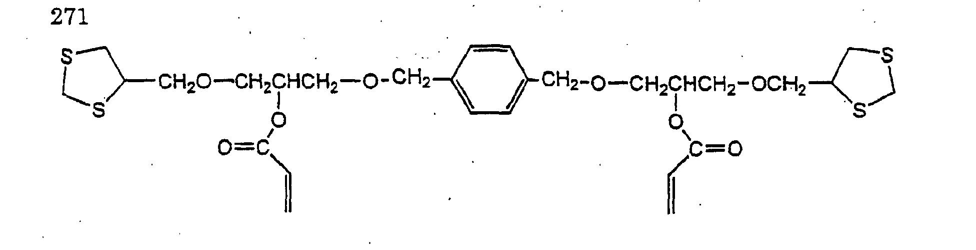

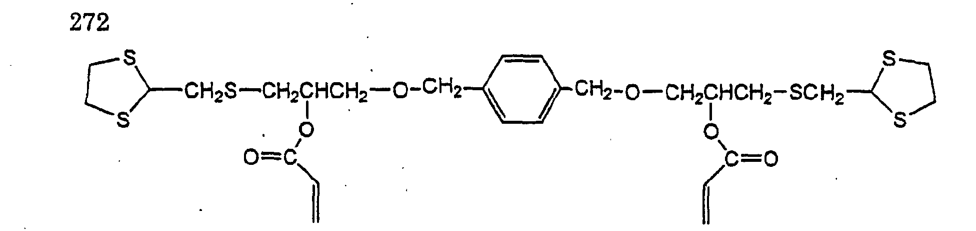

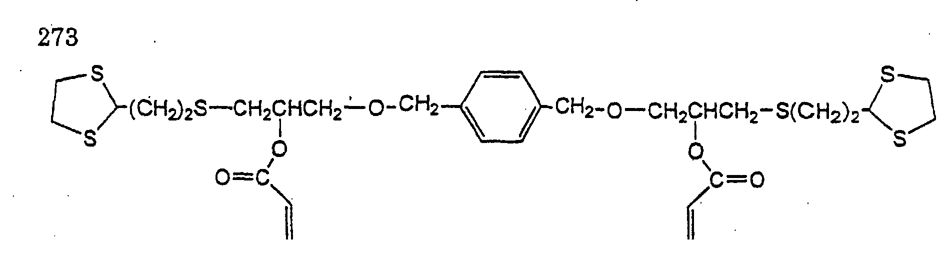

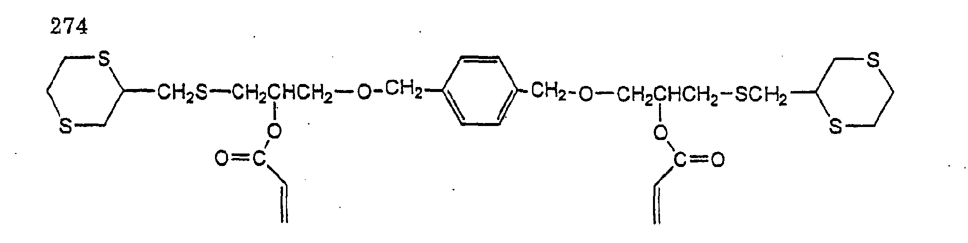

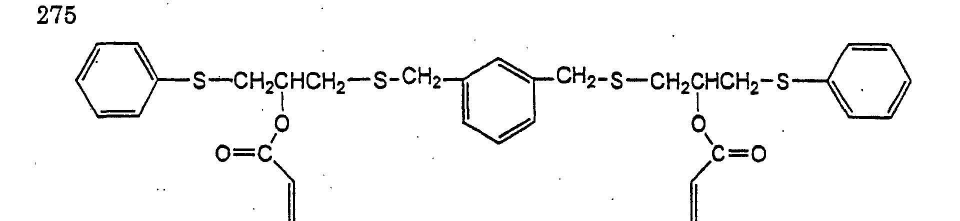

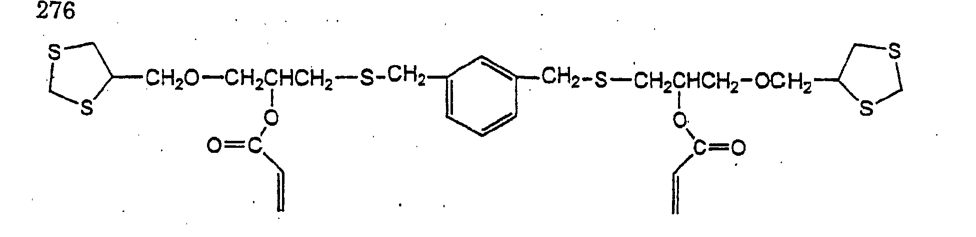

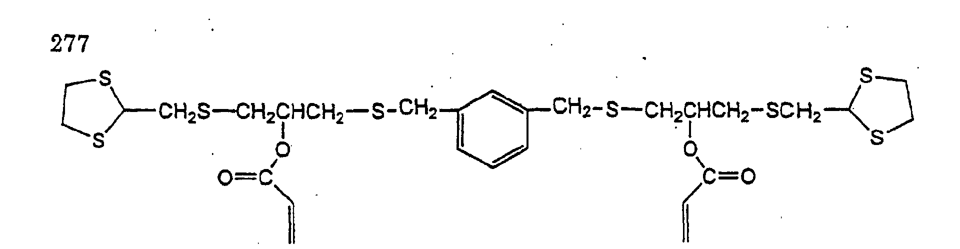

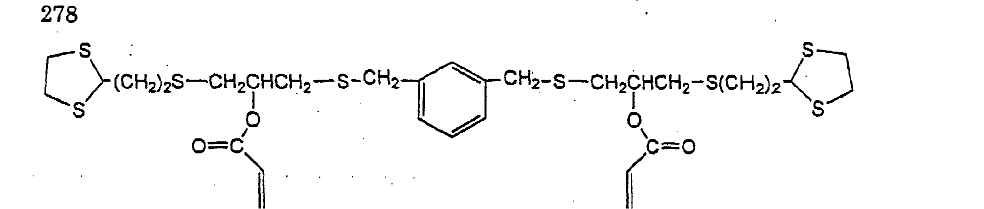

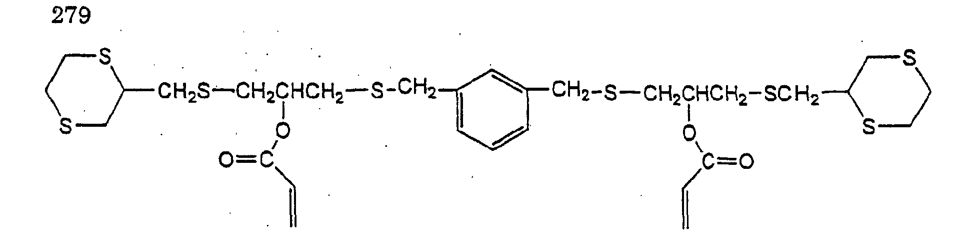

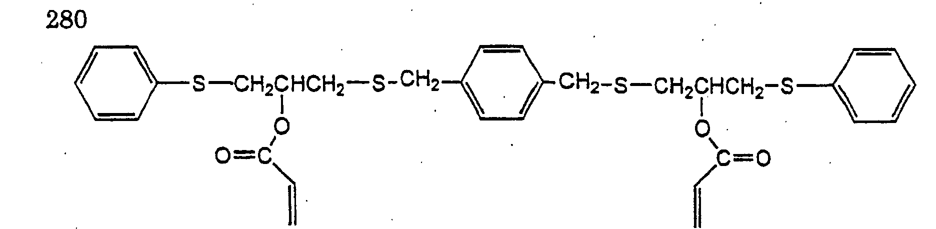

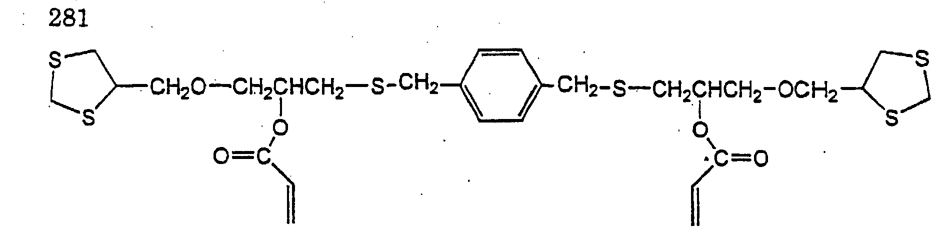

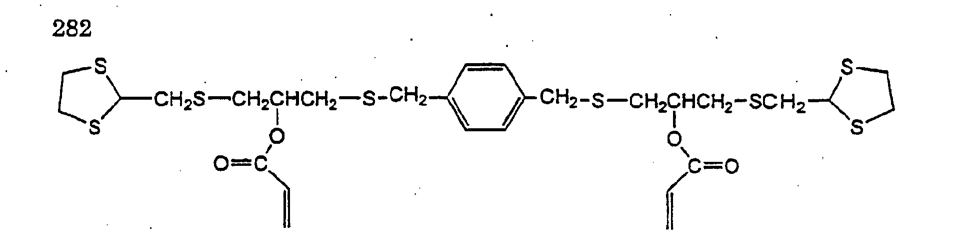

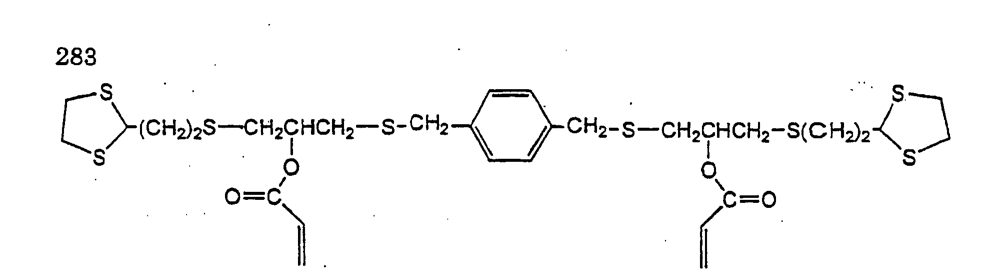

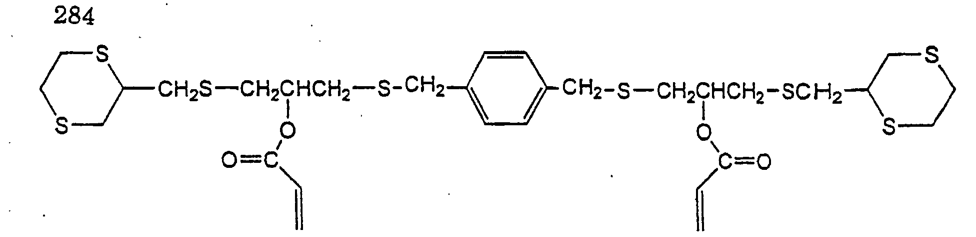

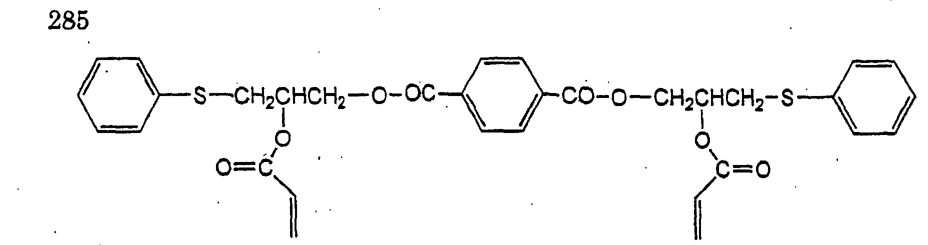

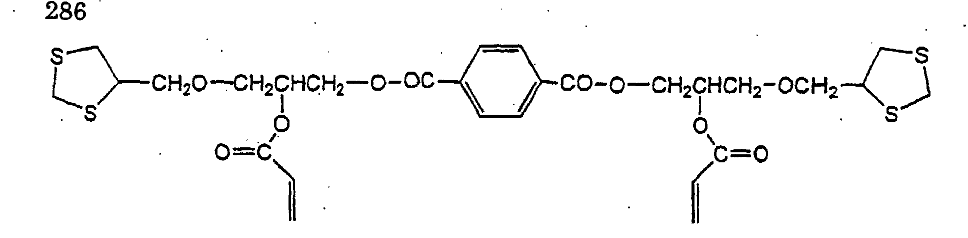

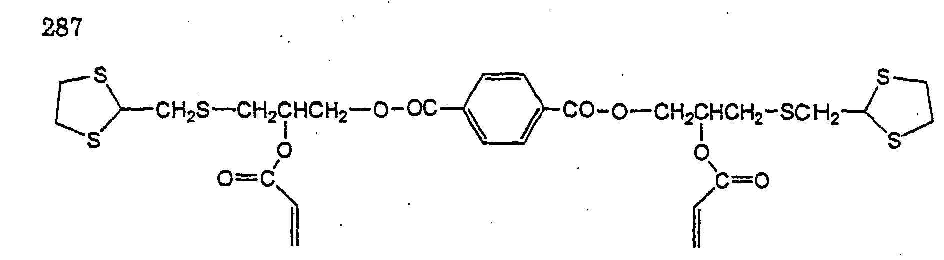

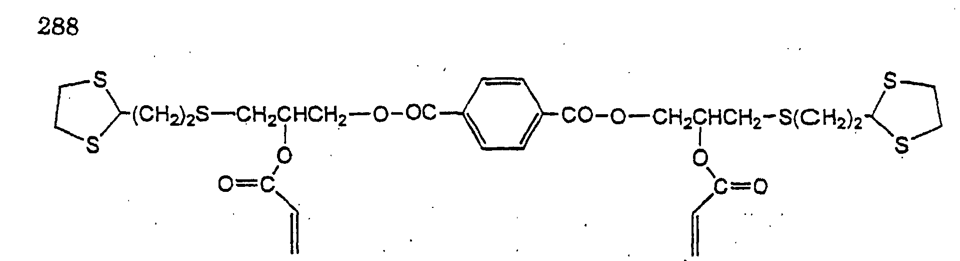

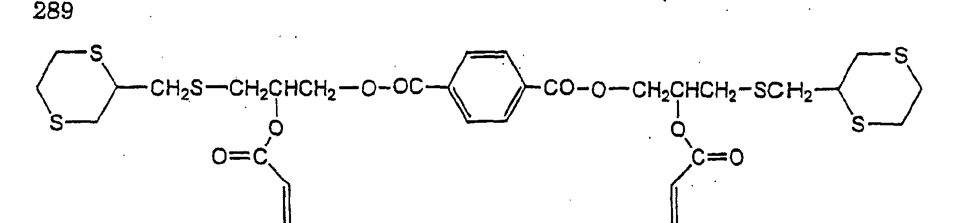

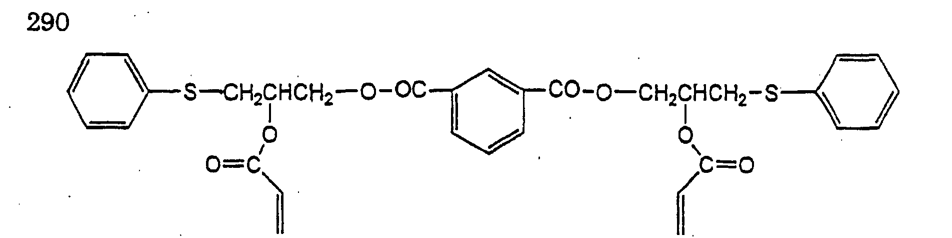

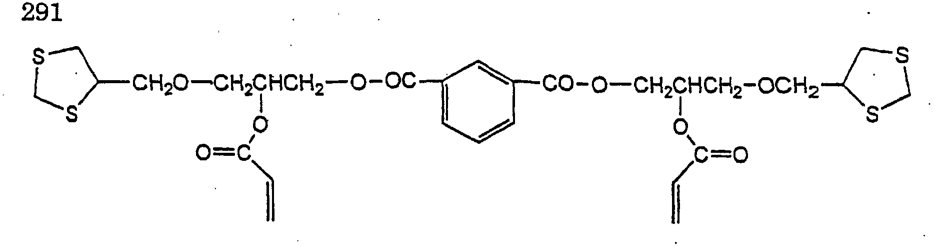

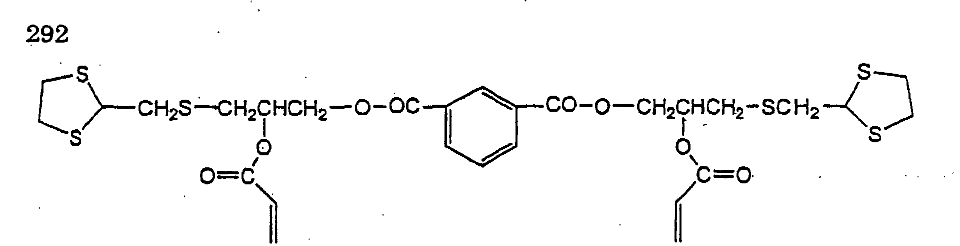

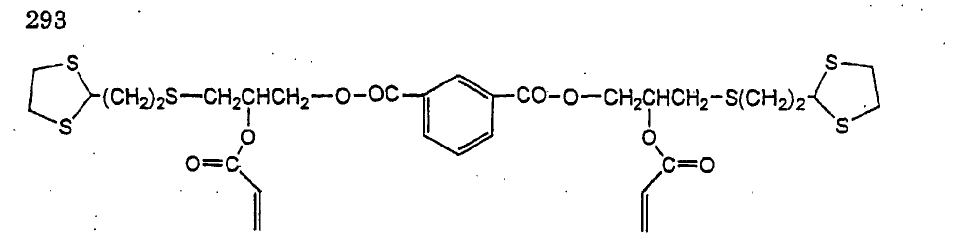

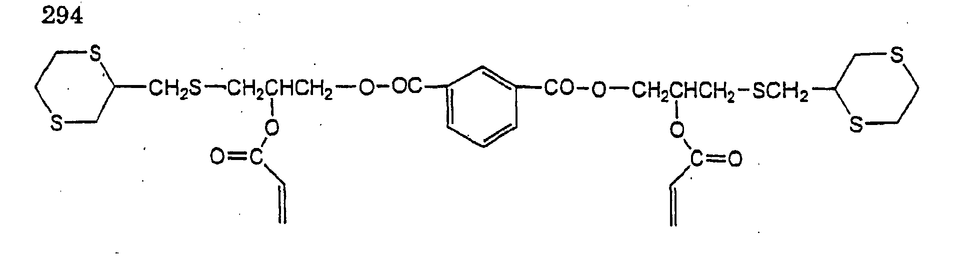

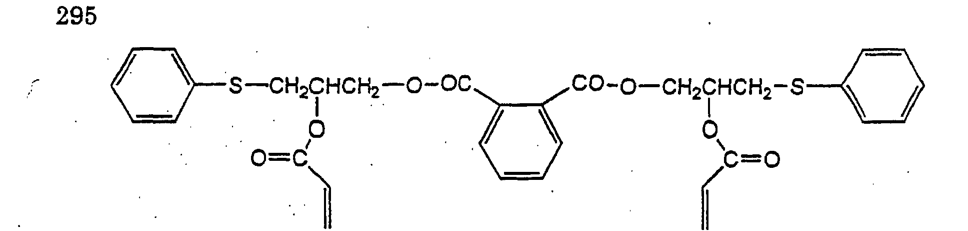

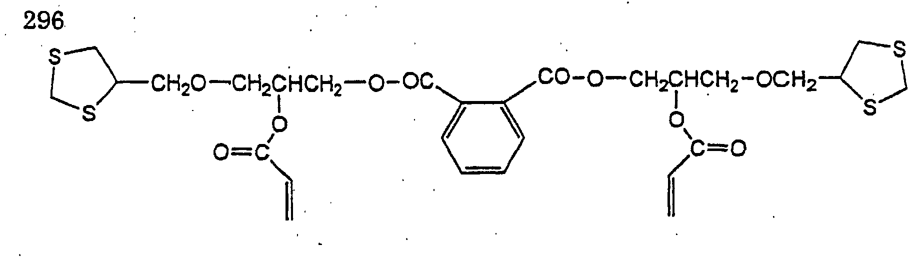

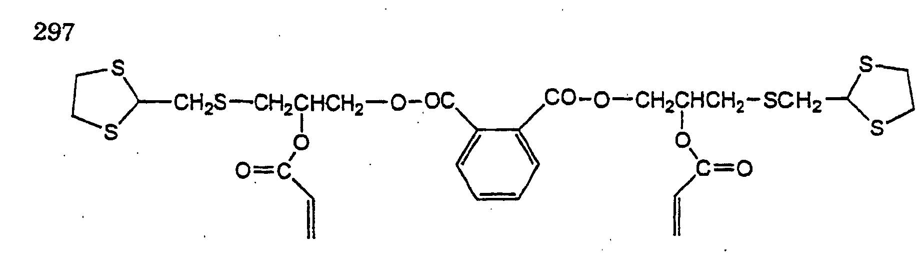

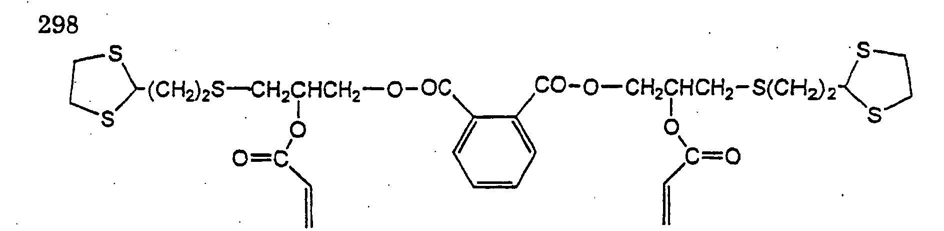

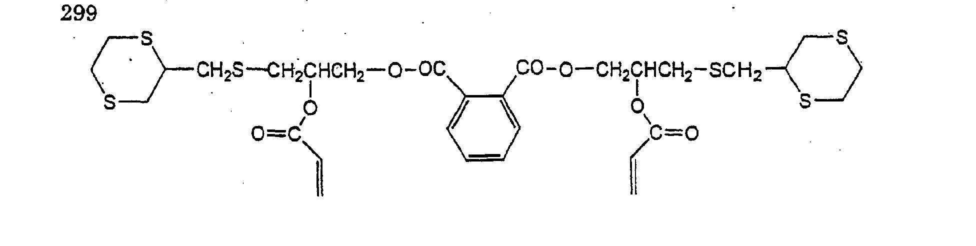

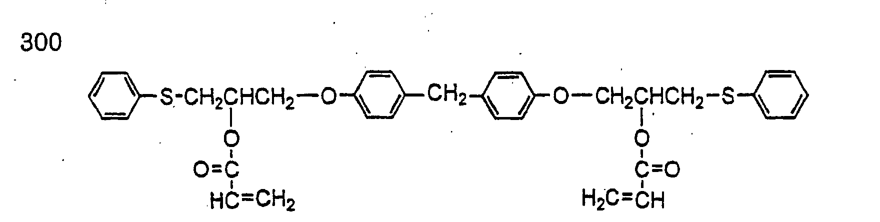

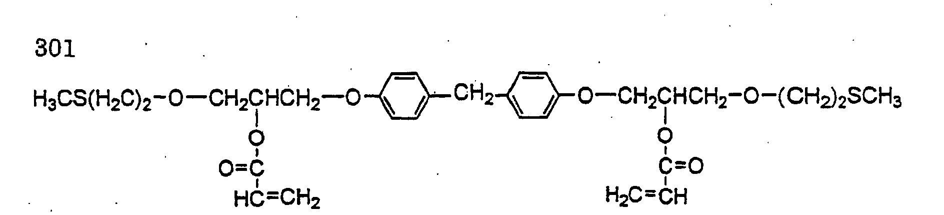

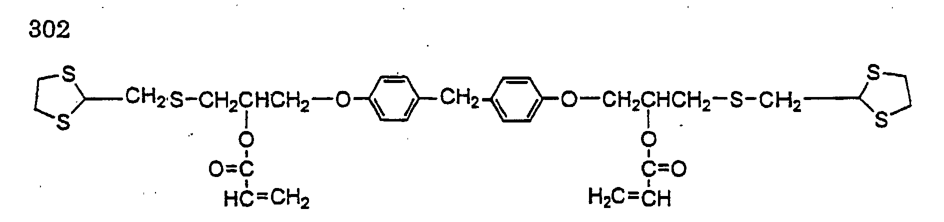

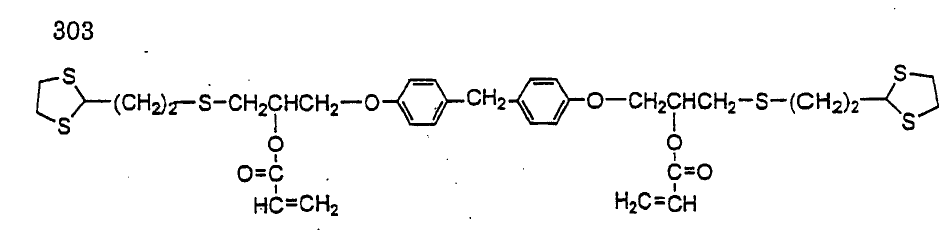

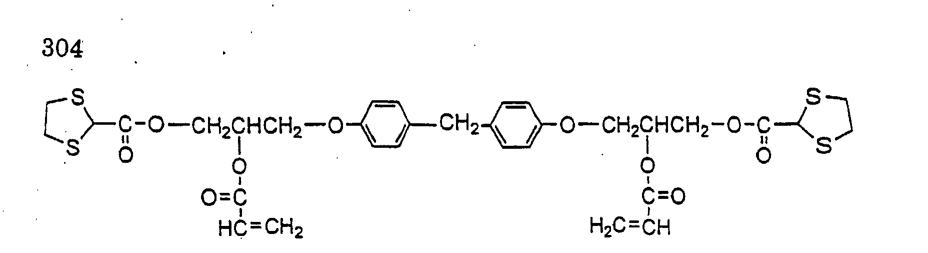

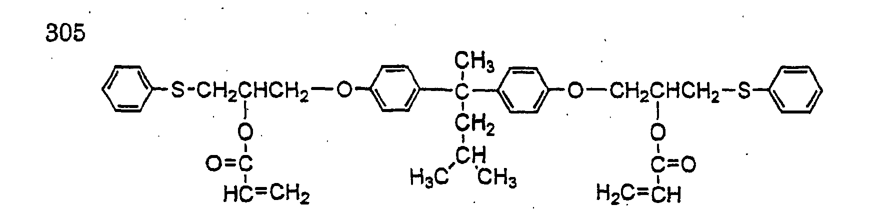

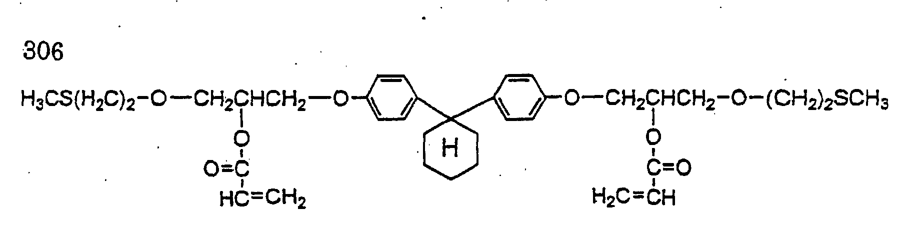

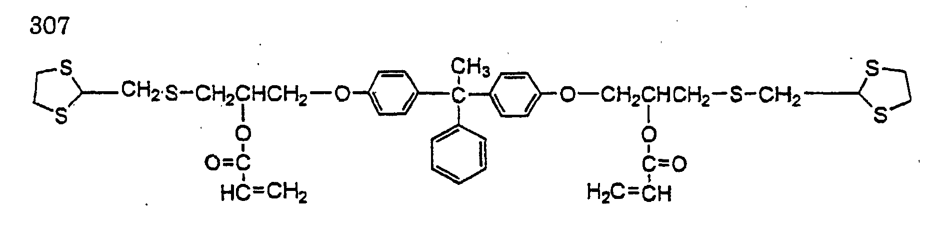

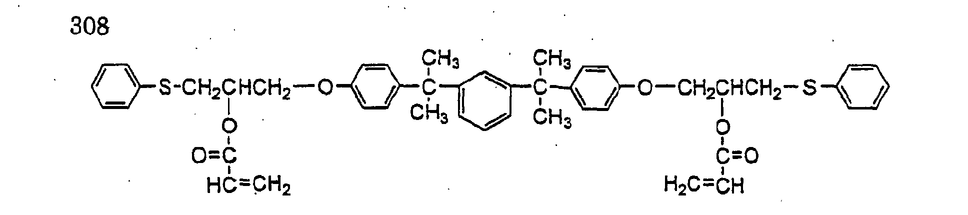

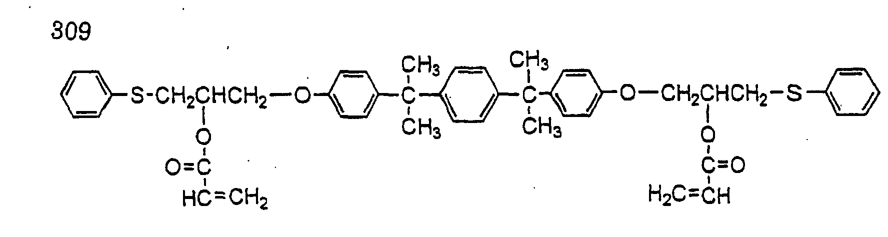

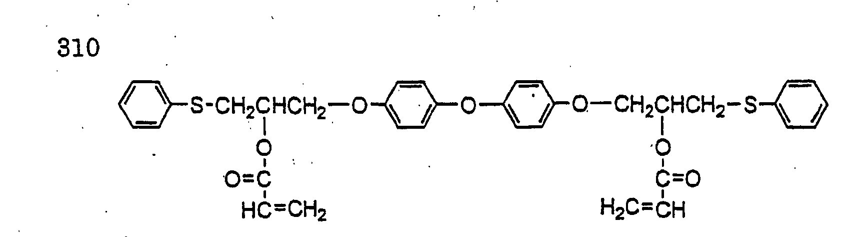

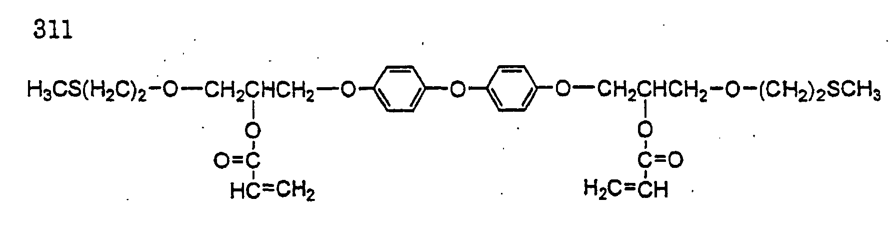

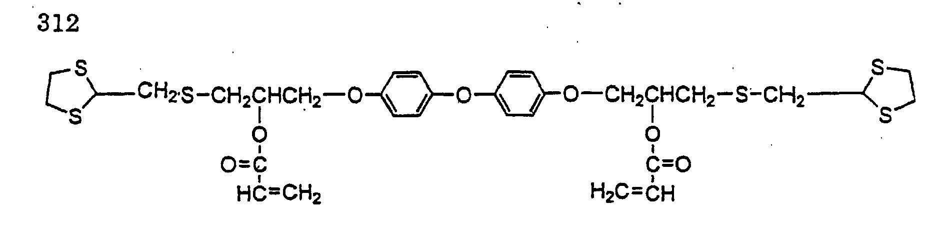

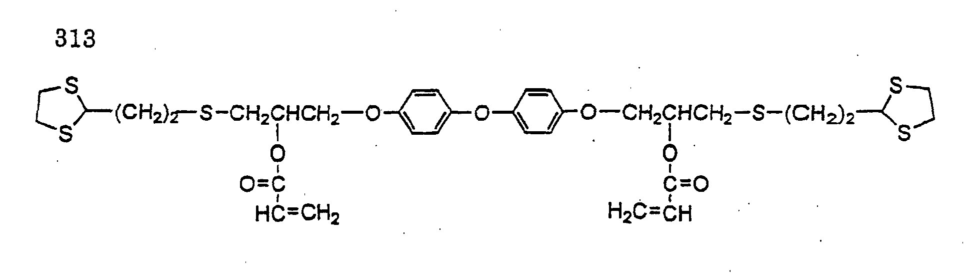

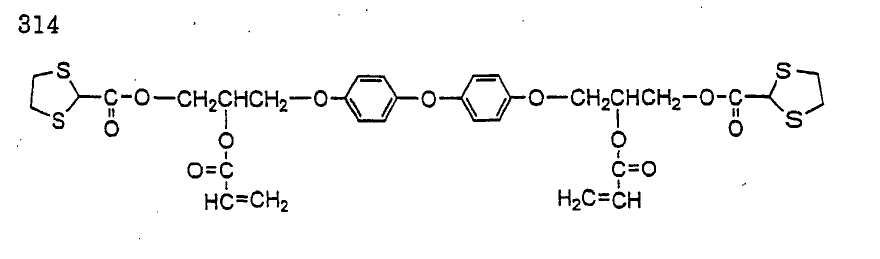

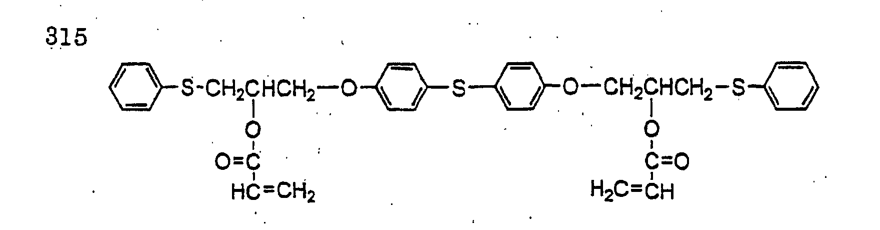

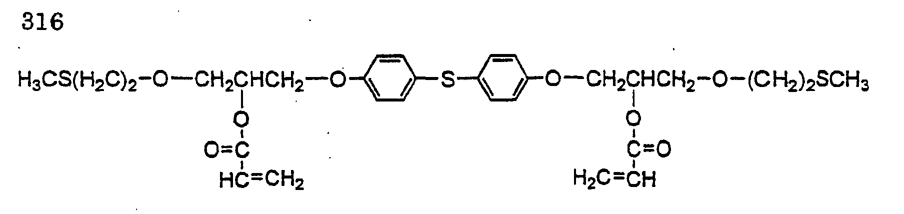

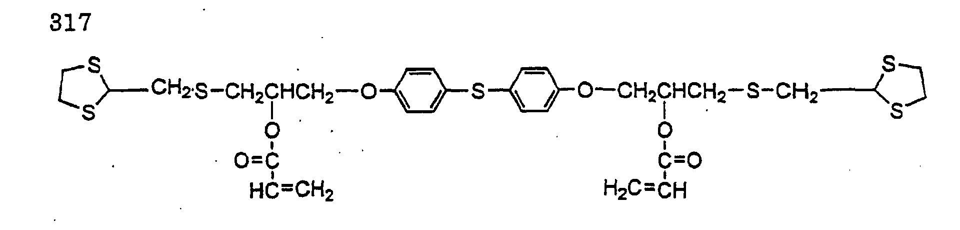

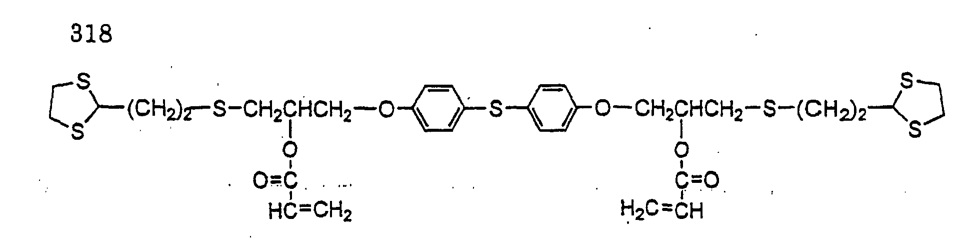

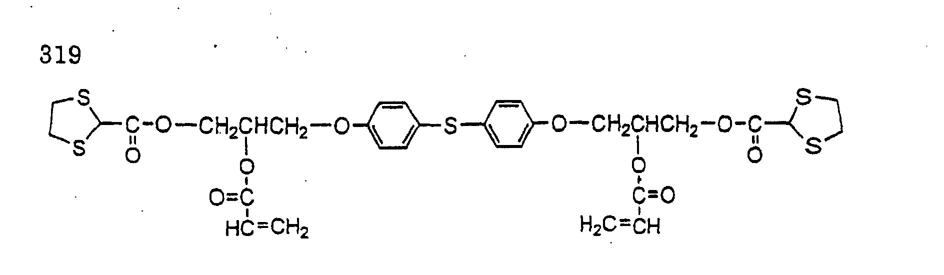

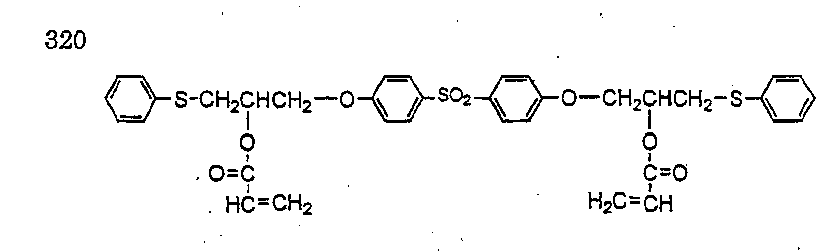

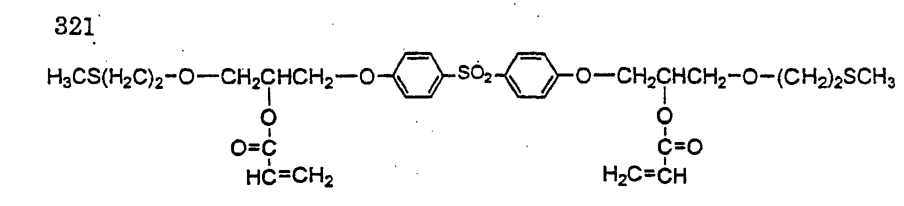

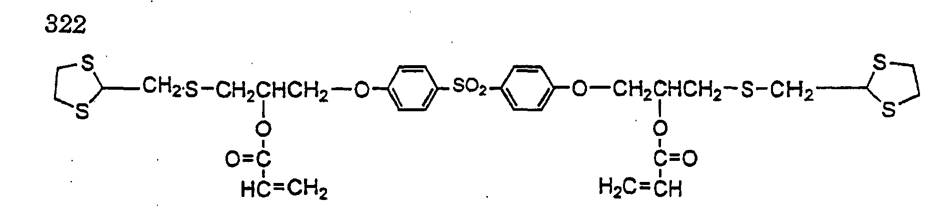

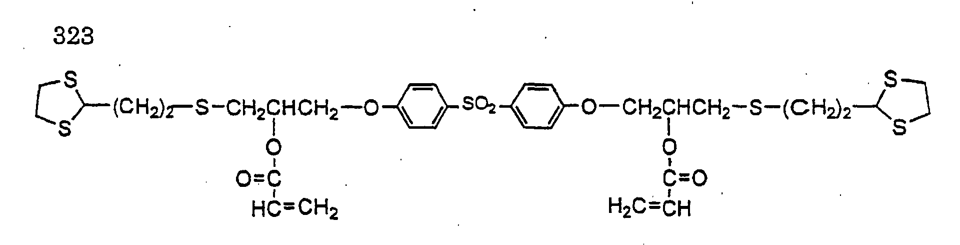

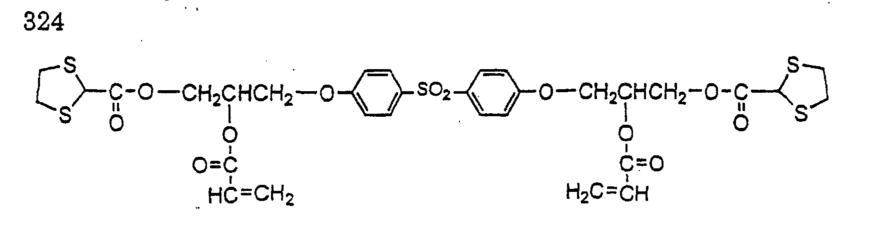

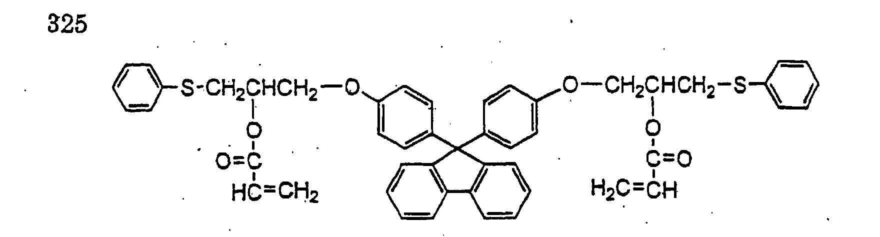

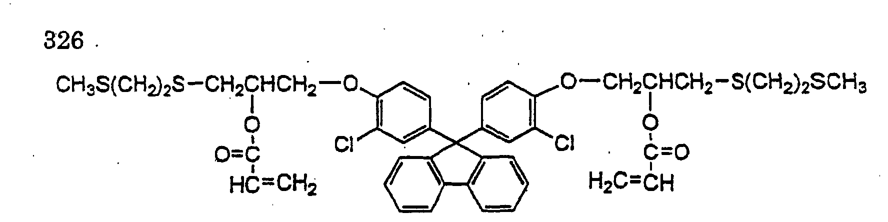

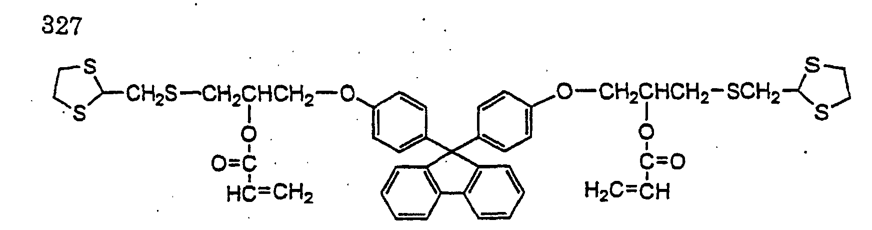

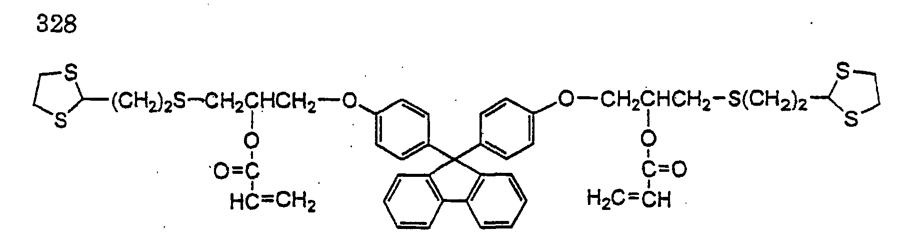

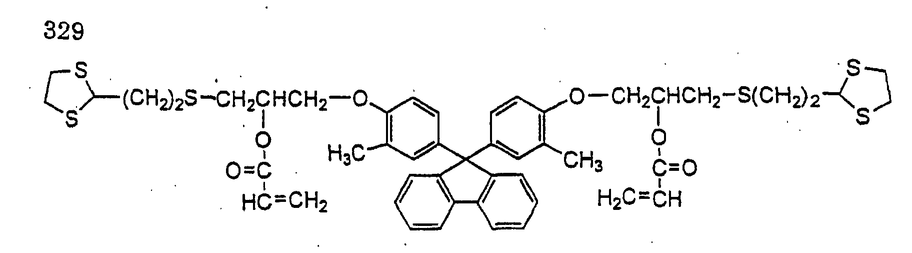

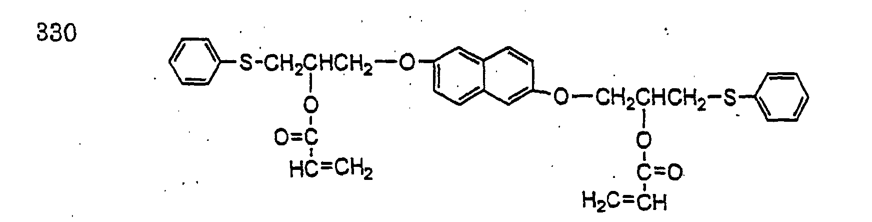

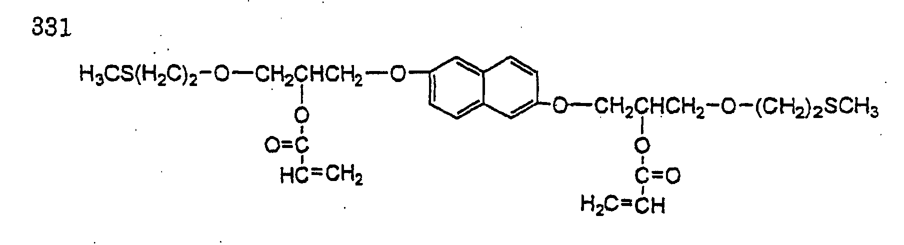

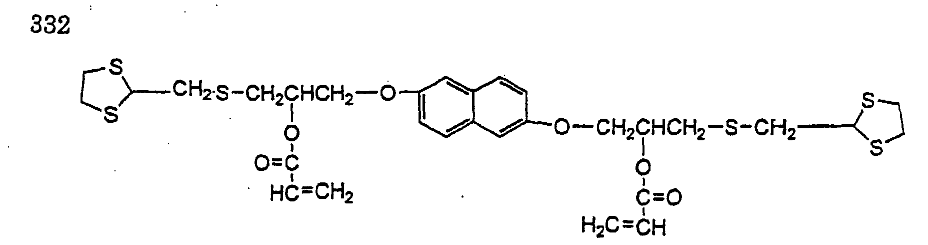

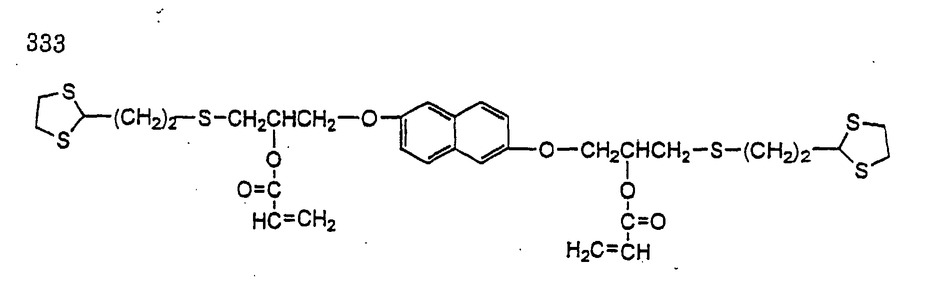

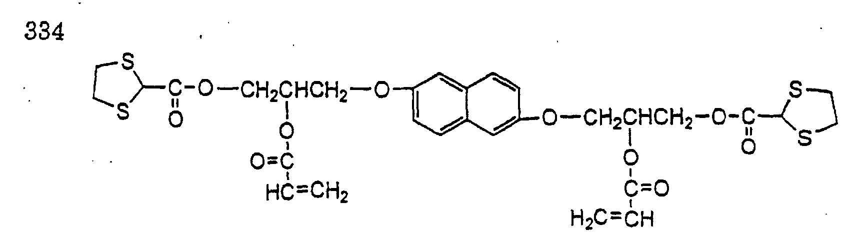

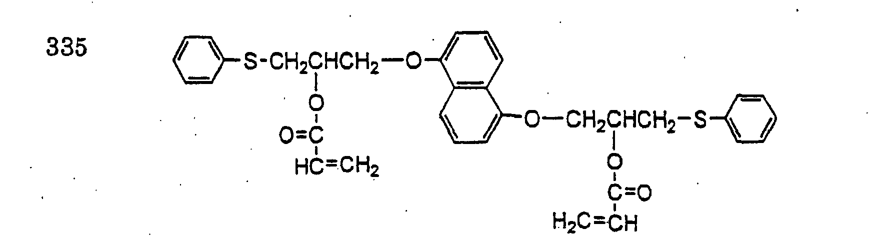

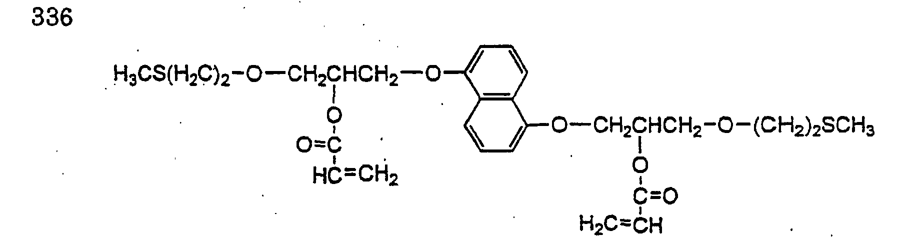

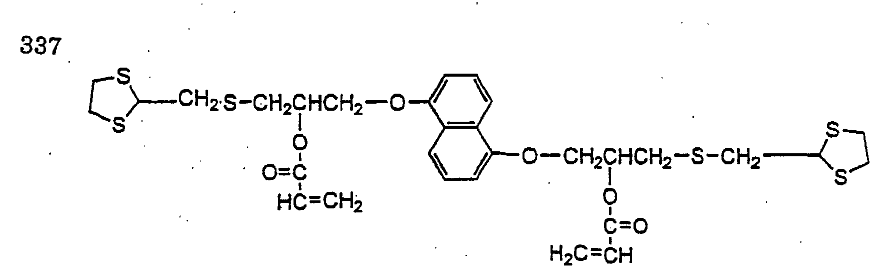

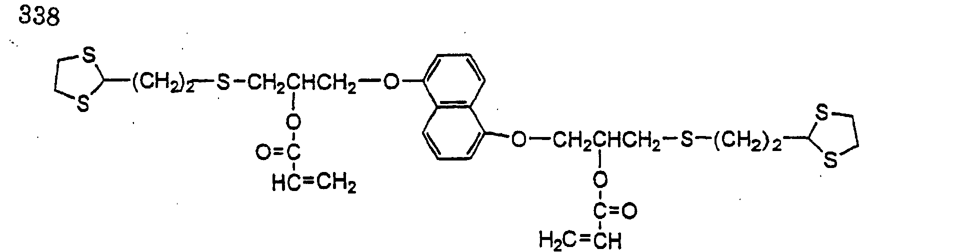

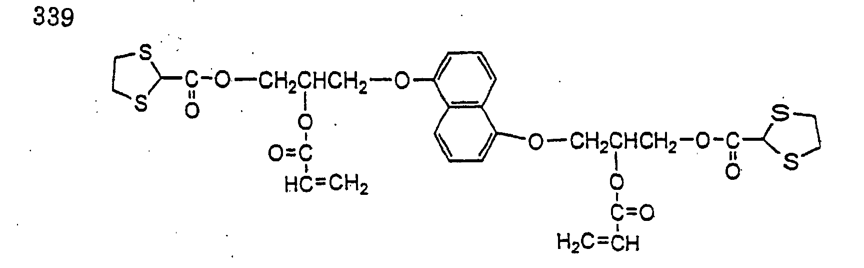

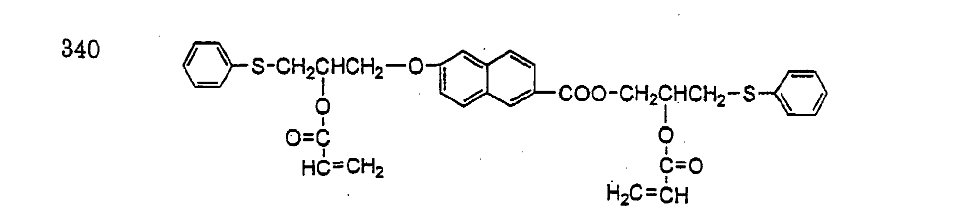

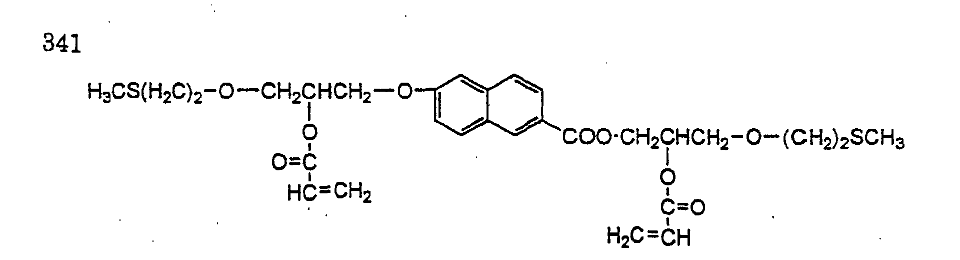

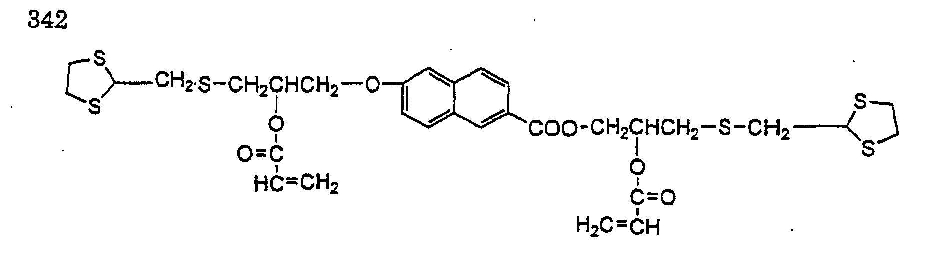

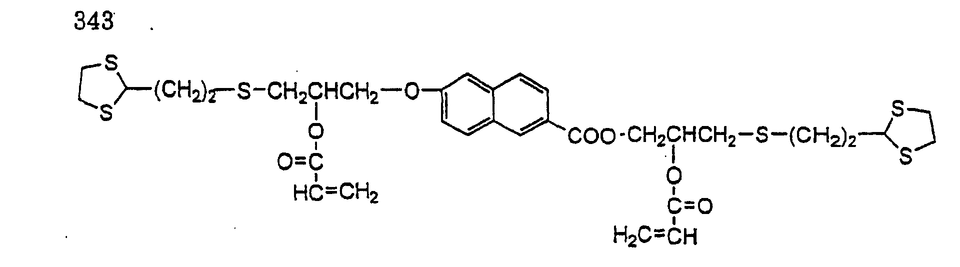

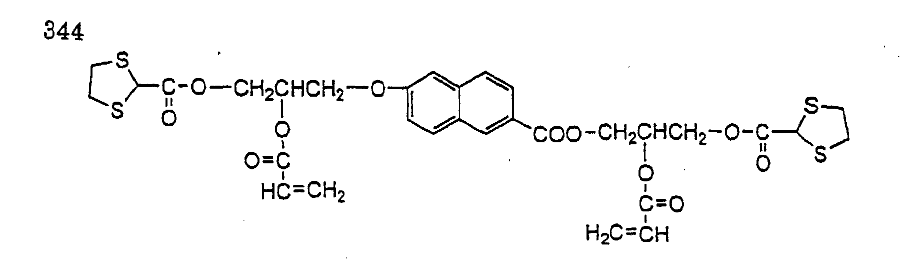

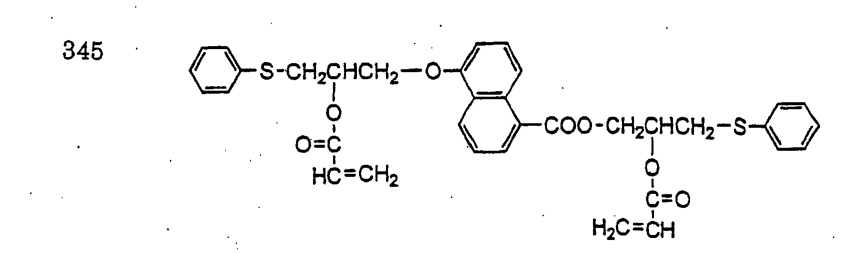

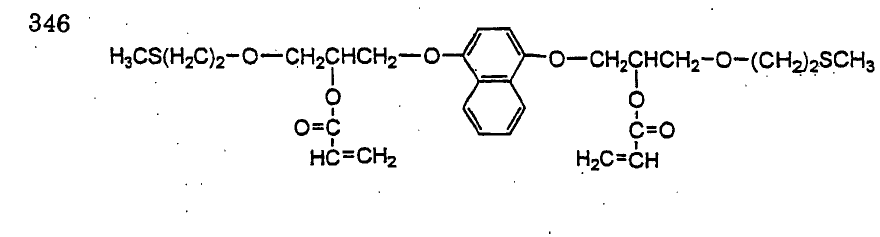

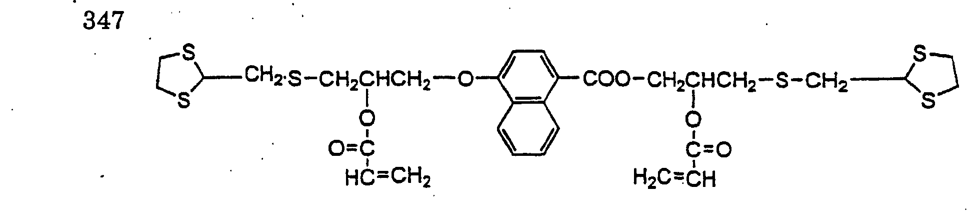

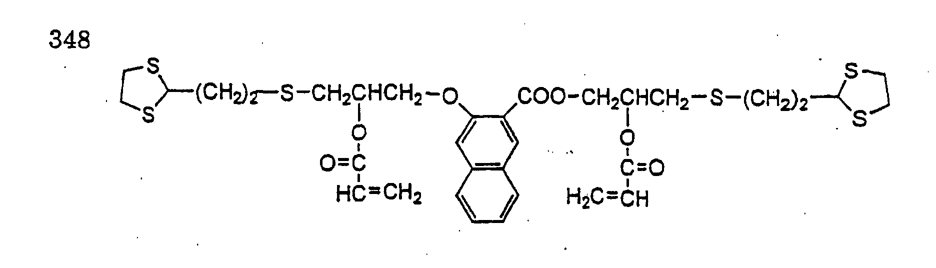

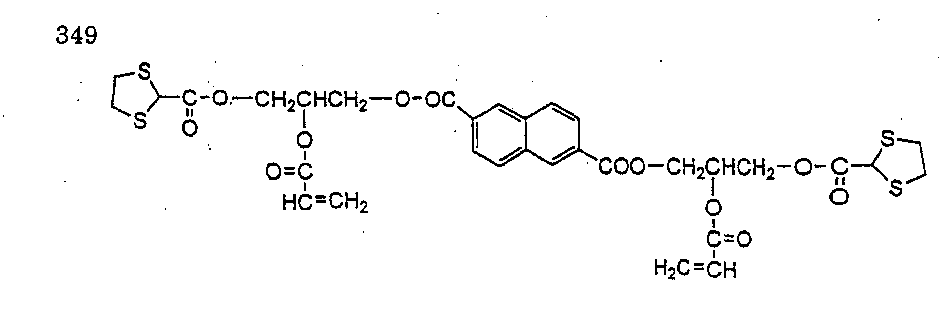

The term "dental material" used in this specification means a material widely used in the dental field, which includes the following dental composition.

The term "dental composition" means a mixture of a polymerizable monomer, a polymerization initiator, a filler, etc., and the dental composition includes an uncured composition and a composition having been cured by polymerization.

The state of the polymerizable monomer represented by the formula (1) that is used in the invention is not specifically restricted. In the use of the polymerizable monomer as a dental material, however, the monomer is preferably liquid at ordinary temperature (20°C in the invention) and preferably has a viscosity of 100 to 1,000,000 cps at ordinary temperature. The polymerizable monomer which is liquid at ordinary temperature and has the above viscosity can be favorably used as a dental material as it is without being polymerized. If the viscosity is lower than the above value, the polymerizable monomer hardly has the desired properties. If the viscosity is higher than the above value, it tends be difficult to homogeneously incorporate a sufficient amount of the later-described filler, particularly an inorganic filler such as a glass powder, into the dental composition. Moreover, mixing with other components takes time, and the polymerization reaction is not completed in a short period of time in some cases. The viscosity of the polymerizable monomer is particularly preferably in the range of 1,000 to 100,000 cps. That the monomer is liquid at ordinary temperature is advantageous from the viewpoint of ease of use, for example, the monomer can be easily used as a dental material or the monomer can easily dissolve other components and additives in the preparation of a cured product from the composition.

In the use for a dental composition, the polymerizable monomer represented by the formula (1) is not limited to a liquid and may be a solid.

The polymerizable monomer represented by the formula (1) for use in the invention exhibits a liquid refractive index, at room temperature (20°C), of preferably 1.55 to 1.65, more preferably 1.57 to 1.63. A cured product of the polymerizable monomer exhibits a refractive index, at room temperature, of preferably 1.57 to 1.65, more preferably 1.58 to 1.65. Owing to such refractive indexes, transparency of the cured composition in which the polymerizable monomer exists together with a filler, etc. can be ensured, as described later.

The polymerizable monomer represented by the formula (1) may be used singly, or may be used in combination with other monomers of the formula (1).

The dental composition of the invention further contains a polymerization initiator (B) in addition to the component (A).

### (B) Polymerization initiator

As the polymerization initiator added to the dental composition or the dental material of the invention, known compounds are employable without any restriction. For example, a photopolymerization initiator, an organic peroxide, a diazo compound and a redox compound are employable.

In case of the photopolymerization initiator, a photosensitizer only or a combination of a photosensitizer and a photopolymerization accelerator is employable.

Examples of the photosensitizers include known α-diketone compounds and phosphorus compounds, which are excited by irradiation with visible rays or ultraviolet rays to initiate polymerization, such as benzyl, camphorquinone, α-naphthyl, p,p'-dimethoxybenzyl, pentadione, 1,4-phenanthrenequinone, naphthoquinone and trimethylbenzoyldiphenylphosphine oxide. These compounds can be used singly or in combination of two or more kinds. Of these compounds, preferably used are camphorquinone and/or trimethylbenzoyldiphenylphosphine oxide.

Examples of the photopolymerization accelerators include:
tertiary amines, such as N,N-dimethylaniline, N,N-diethylaniline, N,N-dibenzylaniline, N,N-dimethyl-p-toluidine, N,N-diethyl-p-toluidine, N,N-dihydroxyethyl-p-toluidine, p-N,N-dimethylaminobenzoic acid, p-N,N-diethylaminobenzoic acid, ethyl p-N,N-dimethylaminobenzoate, ethyl p-N,N-diethylaminobenzoate, methyl p-N,N-dimethylaminobenzoate, methyl p-N,N-diethylaminobenzoate, p-N,N-dimethylaminobenzaldehyde, 2-n-butoxyethyl p-N,N-dimethylaminobenzoate, 2-n-butoxyethyl p-N,N-diethylaminobenzoate, p-N,N-dimethylaminobenzonitrile, p-N,N-diethylaminobenzonitrile, p-N,N-dihydroxyethylaniline, p-dimethylaminophenetyl alcohol, N,N-dimethylaminoethyl methacrylate, triethylamine, tributylamine, tripropylamine and N-ethyl ethanolamine;
combinations of the above tertiary amines and citric acid, malic acid or 2-hydroxypropanoic acid;
barbituric acids, such as 5-butylaminobarbituric acid and 1-benzyl-5-phenylbarbituric acid; and
organic peroxides, such as benzoyl peroxide and di-tert-butyl peroxide.

The above compounds can be used singly or in combination. Of these, preferably used are tertiary aromatic amines wherein a nitrogen atom is directly bonded to an aromatic ring and tertiary aliphatic amines having a polymerizable group, such as ethyl p-N,N-dimethylaminobenzoate, methyl p-N,N-dimethylaminobenzoate, 2-n-butoxyethyl p-N,N-dimethylaminobenzoate and N,N-dimethylaminoethyl methacrylate.

In order to promote curing without delay, a combination of a photosensitizer and a photopolymerization accelerator is preferable, and preferably used is a combination of camphorquinone or trimethylbenzoyldiphenylphosphine oxide and an ester compound of a tertiary aromatic amine wherein a nitrogen atom is directly bonded to an aromatic ring, such as ethyl p-N,N-dimethylaminobenzoate or 2-n-butoxyethyl p-N,N-dimethylaminobenzoate.

The type of the organic peroxide or the diazo compound used as the polymerization initiator is not specifically restricted, but in order to complete the polymerization in a short period of time, a compound whose decomposition half-life at 80°C is not more than 10 hours is preferable.

Examples of the organic peroxides include:
diacyl peroxides, such as acetyl peroxide, isobutyl peroxide, decanoyl peroxide, benzoyl peroxide and succinic acid peroxide;
peroxydicarbonates, such as diisopropyl peroxydicarbonate, di-2-ethylhexyl peroxydicarbonate and diallyl peroxydicarbonate;
peroxy esters, such as tert-butyl peroxyisobutyrate, tert-butyl neodecanate and cumene peroxyneodecanate; and
sulfonyl peroxides, such as acetylcyclohexylsulfonyl peroxide.

Examples of the diazo compounds include 2,2'-azobisisobutyronitrile, 4,4'-azobis(4-cyanovaleric acid), 2,2'-azobis(4-methoxy-2,4-dimethoxyvaleronitrile) and 2,2'-azobis(2-cyclopropylpropionitrile).

Of the above compounds, preferably used are benzoyl peroxide and 2,2'-azobisbutyronitrile.

The type of the redox initiator used as the polymerization initiator is not specifically restricted. For example, there can be mentioned a combination of the organic peroxide and the tertiary amine; a combination of the organic peroxide/sulfinic acid or its alkali metal salt/the tertiary amine; and a combination of an inorganic peroxide and an inorganic reducing agent, such as a combination of an inorganic peroxide (e.g., potassium persulfate) and sodium sulfite or a combination of an inorganic peroxide and sodium hydrogensulfite.

Of these, preferably used are a combination of benzoyl peroxide and N,N-dimethyl-p-toluidine and a combination of benzoyl peroxide and N,N-dihydroxyethyl-p-toluidine.

The polymerization initiator is used in an amount of preferably 0.001 to 10 % by weight based on 100 parts by weight of the (meth)acrylate monomer.

### (C) Filler

The dental composition of the invention contains a filler. The filler is added for the purposes of ensuring mechanical strength, enhancing light transmittance, imparting X-ray opacity and decreasing polymerization shrinkage. In the present invention, the filler (C) is the following glass powder or the following composite filler consisting of the glass powder and a polymer, and usually, the filler (C) does not include a filler composed of an organic compound only.

An inorganic compound used as the filler in the invention is generally a glass powder, and the glass powder has an average particle diameter of usually not more than 2 µm, preferably 0.1 to 1.5 µm. When the particle diameter is in this range, the specific surface area of the filler is not increased markedly. Hence, the relative proportion of the filler to the polymerizable monomer is greatly increased, and therefore the polymerization shrinkage can be effectively decreased. The refractive index of the glass powder is preferably not less than 1.55, particularly preferably 1.57 to 1.65. The difference between the refractive index of the glass powder for use in the invention and the refractive index of a cured product (resin matrix) of the polymerizable monomer in the dental composition containing this glass powder is preferably not more than 0.05, particularly preferably not more than 0.02. That is to say, the glass powder for use in the invention has a refractive index very approximate to that of a cured product of the polymerizable monomer. By the use of such a glass powder, the light transmittance of a cured product of the dental composition of the invention can be enhanced. By the use of such a glass powder, further, the strength of the cured product can be enhanced.

In general, it is clinically important that the presence of the filling material can be clearly confirmed by X-ray photograph, so that the glass powder for use in the invention is preferably one having X-ray opacity. In order to impart X-ray opacity to the glass powder, an element having X-ray opacity (heavy metal element), such as barium, strontium, zirconium, bismuth, tungsten, germanium, molybdenum or lanthanide, is usually added as a glass-constituting element.

With increase of the amount of the heavy metal element that is added in order to impart X-ray opacity to the glass powder, the refractive index of the glass powder becomes higher. Accordingly, the heavy metal element of the glass powder in the dental composition of the invention has a function of imparting X-ray opacity to the glass powder and a function of controlling the refractive index of the glass powder.

If the difference between the refractive index of the cured product of the monomer such as (meth)acrylate and the refractive index of the glass powder is larger than 0.05, the transparency of the cured composition is lowered, and lowering of the photo-curability results in decrease of the curing depth or insufficient curing reaction, whereby properties of the cured product are sometimes lowered. When the refractive index of the glass powder is controlled, it is preferable to adjust the refractive index between the refractive index of the monomer in the uncured state and the refractive index of the cured product of the monomer. This is advantageous in that there is no change between the transparency of the composition before curing and the transparency of the composition after curing. Further, by allowing the refractive index of the cured product of the monomer and the refractive index of the glass powder to agree with each other, the transparency of the composition after curing becomes the highest. The methods to control the refractive index can be properly used according to the requirements for the clinical site.

In case of the dental composition of the invention containing not less than about 60 % by weight of a glass powder of X-ray opacity having an average particle diameter of about sub micron to several µm, if the refractive index of the glass powder contained in the composition is not less than 1.50, the cured product of the composition becomes transparent, and the presence of the cured product can be clearly confirmed by X-rays. The glass powder used herein may be one kind of a glass powder or a mixture of two or more kinds of glass powders having different formulations. When plural kinds of glass powders having different formulations are used, the refractive indexes of the glass powders are approximated to each other, and thereby high transparency of a cured product of the dental composition of the invention can be ensured.

The glass powder is used in an amount of usually 5 to 2000 parts by weight, preferably 100 to 700 parts by weight, based on 100 parts by weight of the polymerizable monomer.

In the dental composition of the invention, a composite filler may be used as the filler.

The composite filler for use in the invention can be prepared by, for example, mixing a polymerizable monomer, a glass powder and a thermal polymerization initiator such as benzoyl peroxide, then thermally polymerizing the mixture and pulverizing the resulting polymer.

By the use of the composite filler, polymerization shrinkage of the dental composition of the invention can be effectively reduced in the curing stage. Since the composite filler is prepared by polymerizing the polymerizable monomer under such conditions (e.g., thermal polymerization conditions) as are capable of raising the degree of polymerization higher than that in the photopolymerization conditions, the cured product exhibits higher mechanical properties than a cured product obtained by photopolymerization. Accordingly, by the incorporation of such a composite filler, mechanical properties and abrasion resistance of the cured product of the dental composition of the invention are improved. Moreover, by allowing the refractive index of a cured product of a mixture of the glass powder used for the composite filler and a polymerizable monomer for constituting the composite filler to agree with the refractive index of the aforesaid polymerizable monomer cured product and the refractive index of the glass powder, higher transparency can be obtained. To the composite filler, fine particle silica, a filler other than the glass powder, such as metallic oxide, a pigment, etc. may be added when needed. The average particle diameter of the composite filler is in the range of usually 1 to 100 µm, preferably 5 to 20 µm.

The composite filler is used in an amount of usually 1 to 2000 parts by weight, preferably 5 to 2000 parts by weight, more preferably 50 to 700 parts by weight, based on 100 parts by weight of the polymerizable monomer.

In the present invention, fine particle silica can be used as the filler in addition to the glass powder.

The fine particle silica employable herein is usually high-purity colloidal silica prepared by a gas phase process, and has no X-ray opacity. Although the fine particle silica has no X-ray opacity, it is sometimes added for the purpose of controlling viscosity or tackiness of a paste. As the fine particle silica, high-purity colloidal silica prepared by, for example, a gas phase process may be used as it is, or high-purity colloidal silica may be used after hydrophobic treatment with a silane compound such as dimethyldichlorosilane. It is preferable to subject the fine particle silica to methacryloxysilane treatment or aminosilane treatment to increase affinity of the fine particle silica for the resin matrix, prior to use. The fine particle silica has a low refractive index of 1.45, and this refractive index is widely different from that of the filler used. However, the average particle diameter of the fine particle silica is usually not more than 0.05 µm and is considerably smaller than 0.3 to 0.7 µm that is a wavelength of visible ray, so that the fine particle silica does not greatly hinder the transparency. Fine particle silica of smaller size can maintain transparency high, and hence it is preferable to use fine particle silica having an average particle diameter of not more than 0.01 µm from the viewpoint of maintenance of high transparency.

The fine particle silica is used in an amount of usually 5 to 250 parts by weight, preferably 10 to 200 parts by weight, particularly preferably 20 to 150 parts by weight, based on 100 parts by weight of the (meth)acrylate monomer contained in the dental composition of the invention.

### (D) Another polymerizable monomer employable in addition to the polymerizable monomer (A) represented by the formula (1)

The polymerizable monomer used in combination with the polymerizable monomer represented by the formula (1) is usually a (meth)acrylate monomer or a (meth)acrylamide monomer.

The polymerizable monomer is, for example, a monofunctional monomer, a polyfunctional monomer which forms a crosslinked structure to effectively enhance physical properties after polymerization, or an acid group-containing monomer which is added for the purpose of imparting, to the composition, properties of adhesion to dentin or the like.

Examples of the monofunctional monomers for use in the invention include:
alkyl esters of (meth)acrylic acid, such as methyl (meth)acrylate, ethyl (meth)acrylate, butyl (meth)acrylate, hexyl (meth)acrylate, 2-ethylhexyl (meth)acrylate, dodecyl (meth)acrylate, lauryl (meth)acrylate, cyclohexyl (meth)acrylate, benzyl (meth)acrylate, isobornyl (meth)acrylate and adamantyl (meth)acrylate;
hydroxyalkyl esters of (meth)acrylic acid, such as 2-hydroxyethyl (meth)acrylate, 2- or 3-hydroxypropyl (meth)acrylate, 4-hydroxybutyl (meth)acrylate, 5-hydroxypentyl (meth)acrylate, 6-hydroxyhexyl (meth)acrylate, 1,2- or 1,3-dihydroxypropyl mono(meth)acrylate and erythritol mono(meth)acrylate;
polyalkylene glycol mono(meth)acrylates, such as diethylene glycol mono(meth)acrylate, triethylene glycol mono(meth)acrylate, polyethylene glycol mono(meth)acrylate and polypropylene glycol mono(meth)acrylate;
(poly)glycol monoalkyl ether (meth)acrylates, such as ethylene glycol monomethyl ether (meth)acrylate, ethylene glycol monoethyl ether (meth)acrylate, diethylene glycol monomethyl ether (meth)acrylate, triethylene glycol monomethyl ether (meth)acrylate, polyethylene glycol monomethyl ether (meth)acrylate and polypropylene glycol monoalkyl ether (meth)acrylate;
fluoroalkyl esters of (meth)acrylic acid, such as perfluorooctyl (meth)acrylate and hexafluorobutyl (meth)acrylate;
silane compounds having (meth)acryloxyalkyl group, such as γ-(meth)acryloxypropyltrimethoxysilane and γ-(meth)acryloxypropyltri(trimethylsiloxy)silane;
carboxylic acid-containing (meth)acrylates, such as β-methacryloyloxyethyl hydrogenphthalate, β-methacryloyloxyethyl hydrogensuccinate and β-methacryloyloxyethyl maleate;
halogen-containing (meth)acrylates, such as 3-chloro-2-hydroxypropyl methacrylate; and
(meth)acrylates having heterocyclic ring, such as tetrafurfuryl (meth)acrylate.

Examples of the polyfunctional monomers include:
poly(meth)acrylates of alkanepolyols, such as ethylene glycol di(meth)acrylate, propylene glycol di(meth)acrylate, butylene glycol di(meth)acrylate, neopentyl glycol di(meth)acrylate, hexylene glycol di(meth)acrylate, trimethylolpropane tri(meth)acrylate and pentaerythritol tetra(meth)acrylate;
polyoxyalkanepolyol poly(meth)acrylates, such as diethylene glycol di(meth)acrylate, triethylene glycol di(meth)acrylate, polyethylene glycol di(meth)acrylate, dipropylene glycol di(meth)acrylate, polypropylene glycol di(meth)acrylate, dibutylene glycol di(meth)acrylate and dipentaerythritol hexa(meth)acrylate; and
aliphatic, alicyclic or aromatic (meth)acrylates represented by the following formula: 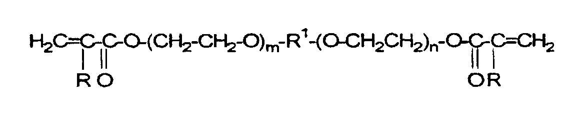
wherein R is a hydrogen atom or a methyl group, m and n are each 0 or a positive integer, and R¹ is any one of the following aromatic groups.

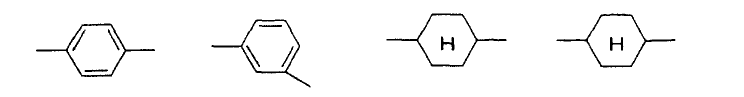

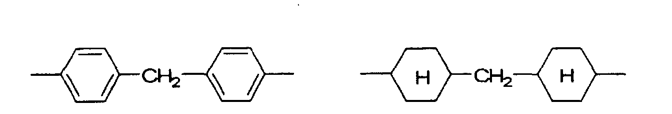

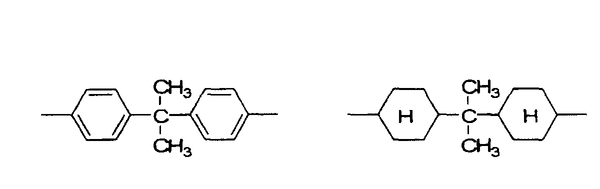

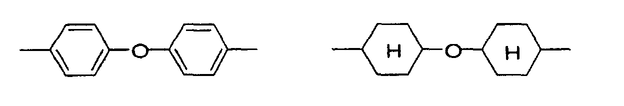

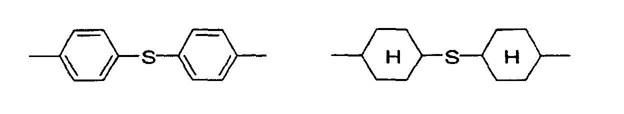

Also available are alicyclic or aromatic epoxy di(meth)acrylates represented by the following formula: 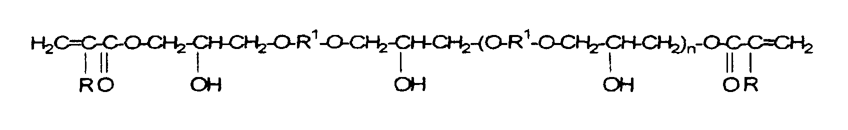 wherein R is a hydrogen atom or a methyl group, n is 0 or a positive integer, and R¹ is -(CH₂)₂-, -(CH₂)₄- or any one of the following aromatic groups.

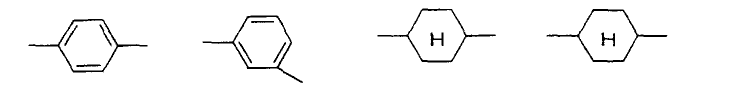

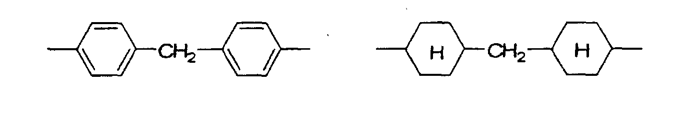

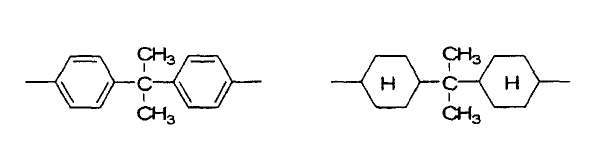

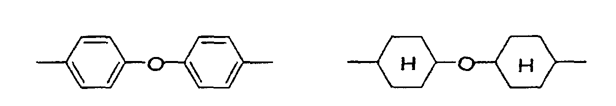

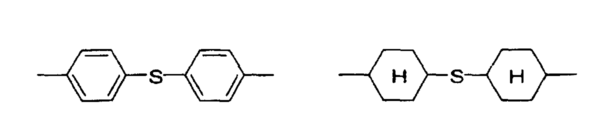

Also available are polyfunctional (meth)acrylates having an urethane bond in the molecule, which are represented by the following formula: 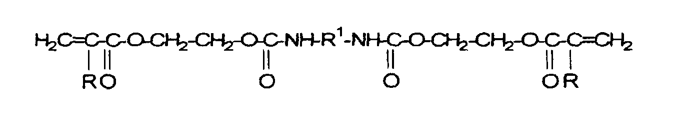 wherein R is a hydrogen atom or a methyl group, and R¹ is -(CH₂)₂-, -(CH₂)₄-, -(CH₂)₆- or any one of the following groups.

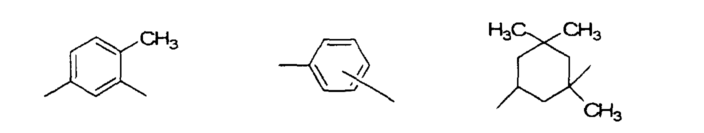

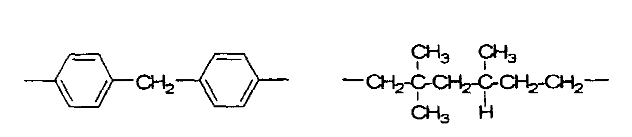

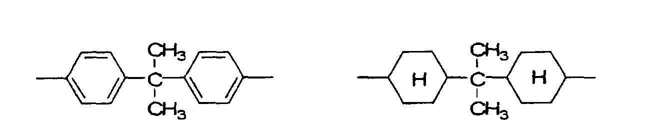

Examples of the acid group-containing monomers for use in the invention are described below. As monomers having at least one carboxyl group in one molecule, there can be mentioned monocarboxylic acids, dicarboxylic acids, tricarboxylic acids, tetracarboxylic acids, and their derivatives. Examples of such acids and derivatives include (meth)acrylic acid, maleic acid, p-vinylbenzoic acid, 11-(meth)acryloyloxy-1,1-undecanedicarboxylic acid (MAC-10), 1,4-di(meth)acryloyloxyethylpyromellitic acid, 6-(meth)acryloyloxyethylnaphthalene-1,2,6-tricarboxylic acid, 4-(meth)acryloyloxymethyltrimellitic acid and anhydride thereof, 4-(meth)acryloyloxyethyltrimellitic acid and anhydride thereof, 4-(meth)acryloyloxybutyltrimellitic acid and anhydride thereof, 4-[2-hydroxy-3-(meth)acryloyloxy]butyltrimellitic acid and anhydride thereof, 2,3-bis(3,4-dicarboxybenzoyloxy)propyl (meth)acrylate, N-o-di(meth)acryloyloxytyrosine, o-(meth)acryloyloxytyrosine, N-(meth)acryloyloxytyrosine, N-(meth)acryloyloxyphenylalanine, N-(meth)acryloyl-p-aminobenzoic acid, N-(meth)acryloyl-o-aminobenzoic acid, N-(meth)acryloyl-5-aminosalicylic acid, N-(meth)acryloyl-4-aminosalicylic acid, 2-, 3- or 4-(meth)acryloyloxybenzoic acid, an addition product (PMDM) of 2-hydroxyethyl (meth)acrylate with pyromellitic dianhydride, an addition product of 2-hydroxyethyl (meth)acrylate with maleic anhydride, an addition product (BTDA) of 2-hydroxyethyl (meth)acrylate with 3,3',4,4'-benzophenonetetracarboxylic dianhydride, an addition product of 2-hydroxyethyl (meth)acrylate with 3,3',4,4'-biphenyltetracarboxylic dianhydride, 2-(3,4-dicarboxybenzoyloxy)-1,3-di(meth)acryloyloxypropane, an addition product of N-phenylglycine or N-tolylglycine with glycidyl (meth)acrylate, 4-[(2-hydroxy-3-(meth)acryloyloxypropyl)amino]phthalic acid, and 3- or 4-[N-methyl-N-(2-hydroxy-3-(meth)acryloyloxypropyl)amino]phthalic acid.

As monomers having at least one phosphoric acid group in one molecule, there can be mentioned, for example, 2-(meth)acryloyloxyethyl acid phosphate, 2- or 3-(meth)acryloyloxypropyl acid phosphate, 4-(meth)acryloyloxybutyl acid phosphate, 6-(meth)acryloyloxyhexyl acid phosphate, 8-(meth)acryloyloxyoctyl acid phosphate, 10-(meth)acryloyloxydecyl acid phosphate, 12-(meth)acryloyloxydodecyl acid phosphate, bis{2-(meth)acryloyloxyethyl}acid phosphate, bis{2- or 3-(meth)acryloyloxypropyl}acid phosphate, 2-(meth)acryloyloxyethylphenyl acid phosphate and 2-(meth)acryloyloxyethyl-p-methoxyphenyl acid phosphate. In these compounds, the phosphoric acid group can be replaced with a thiophosphoric acid group.

As monomers having at least one sulfonic acid group in one molecule, there can be mentioned, for example, 2-sulfoethyl (meth)acrylate, 2- or 1-sulfo-1- or -2-propyl (meth)acrylate, 1- or 3-sulfo-2-butyl (meth)acrylate, 3-bromo-2-sulfo-2-propyl (meth)acrylate, 3-methoxy-1-sulfo-2-propyl (meth)acrylate and 1,1-dimethyl-2-sulfoethyl (meth)acrylamide.

Of the above compounds, particularly preferable are (meth)acrylic acid esters, which are low-poisonous, can be rapidly polymerized, hardly undergo hydrolysis and are readily prepared. As the monofunctional polymerizable (meth)acrylic acid esters, particularly preferably used are, for example, alkyl methacrylates, such as methyl methacrylate (refractive index: 1.42) and ethyl methacrylate (refractive index: 1.42); hydroxyl group-containing (meth)acrylates, such as 2-hydroxyethyl methacrylate (refractive index: 1.45); and (meth)acrylates having ethylene glycol chain in the molecule, such as diethylene glycol monomethyl ether methacrylate (refractive index: 1.44) and tetraethylene glycol monomethyl ether methacrylate (refractive index: 1.45).

As the polyfunctional polymerizable (meth)acrylic acid esters, particularly preferably used are, for example, di(meth)acrylates having ethylene glycol chain in the molecule, such as ethylene glycol dimethacrylate (refractive index: 1.45) and triethylene glycol dimethacrylate (refractive index: 1.46); and compounds represented by the following formulas: 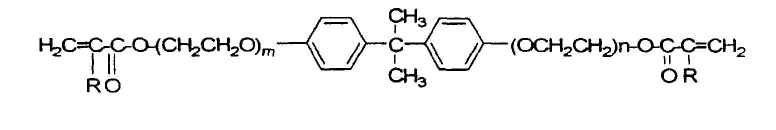 wherein R is a methyl group and m+n is 2.6 on the average (refractive index: 1.54), 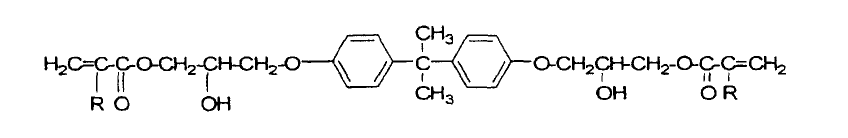 wherein R is a methyl group (refractive index: 1.54), 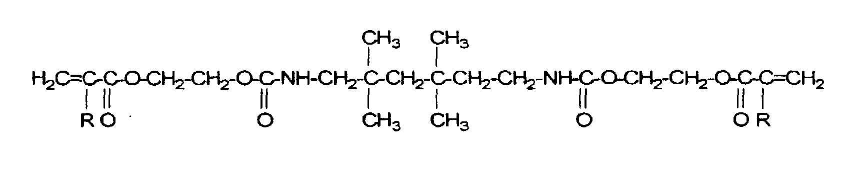 wherein R is a methyl group (refractive index: 1.48).

As the acid group-containing monomers, particularly preferably used are, for example, 11-methacryloyloxy-1,1-undecanedicarboxylic acid, 4-methacryloyloxyethyltrimellitic anhydride, N-methacryloyl-5-aminosalicylic acid, 2-(meth)acryloyloxyethylphenyl acid phosphate, 10-(meth)acryloyloxydecyl acid phosphate and 2-methyl-2-(meth)acrylamidopropanesulfonic acid.

The acid group-containing monomer is used in an amount of usually 0.1 to 50 parts by weight, preferably 0.5 to 20 parts by weight, particularly preferably 1 to 10 parts by weight, based on 100 parts by weight of the (meth)acrylate monomer contained in the dental composition of the invention.

In the dental composition of the invention, it is desirable to select and use the (meth)acrylate monomer in such a manner that the difference between the refractive index of a cured product of a mixture of the polymerizable monomer represented by the formula (1) and this monomer and the refractive index of the glass powder added as a filler should become not more than 0.05, particularly 0 to 0.02.

By the polymerization of the (meth)acrylate monomer, the refractive index of a cured product of the monomer usually becomes higher than the refractive index of the monomer by about 0.02 to 0.03. Further, when a polar group (e.g., hydroxyl group, carboxylic acid group), an aromatic ring, a heavy element (e.g., halogen atom) or a sulfur element is incorporated into the (meth)acrylate monomer, the refractive index of the (meth)acrylate monomer generally tends to become higher than that of the monomer not containing such a group or an element. The refractive index of the monomer containing such a group or a heavy element is in the range of usually 1.48 to 1.54. On the other hand, the refractive index of a monomer having only an alkyl group as its structure or a (meth)acrylic monomer having a fluoroalkyl group tends to become lower that that of a monomer not having such a group, and the refractive index of the (meth)acrylic monomer is in the range of usually 1.40 to 1.48.

The content of other polymerizable monomers in the dental composition of the invention is in the range of preferably 5 to 50 % by weight, particularly preferably 10 to 30 % by weight.

The dental composition of the invention contains at least the polymerizable monomer represented by the formula (1), the filler and the polymerization initiator, and to the composition, other components, such as pigment, dye, stabilizer, polymer powder and ultraviolet light absorber, may be added when needed, within limits not detrimental to the desired effects.

In the process for preparing the composition of the invention, the polymerizable monomer (A) represented by the formula (1), the polymerization initiator (B), the filler (C), and if desired, the polymerizable monomer (D) other than the polymerizable monomer (A) are mixed to carry out polymerization reaction. Since it is necessary that the polymerizable composition should not be contaminated with insoluble matters or foreign matters, the composition may be filtered to remove them prior to polymerization. It is more preferable to sufficiently defoam the composition under reduced pressure prior to polymerization curing, because introduction of bubbles into the cured product can be prevented.

The dental composition of the invention can undergo polymerization reaction by irradiation with ultraviolet rays, visible rays or the like, similarly to the conventional photopolymerizable materials. Examples of light sources employable for the irradiation include fluorescent lamp, various mercury lamps, xenon lamp, tungsten lamp, halogen lamp and sunlight. The light irradiation time is in the range of 1 second to 5 minutes. The temperature for the photopolymerization is in the range of usually 0 to 100°C, preferably 5 to 60°C. It is preferable to complete the polymerization curing in the shortest time at ordinary temperature in consideration of particularity of the circumstances where the composition is used, such as situation of the dental treatment and a burden on the patient, and the formulation of the composition may be adjusted so as to complete the polymerization curing in a period of 1 to 30 minutes.

The dental composition of the invention satisfies requirements for dental materials or compositions, such as mechanical strength, abrasion resistance, water resistance, curability and stain resistance, and has excellent and well-balanced transparency and X-ray opacity though the transparency and the X-ray opacity are hardly compatible with each other. The light transmittance (transparency) of a cured product of the dental composition is preferably not less than 1 %, more preferably not less than 5 %. The X-ray opaque value of the dental composition is in the range of preferably 100 to 1000 % (based on aluminum), more preferably 200 to 800 % (based on aluminum), still more preferably more than 300 % and not more than 800 % (based on aluminum), particularly preferably 400 to 800 % (based on aluminum). The composition of the invention further has a property of small polymerization shrinkage in addition to the above properties. On this account, the composition of the invention can be used for restoration of a defected part or filling of a cavity drilled, and besides the composition can be widely used for joining or adhesion of dentin of front tooth or the like, preparation of temporary tooth, bonding of bridge, adhesion of facing crown, etc.

For the dental material of the invention, the polymerizable monomer (A) represented by the formula (1) can be used singly or in combination with plural different polymerizable monomers.

The polymerizable monomer is preferably liquid at ordinary temperature and desirably has a viscosity of 100 to 1,000,000 cps, preferably 1,000 to 100,000 cps. This monomer is used as, for example, a primer that is used for undercoating before application of a resin adhesive. To the polymerizable monomer, additives, such as germicide, disinfectant, stabilizer and preservative, may be added when needed, within limits not detrimental to the effects of the invention.

The present invention includes a dental material containing, together with the polymerizable monomer, a polymerization initiator that is used for curing, and such a dental material can be used as, for example, a bonding agent, a resin type adhesive or a temporary adhesive. As the polymerization initiator, the same substance as used for the aforesaid curable composition is employable.

### INDUSTRIAL APPLICABILITY

The sulfur-containing unsaturated carboxylic acid ester compound having a substituent containing a sulfur atom and at least two (meth)acrylic acid residues through an oxygen atom bonded to a secondary or tertiary carbon atom, specifically the polymerizable monomer represented by the formula (1), can be widely used singly or in combination with a polymerization initiator and a filler to serve as a dental material or a dental composition. The dental composition containing at least the polymerizable monomer represented by the formula (1), the filler and the polymerization initiator satisfies requirements for dental compositions, such as mechanical strength, abrasion resistance and water resistance, is excellent in X-ray opacity, transparency, curability (particularly photo-curability) and stain resistance, and provides a cured product having small polymerizable shrinkage (especially when a composite filler is contained).

Since the dental composition of the invention has X-ray opacity superior to that of the conventional compositions, the dental treatment can be made with confirming the position of the composition used.

### EXAMPLE

The present invention is further described with reference to the following examples and comparative examples, but it should be construed that the invention is in no way limited to those examples.

The materials used in the following examples are described below.

### Monomer

MNA-10: 1,3-bis(3-phenylthio-2-acryloyloxypropyloxy)benzene (refractive index of cured product of the compound only: 1.62)

MNA-12: 1,3-{2'-acryloyloxy-3'-[2-(1,3-dithiolan-2-yl)ethylthio]propyloxy}benzene (refractive index of cured product of the compound only: 1.61, high-viscosity liquid)

MNA-13: 1,3-bis(3-phenylthio-2-methacryloyloxypropyloxy)benzene (refractive index of cured product of the compound only: 1.61, high-viscosity liquid)

MNA-30: 4,4'-bis(3-phenylthio-2-acryloyloxypropyloxy)biphenyl (refractive index of cured product of the compound only: 1.61, high-viscosity liquid)

Bis-GMA: 2,2-bis[4-(2-hydroxy-3-methacryloxypropoxy)phenyl]propane (refractive index: 1.54, high-viscosity liquid)

TEGDMA: triethylene glycol dimethacrylate (refractive index: 1.46)

UDMA: 1,6-bis(methacryloxyethyloxycarbonylamino)-2,2,4-(or -2,4,4-)trimethylhexane (refractive index: 1.48)

Bis-MPEPP: 2,2-bis(4-methacryloxypolyethoxyphenyl)propane (refractive index: 1.54)

### Glass powder

A glass: obtained by treating a glass (refractive index: 1.60, average particle diameter: 5 µm) comprising 40 % by weight of silicon dioxide, 40 % by weight of barium oxide, 10 % by weight of boron oxide and 10 % by weight of aluminum oxide, with 1 % by weight of [3-(methacryloyloxy)propyl]trimethoxysilane in a conventional manner

B glass: obtained by treating a glass (refractive index: 1.60, average particle diameter: 1 µm) comprising 40 % by weight of silicon dioxide, 40 % by weight of barium oxide, 10 % by weight of boron oxide and 10 % by weight of aluminum oxide, with 3 % by weight of [3-(methacryloyloxy)propyl]trimethoxysilane in a conventional manner

C glass: obtained by treating a glass (refractive index: 1.55, average particle diameter: 1 µm) comprising 50 % by weight of silicon dioxide, 30 % by weight of barium oxide, 10 % by weight of boron oxide and 10 % by weight of aluminum oxide, with 3 % by weight of [3-(methacryloyloxy)propyl]trimethoxysilane in a conventional manner

### Fine particle silica

R-812: obtained by hydrophobic treatment of colloidal silica (average particle diameter: 0.007 µm) with dimethyldichlorosilane (proportion of particles having particle diameters of not less than 0.01 µm: about 10 %, available from Nippon Aerozil K.K.)

### Preparation of samples and measurement of various properties

### Measurement of refractive index

The refractive index was measured at 20°C in a conventional manner by the use of an Abbe's refractometer (IT model manufactured by Atago K.K.).

### Curing of composition

A dental composition (composite resin) experimentally prepared was filled in a mold of desired shape and irradiated with visible rays for 60 seconds by the use of a visible ray irradiation apparatus (Translux CL manufactured by Kulzer Co.) to cure the composition.

### Flexural strength and X-ray opacity

Tests were carried out in accordance with 7.8 (flexural strength) and 7.11 (X-ray opacity) of ISO-4049 (1988).

The flexural strength was measured by the use of an autograph AGS-2000G manufactured by Shimadzu Seisakusho K.K. at a crosshead speed of 1 mm/min. The X-ray opacity was measured by X-ray photographing a circular cured product having a thickness of 2.0 mm by the use of an X-ray control apparatus (PCX-100 manufactured by Asahi Roentgen Kogyo K.K.) and then calculating the density of the photographed image as an Al equivalent (%) by a densitometer (PDA15 manufactured by Konica K.K.) based on the density of a photographed image of an Al plate of the same thickness.

### Light transmittance (transparency)

A Teflon mold of 1 mm thickness having a rectangular hole of 10 mm (width) × 25 mm (length) was filled with a dental composition (composite resin), then sandwiched between a polyester film and a glass plate and irradiated with visible rays for 60 seconds per spot by the use of a visible ray irradiation apparatus (Translux CL manufactured by Kulzer Co.) to cure the composition. Making reference to the description of 7.8.2.2 of ISO4049 (1988), irradiation with visible rays was carried out so that the whole sample should be irradiated with the rays uniformly and sufficiently. The light transmittance of the sample at a wavelength of 480 nm was measured by the use of an ultraviolet-visible spectrophotometer (UV-160A manufactured by Shimadzu Seisakusho K.K.).

### Evaluation of polymerization shrinkage of the composition

As shown in Fig. 1, an alumina ceramic tube having an inner diameter of 6 mm and a height of 5 mm was filled with a dental composition (composite resin). In order to prevent disconnection of the polymerized dental composition from the tube due to shrinkage, the tube was sufficiently filled with the composition so that the composition came out of one end of the tube and covered the tube. Then, the dental composition (composite resin) filled in the tube was irradiated with a light for 180 seconds from the upper surface side of the tube by the use of a visible ray irradiation apparatus (Translux CL manufactured by Kulzer) to cure the dental composition (composite resin) in the tube. Thereafter, the tube with the dental composition was placed in an industrial visible ray irradiation apparatus (α-Light II manufactured by Morita Seisakusho K.K.) and irradiated with visible rays around the tube for 300 seconds to completely cure the dental composition (composite resin) present inside and outside the tube.

After the curing, the alumina tube was cut at the position of 2 mm from the top of the tube. The cut surface was subjected to mirror polishing and cleaned by an ultrasonic cleaner. Then, the gap formed between the cured product and the tube was measured at 8 spots, and the total of widths of the gaps at the facing two measuring spots was taken as a width of gap due to polymerization shrinkage.

### Example 1

### Preparation of composite resin

In a monomer mixture (refractive index: 1.58) consisting of 50 parts by weight of MNA-10 and 50 parts by weight of Bis-MPEPP, 0.5 part by weight of camphorquinone and 0.5 part by weight of ethyl N,N-dimethylaminobenzoate were dissolved. The resulting component was mixed with 400 parts by weight of the A glass and 20 parts by weight of R-812 to prepare a dental composition as a homogeneous paste. The polymerization shrinkage of a cured product of the dental composition was extremely small, as shown as a gap width in Table 1. The refractive index of the cured product of the dental composition was 1.60.

The flexural strength, light transmittance and X-ray opacity of the cured product of the dental composition are set forth in Table 1.

### Example 2

### Preparation of composite filler

In a monomer mixture (refractive index: 1.58) consisting of 75 parts by weight of MNA-10 and 25 parts by weight of TEGDMA, 0.5 part by weight of benzoyl peroxide was dissolved. The resulting component and 400 parts by weight of the B glass were sufficiently mixed to prepare a homogeneous paste. The paste was heated at 120°C for 15 minutes by a compression molding machine with application of pressure to cure the paste. The cured product of the paste was pulverized by a ball mill and sieved to prepare a composite filler A having an average particle diameter of about 20 µm.

### Preparation of composite resin

In a monomer mixture (refractive index: 1.58) consisting of 75 parts by weight of MNA-10 and 25 parts by weight of TEGDMA, 0.5 part by weight of camphorquinone and 0.5 part by weight of ethyl N,N-dimethylaminobenzoate were dissolved. The resulting component was mixed with 350 parts by weight of the A glass, 50 parts by weight of the composite filler A and 30 parts by weight of R-812 to prepare a dental composition as a homogeneous paste. The polymerization shrinkage of a cured product of the dental composition was extremely small, as shown as a gap width in Table 1. The refractive index of the cured product of the dental composition was 1.60.

The flexural strength, light transmittance and X-ray opacity of the cured product of the dental composition are set forth in Table 1.

### Example 3

### Preparation of composite resin

In a monomer mixture (refractive index: 1.58) consisting of 60 parts by weight of MNA-12 and 40 parts by weight of Bis-MPEPP, 0.5 part by weight of camphorquinone and 0.5 part by weight of ethyl N,N-dimethylaminobenzoate were dissolved. The resulting component was mixed with 400 parts by weight of the A glass and 20 parts by weight of R-812 to prepare a dental composition as a homogeneous paste. The polymerization shrinkage of a cured product of the dental composition was extremely small, as shown as a gap width in Table 1. The refractive index of the cured product of the dental composition was 1.60.

The flexural strength, light transmittance and X-ray opacity of the cured product of the dental composition are set forth in Table 1.

### Example 4

### Preparation of composite resin

In a monomer mixture (refractive index: 1.58) consisting of 60 parts by weight of MNA-13 and 40 parts by weight of Bis-MPEPP, 0.5 part by weight of camphorquinone and 0.5 part by weight of ethyl N,N-dimethylaminobenzoate were dissolved. The resulting component was mixed with 350 parts by weight of the B glass and 25 parts by weight of R-812 to prepare a dental composition as a homogeneous paste. The polymerization shrinkage of a cured product of the dental composition was extremely small, as shown as a gap width in Table 1. The refractive index of the cured product of the dental composition was 1.60.

The flexural strength, light transmittance and X-ray opacity of the cured product of the dental composition are set forth in Table 1.

### Example 5

### Preparation of composite resin

In a monomer mixture (refractive index: 1.58) consisting of 60 parts by weight of MNA-30 and 40 parts by weight of Bis-MPEPP, 0.5 part by weight of camphorquinone and 0.5 part by weight of ethyl N,N-dimethylaminobenzoate were dissolved. The resulting component was mixed with 400 parts by weight of the A glass and 20 parts by weight of R-812 to prepare a dental composition as a homogeneous paste. The polymerization shrinkage of a cured product of the dental composition was extremely small, as shown as a gap width in Table 1. The refractive index of the cured product of the dental composition was 1.60.

The flexural strength, light transmittance and X-ray opacity of the cured product of the dental composition are set forth in Table 1.

### Comparative Example 1

### Preparation of composite resin

In a monomer mixture (refractive index: 1.52) consisting of 70 parts by weight of Bis-GMA and 30 parts by weight of TEGDMA, 0.5 part by weight of camphorquinone and 0.5 part by weight of ethyl N,N-dimethylaminobenzoate were dissolved. The resulting component was mixed with 400 parts by weight of the A glass and 25 parts by weight of R-812 to prepare a dental composition as a homogeneous paste. The polymerization shrinkage of the dental composition is set forth as a gap width in Table 1. The refractive index of a cured product of the dental composition was 1.54.

The flexural strength, light transmittance and X-ray opacity of the cured product of the dental composition are set forth in Table 1.

### Comparative Example 2

### Preparation of composite resin

In a monomer mixture (refractive index: 1.52) consisting of 70 parts by weight of Bis-GMA and 30 parts by weight of TEGDMA, 0.5 part by weight of camphorquinone and 0.5 part by weight of ethyl N,N-dimethylaminobenzoate were dissolved. The resulting component was mixed with 350 parts by weight of the C glass and 20 parts by weight of R-812 to prepare a dental composition as a homogeneous paste. The polymerization shrinkage of the dental composition is set forth as a gap width in Table 1. The refractive index of a cured product of the dental composition was 1.55.

The flexural strength, light transmittance and X-ray opacity of the cured product of the dental composition are set forth in Table 1.

### Comparative Example 3

### Preparation of composite resin

In a monomer mixture (refractive index: 1.52) consisting of 35 parts by weight of Bis-GMA, 35 parts by weight of Bis-MPEPP and 30 parts by weight of TEGDMA, 0.5 part by weight of camphorquinone and 0.5 part by weight of ethyl N,N-dimethylaminobenzoate were dissolved. The resulting component was mixed with 400 parts by weight of the A glass and 20 parts by weight of R-812 to prepare a dental composition as a homogeneous paste. The polymerization shrinkage of the dental composition is set forth as a gap width in Table 1. The refractive index of a cured product of the dental composition was 1.54.

The flexural strength, light transmittance and X-ray opacity of the cured product of the dental composition are set forth in Table 1.

### Comparative Example 4

### Preparation of composite resin

In a monomer mixture (refractive index: 1.52) consisting of 35 parts by weight of Bis-GMA, 35 parts by weight of Bis-MPEPP, 15 parts by weight of TEGDMA and 15 parts by weight of UDMA, 0.5 part by weight of camphorquinone and 0.5 part by weight of ethyl N,N-dimethylaminobenzoate were dissolved. The resulting component was mixed with 400 parts by weight of the A glass and 20 parts by weight of R-812 to prepare a dental composition as a homogeneous paste. The refractive index of a cured product of the dental composition was 1.54.

The flexural strength, light transmittance and X-ray opacity of the cured product of the dental composition are set forth in Table 1.

### Comparative Example 5

### Preparation of composite resin

In 100 parts by weight of Bis-MPEPP (refractive index: 1.54), 0.5 part by weight of camphorquinone and 0.5 part by weight of ethyl N,N-dimethylaminobenzoate were dissolved. The resulting component was mixed with 400 parts by weight of the A glass and 20 parts by weight of R-812 to prepare a dental composition as a homogeneous paste. The refractive index of a cured product of the dental composition was 1.56.

The flexural strength, light transmittance and X-ray opacity of the cured product of the dental composition are set forth in Table 1.

### Comparative Example 6

### Preparation of composite resin

An attempt to mix Bis-GMA (refractive index: 1.54) with the A glass was made, but they could not be mixed because the viscosity of the BIS-GMA was too high.

**Table 1**

| (Flexural strength, light transmittance, X-ray opacity and polymerization shrinkage of composite resin) | | | | |
|---|---|---|---|---|
| | Flexural strength (MPa) | Light transmittance (T %) | X-ray opacity (%, aluminum base) | Gap width (µm) |
| Ex. 1 | 124 | 13.4 | 580 | 27.6 |
| Ex. 2 | 102 | 7.3 | 530 | 22.8 |
| Ex. 3 | 117 | 14.1 | 580 | 24.3 |
| Ex. 4 | 128 | 10.1 | 500 | 25.5 |
| Ex. 5 | 103 | 13.3 | 570 | 23.2 |
| Comp. Ex.1 | 122 | 0.6 | 580 | 41.5 |
| Comp. Ex.2 | 106 | 11.8 | 220 | 46.4 |
| Comp. Ex.3 | 121 | 0.6 | 570 | 45.2 |
| Comp. Ex.4 | 129 | 0.5 | 580 | - |
| Comp. Ex.5 | 103 | 0.8 | 580 | - |

As can be seen from Table 1, with regard to the mechanical strength of the composite resin that is indicated by the flexural strength, there was no large difference between the examples and the comparative examples, and they had sufficient strengths nearly equal to one another. On the other hand, with regard to both the light transmittance indicating transparency and the X-ray opacity, the composite resins of the examples exhibited excellent results. Comparative Example 2 showed light transmittance of good value but was inferior to the examples and other comparative examples in the X-ray opacity. The composite resins of other comparative examples were all equal to the composite resins of the examples in the X-ray opacity, but they showed light transmittance of much lower values than the composite resins of the examples.

## Claims

1. A dental material comprising a sulfur-containing unsaturated carboxylic acid ester compound having a substituent containing a sulfur atom and at least two (meth)acrylic acid residues through an oxygen atom bonded to a secondary or tertiary carbon atom.

2. The dental material as claimed in claim 1, comprising at least one polymerizable monomer (A) represented by the following formula (I): 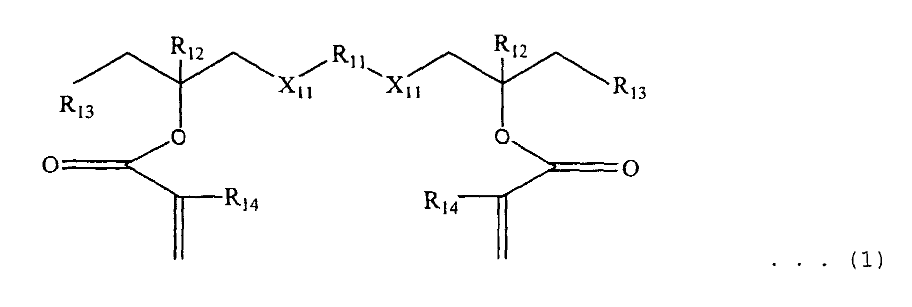 wherein R₁₁ is a divalent organic group, each X₁₁ is independently an oxygen atom, a sulfur atom, -COO- group or -(CH₂)_{q}X₁₂- group (X₁₂ is an oxygen atom or a sulfur atom, and q is an integer of 1 to 3), each R₁₂ is independently a hydrogen atom or an alkyl group, each R₁₃ is independently a substituent containing a sulfur atom, and each R₁₄ is independently a hydrogen atom or a methyl group.

3. The dental material as claimed in claim 2, wherein R₁₁ in the formula (1) is a group represented by any one of the following formulas (2) to (4): 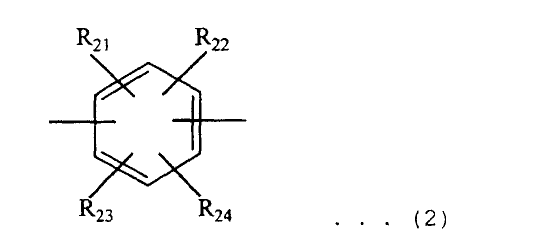 wherein R₂₁, R₂₂, R₂₃ and R₂₄ are each independently a hydrogen atom, an alkyl group, an alkoxyl group, a nitro group or a halogen atom, 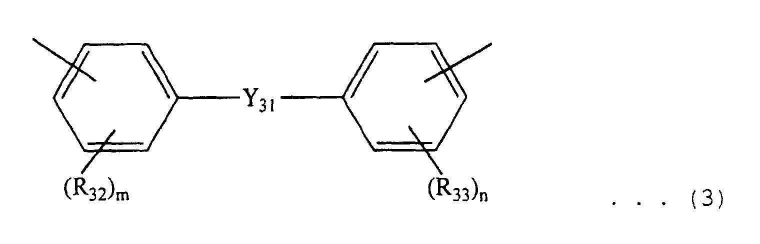 wherein Y₃₁ is a single bond, -C(R₃₁)₂- group (each R₃₁ is independently a hydrogen atom or a methyl group), -O- group, -S- group or -SO₂- group, R₃₂ and R₃₃ are each independently an alkyl group, an alkenyl group, an aralkyl group, an aryl group, an alkoxyl group, an alkylthio group, a nitro group or a halogen atom, and m and n are each independently 0 or an integer of 1 to 4, 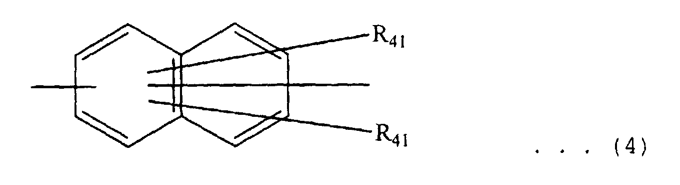 wherein each R₄₁ is independently a hydrogen atom or an alkyl group.

4. A dental composition comprising:
(A) at least one polymerizable monomer represented by the formula (1),
(B) a polymerization initiator, and
(C) a filler.

5. The dental composition as claimed in claim 4, further comprising (D) another polymerizable monomer in addition to the polymerizable monomer (A) represented by the formula (1).

6. The dental composition as claimed in claim 4 or 5, wherein the filler (C) has an X-ray opaque value of 100 to 1000 % based on the X-ray opaque value of aluminum.

7. The dental composition as claimed in claim 4 or 5, wherein the light transmittance of a cured product of said dental composition having a thickness of 1 mm, at a wavelength of 480 nm, is not less than 1 %.

8. The dental composition as claimed in claim 4 or 5, wherein the refractive index of a cured product of the polymerizable monomer (A) represented by the formula (1) or a cured product of a mixture of the polymerizable monomer (A) and the polymerizable monomer (D) is not less than 1.55, and the difference between said refractive index and a refractive index of the filler (C) is not more than 0.05.

9. The dental composition as claimed in claim 4, wherein the refractive index of the filler (C) is not less than 1.55.

10. The dental composition as claimed in claim 4, wherein a part or all of the filler (C) is contained as a composite filler formed from a polymerizable monomer and an inorganic compound.
